(19) Europäisches Patentamt
European Patent Office
Office européen des brevets

(11) EP 4 610 256 A1

(12) **EUROPEAN PATENT APPLICATION**
published in accordance with Art. 153(4) EPC

(43) Date of publication:
**03.09.2025 Bulletin 2025/36**

(21) Application number: **23881873.6**

(22) Date of filing: **25.10.2023**

(51) International Patent Classification (IPC):
***C07D 407/04*** (2006.01) ***C07D 471/04*** (2006.01)
***A61K 31/4375*** (2006.01) ***A61K 31/519*** (2006.01)
***A61P 35/00*** (2006.01)

(52) Cooperative Patent Classification (CPC):
**A61K 31/4375; A61K 31/519; A61P 35/00; C07D 407/04; C07D 471/04**

(86) International application number:
**PCT/CN2023/126415**

(87) International publication number:
**WO 2024/088296 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.10.2022 CN 202211312683**
**09.12.2022 CN 202211589461**
**07.03.2023 CN 202310225022**
**19.05.2023 CN 202310567051**

(71) Applicant: **Tuojie Biotech (Shanghai) Co., Ltd.**
**Shanghai 201203 (CN)**

(72) Inventors:
- **ZHU, Guodong**
  **Shanghai 201203 (CN)**
- **HU, Tao**
  **Shanghai 201203 (CN)**
- **CHEN, Meijun**
  **Shanghai 201203 (CN)**
- **HUO, Shuhua**
  **Shanghai 201203 (CN)**
- **LI, Jiao**
  **Shanghai 201203 (CN)**
- **LI, Yunfei**
  **Shanghai 201203 (CN)**

(74) Representative: **Dragotti & Associati S.R.L.**
**Via Nino Bixio, 7**
**20129 Milano (IT)**

(54) **PIPERIDINOPYRIMIDINE DERIVATIVE, PREPARATION METHOD THEREFOR AND USE THEREOF IN MEDICINE**

(57) The present disclosure relates to a piperidinopyrimidine derivative, a preparation method therefor and the use thereof in medicine. Specifically, the present disclosure relates to a piperidinopyrimidine derivative as represented by general formula (I), a preparation method therefor, a pharmaceutical composition containing the derivative, and the use thereof as a CDK7 inhibitor in the treatment of diseases or conditions related to abnormal activity of CDK7. Each group in general formula (I) is as defined in the description.

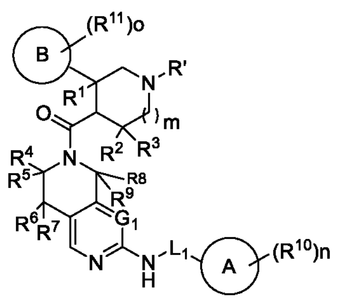

(I)

EP 4 610 256 A1

## Description

### TECHNICAL FIELD

The present disclosure pertains to the field of pharmaceutics and relates to a piperidinopyrimidine derivative, a preparation method therefor, and pharmaceutical use thereof. In particular, the present disclosure relates to a pyrimidine derivative represented by general formula (I), a preparation method therefor, a pharmaceutical composition comprising the derivative, and use thereof as a CDK7 inhibitor in the treatment of a disease or disorder associated with CDK7 activity.

### BACKGROUND

Cyclin-dependent kinases (CDKs) represent an important category of kinases and play a crucial role in the division and proliferation of cancer cells, as well as in the transcriptional regulation of oncogenes. To date, over 20 subtypes of cyclin-dependent kinases (CDKs) have been identified. Due to the domain sequence and structural similarities between the CDK family member kinases,
selectively and precisely regulating each subtype poses a significant challenge.

Cyclin-dependent kinase 7 (CDK7) is a special member of the CDK family, possessing a dual function in the regulation of cell division and transcription. CDK7 binds to cyclin H and MAT1, forming a trimeric cyclin-activating kinase (CAK). The kinase activates the activity of the corresponding CDKs through the phosphorylation of CDKs involved in cell cycle control (including CDK1, CDK2, CDK4, and CDK6), thereby regulating the cell cycle. CDK7 also acts as a component of the common transcription factor II H (TFIIH) and participates in the auxiliary regulation of transcription. It is involved in the initiation of transcription through the phosphorylation of the Rbp1 subunit of RNA polymerase II (RNAPII) and can regulate transcriptional elongation by phosphorylating the CDK9 complex.

A hallmark of cancer is uncontrolled cell proliferation and transcriptional dysregulation. Therefore, CDK7 inhibitors that simultaneously inhibit transcription and the cell cycle process are a theoretically and relatively feasible target for cancer treatment. Currently, no drugs selectively regulating the target are available on the market. We envision developing a highly selective CDK7 inhibitor to treat diseases associated with CDK7 activity.

Disclosed patent applications of CDK7 inhibitors include WO2016058544, WO2018013867, WO2019143719, WO2019143730, WO2019099298, WO2020093006, WO2020093011, WO2022064009A, etc.

### SUMMARY

The present disclosure aims to provide a compound represented by formula (I) or a pharmaceutically acceptable salt thereof:

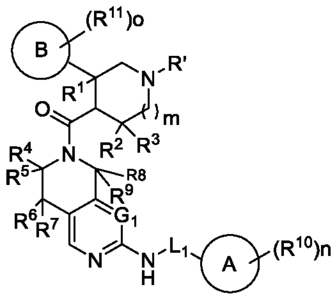

(I) ,

wherein $G_1$ is N or $CR^{1a}$;

$R^1$, $R^2$, $R^3$, and $R^{1a}$ are each independently selected from the group consisting of hydrogen, deuterium, cyano, hydroxy, $C_{1-6}$ alkyl, and halogen (e.g., fluorine, chlorine, bromine, or iodine), and at least one thereof is not hydrogen or deuterium;

$R^4$, $R^5$, $R^6$, $R^7$, $R^8$, and $R^9$ are each independently selected from the group consisting of hydrogen, deuterium, halogen, $C_{1-6}$ alkyl, cyano, $C_{2-6}$ alkenyl, $C_{2-6}$ alkynyl, and 3- to 6-membered cycloalkyl, wherein the $C_{1-6}$ alkyl, $C_{2-6}$ alkenyl, $C_{2-6}$ alkynyl, or 3- to 6-membered cycloalkyl is optionally substituted with one or more substituents independently selected from the group consisting of $R^A$, and $R^A$ is selected from the group consisting of deuterium, halogen, hydroxy, cyano, and 3- to 6-membered cycloalkyl;

$L_1$ is selected from the group consisting of a chemical bond and $C_{1-6}$ alkylene, wherein the $C_{1-6}$ alkylene is optionally

substituted with one or more substituents independently selected from the group consisting of $R^B$, and $R^B$ is selected from the group consisting of deuterium, halogen, hydroxy, $C_{1-6}$ alkoxy, amino, and oxo, or two $R^B$ attached to the same carbon atom, together with the carbon atom to which they are both attached, form 3- to 6-membered cycloalkyl or 3- to 7-membered heterocyclyl containing at least one heteroatom selected from the group consisting of N, O, and S;

ring A is selected from the group consisting of 3- to 6-membered cycloalkyl, 6- to 10-membered aryl, 5- to 12-membered heteroaryl, and 3- to 12-membered heterocyclyl;

each $R^{10}$ is independently selected from the group consisting of deuterium, cyano, halogen, hydroxy, amino, $C_{2-6}$ alkynyl, $C_{2-6}$ alkenyl, -S-$C_{1-6}$ alkyl, $C_{1-6}$ alkoxy, oxo, $C_{1-6}$ alkyl, 3- to 6-membered cycloalkyl, 6- to 10-membered aryl, 5- to 12-membered heteroaryl, 3- to 12-membered heterocyclyl, -NH-(C=O)-$C_{1-6}$ alkyl, -NH-(C=O)-$C_{3-6}$ cycloalkyl, -NH(C=O)-O$C_{1-6}$ alkyl, -NH(C=O)-O$C_{3-6}$ cycloalkyl, -O(C=O)NH$C_{1-6}$ alkyl, -O(C=O)NH-$C_{3-6}$ cycloalkyl, -(C=O)NH-$C_{1-6}$ alkyl, -(C=O)-NH-$C_{3-6}$ cycloalkyl, -(C=O)-$C_{1-6}$ alkyl, -(C=O)-$C_{3-6}$ cycloalkyl, -$SO_2$-$C_{1-6}$ alkyl, -$SO_2$-$C_{3-6}$ cycloalkyl, -$SO_2$-$NH_2$, - $SO_2$-NH-$C_{1-6}$ alkyl, -$SO_2$-NH-$C_{3-6}$ cycloalkyl, -$SO_2$-N($C_{1-6}$ alkyl)$_2$, -$SO_2$-NH($C_{3-6}$ cycloalkyl)$_2$, -S(O)(NH)-$C_{1-6}$ alkyl, and -S(O)(NH)-$C_{3-6}$ cycloalkyl, wherein

the $C_{1-6}$ alkyl, 3- to 6-membered cycloalkyl, 6- to 10-membered aryl, 5- to 12-membered heteroaryl, or 3- to 12-membered heterocyclyl is optionally substituted with one or more substituents independently selected from the group consisting of $R^C$, and $R^C$ is selected from the group consisting of deuterium, halogen, $C_{1-6}$ alkoxy, hydroxy, amino, oxo, $C_{2-6}$ alkynyl, $C_{2-6}$ alkenyl, cyano, $C_{1-6}$ hydroxyalkyl, 3- to 12-membered heterocyclyl, $C_{1-6}$ alkyl, $C_{3-6}$ cycloalkyl, 6- to 10-membered aryl, and 5- to 12-membered heteroaryl;

$R^{11}$ is selected from the group consisting of hydrogen, deuterium, halogen, cyano, $C_{1-6}$ alkyl, $C_{1-6}$ alkoxy, 3- to 6-membered cycloalkyl, 3- to 12-membered heterocyclyl, $C_{2-6}$ alkenyl, and $C_{2-6}$ alkynyl, wherein the $C_{1-6}$ alkyl, $C_{1-6}$ alkoxy, 3- to 6-membered cycloalkyl, 3- to 12-membered heterocyclyl, $C_{2-6}$ alkenyl, or $C_{2-6}$ alkynyl is optionally substituted with one or more substituents independently selected from the group consisting of $R^D$, and $R^D$ is selected from the group consisting of hydrogen, deuterium, halogen, cyano, hydroxy, alkynyl, $C_{1-6}$ alkoxy, $C_{1-6}$ hydroxyalkyl, and 3- to 6-membered cycloalkyl;

ring B is selected from the group consisting of 5- to 12-membered heteroaryl and 6- to 12-membered aryl;

R' is selected from the group consisting of hydrogen, cyano, $C_{1-6}$ alkyl, 3- to 6-membered cycloalkyl, and 3- to 12-membered heterocyclyl, wherein the $C_{1-6}$ alkyl, 3- to 6-membered cycloalkyl, or 3- to 12-membered heterocyclyl is optionally substituted with one or more substituents independently selected from the group consisting of $R^E$, and $R^E$ is selected from the group consisting of hydrogen, deuterium, halogen, cyano, hydroxy, alkynyl, $C_{1-6}$ alkoxy, $C_{1-6}$ hydroxyalkyl, 3- to 6-membered cycloalkyl, and 3- to 7-membered heterocyclyl;

m is selected from the group consisting of 0 and 1;

n is selected from the group consisting of 0, 1, 2, 3, 4, and 5;

o is selected from the group consisting of 0, 1, 2, 3, and 4.

In another aspect, the present disclosure provides a compound represented by formula (VI) or a pharmaceutically acceptable salt thereof,

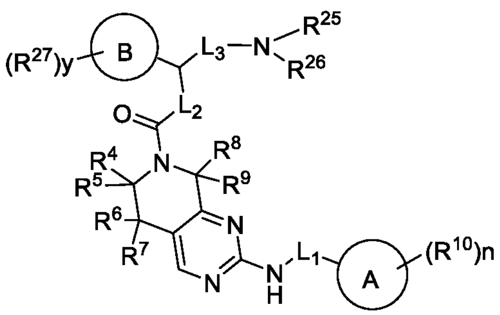

(VI)

wherein $L_2$ is selected from the group consisting of -NH- and -O-;

ring B is selected from the group consisting of 5- to 12-membered heteroaryl and 6- to 12-membered aryl;

$L_3$ is selected from the group consisting of $C_{0-6}$ alkylene, wherein the $C_{0-6}$ alkylene is optionally substituted with one or more substituents independently selected from the group consisting of $R^I$, and $R^I$ is selected from the group consisting of deuterium, halogen, oxo, hydroxy, amino, and $C_{1-6}$ alkyl;

$R^{25}$ and $R^{26}$ are each independently selected from the group consisting of hydrogen, $C_{1-6}$ alkyl, and $C_{1-6}$ haloalkyl, or $R^{25}$ and $R^{26}$, together with the nitrogen atom to which they are attached, form a 4- to 7-membered nitrogen-containing

heterocyclic ring, wherein the 4- to 7-membered nitrogen-containing heterocyclic ring is optionally substituted with one or more substituents independently selected from the group consisting of $R^J$, and $R^J$ is selected from the group consisting of halogen, hydroxy, cyano, nitro, and amino;

each $R^{27}$ is independently selected from the group consisting of deuterium, halogen, hydroxy, carboxyl, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 6-membered cycloalkyl, -S-$C_{1-6}$ alkyl, $C_{1-6}$ alkoxy, $C_{1-6}$ haloalkoxy, -NH-$C_{1-6}$ alkyl, -NH($C_{1-6}$ alkyl)$_2$, -(C=O)-$NH_2$, alkyl-(C=O)-NH-$C_{1-6}$ alkyl, -(C=O)-NH-($C_{1-6}$ alkyl)$_2$, -(C=O)$C_{1-6}$ alkyl, and -NH-(C=O)$C_{1-6}$ alkyl;

y is selected from the group consisting of 0, 1, 2, 3, and 4;

$R^4$, $R^5$, $R^6$, $R^7$, $R^8$, $R^9$, $L_1$, ring A, $R^{10}$, and n are each as defined in claim 1, provided that $R^4$ and $R^5$ are not simultaneously hydrogen.

In an optional embodiment, provided in the present disclosure is the compound represented by formula (VI) or the pharmaceutically acceptable salt thereof, wherein $L_2$ is -NH-.

In an optional embodiment, provided in the present disclosure is the compound represented by formula (VI) or the pharmaceutically acceptable salt thereof, wherein $L_2$ is -O-.

In an optional embodiment, provided in the present disclosure is the compound represented by formula (VI) or the pharmaceutically acceptable salt thereof, wherein $L_3$ is -$CH_2CH_2$-.

In an optional embodiment, provided in the present disclosure is the compound represented by formula (VI) or the pharmaceutically acceptable salt thereof, wherein ring B is phenyl or pyridyl.

In an optional embodiment, provided in the present disclosure is the compound represented by formula (VI) or the pharmaceutically acceptable salt thereof, wherein ring B is phenyl.

In an optional embodiment, provided in the present disclosure is the compound represented by formula (VI) or the pharmaceutically acceptable salt thereof, wherein each $R^{27}$ is independently selected from the group consisting of hydrogen, deuterium, halogen, cyano, hydroxy, carboxyl, $C_{1-6}$ alkyl, $C_{1-6}$ haloalkyl, $C_{1-6}$ hydroxyalkyl, $C_{2-6}$ alkynyl, 3- to 6-membered cycloalkyl, 3- to 12-membered heterocycloalkyl, 5- to 12-membered aryl, and heteroaryl.

In an optional embodiment, provided in the present disclosure is the compound represented by formula (VI) or the pharmaceutically acceptable salt thereof, wherein each $R^{27}$ is independently selected from the group consisting of halogen, $C_{1-6}$ alkyl, $C_{1-6}$ haloalkyl, and $C_{2-6}$ alkynyl.

In an optional embodiment, provided in the present disclosure is the compound represented by formula (VI) or the pharmaceutically acceptable salt thereof, wherein each $R^{27}$ is independently selected from the group consisting of chlorine, fluorine, trifluoromethyl, and ethynyl.

In an optional embodiment, provided in the present disclosure is the compound represented by formula (VI) or the pharmaceutically acceptable salt thereof, wherein $R^{25}$ and $R^{26}$ are each independently selected from the group consisting of hydrogen and $C_{1-6}$ alkyl.

In an optional embodiment, provided in the present disclosure is the compound represented by formula (I) or (VI) or the pharmaceutically acceptable salt thereof, wherein $R^4$, $R^5$, $R^6$, $R^7$, $R^8$, and $R^9$ are each independently selected from the group consisting of hydrogen, deuterium, halogen, and $C_{1-6}$ alkyl.

In an optional embodiment, provided in the present disclosure is the compound represented by formula (I) or (VI) or the pharmaceutically acceptable salt thereof, wherein $R^4$ is methyl.

In an optional embodiment, provided in the present disclosure is the compound represented by formula (I) or (VI) or the pharmaceutically acceptable salt thereof, wherein $R^5$, $R^6$, $R^7$, $R^8$, and $R^9$ are each independently selected from the group consisting of hydrogen and deuterium.

In an optional embodiment, provided in the present disclosure is the compound represented by formula (I) or (VI) or the pharmaceutically acceptable salt thereof, wherein $R^5$, $R^6$, $R^7$, $R^8$, and $R^9$ are each independently selected from the group consisting of hydrogen and deuterium, and $R^4$ is methyl.

In an optional embodiment, provided in the present disclosure is the compound represented by formula (I) or (VI) or the pharmaceutically acceptable salt thereof, wherein $L_1$ is a chemical bond.

In an optional embodiment, provided in the present disclosure is the compound represented by formula (I) or (VI) or the pharmaceutically acceptable salt thereof, wherein $L_1$ is selected from the group consisting of $C_{1-6}$ alkylene, wherein the $C_{1-6}$ alkylene is optionally substituted with one or more substituents independently selected from the group consisting of $R^B$, and $R^B$ is selected from the group consisting of deuterium, halogen, hydroxy, and $C_{1-6}$ alkoxy.

In an optional embodiment, provided in the present disclosure is the compound represented by formula (I) or (VI) or the pharmaceutically acceptable salt thereof, wherein $L_1$ is methylene, wherein the methylene is optionally substituted with one or more substituents independently selected from the group consisting of $R^B$, and $R^B$ is selected from the group consisting of deuterium, halogen, hydroxy, and $C_{1-6}$ alkoxy.

In an optional embodiment, provided in the present disclosure is the compound represented by formula (I) or (VI) or the pharmaceutically acceptable salt thereof, wherein ring A is selected from the group consisting of a pyrazole ring, an imidazole ring, a tetrahydropyran ring, a pyrimidine ring, and cyclohexyl.

In an optional embodiment, provided in the present disclosure is the compound represented by formula (I) or (VI) or the pharmaceutically acceptable salt thereof, wherein $R^{10}$ is selected from the group consisting of deuterium, cyano, $C_{2-6}$ alkynyl, -S-$C_{1-6}$ alkyl, $C_{1-6}$ alkoxy, and $C_{1-6}$ alkyl, wherein the $C_{2-6}$ alkynyl, -S-$C_{1-6}$ alkyl, $C_{1-6}$ alkoxy, or $C_{1-6}$ alkyl is optionally substituted with one or more substituents independently selected from the group consisting of $R^C$, and $R^C$ is selected from the group consisting of deuterium, halogen, $C_{1-6}$ alkoxy, hydroxy, amino, oxo, and $C_{2-6}$ alkynyl.

In an optional embodiment, provided in the present disclosure is the compound represented by formula (I) or (VI) or the pharmaceutically acceptable salt thereof, wherein

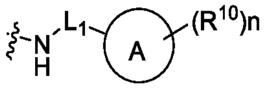

is selected from the group consisting of

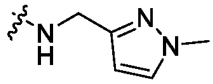

,

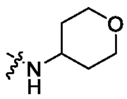

,

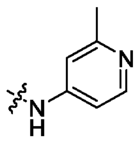

,

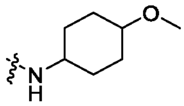

, and

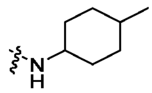

.

In some embodiments, the compound represented by formula (VI) or the pharmaceutically acceptable salt thereof provided in the present disclosure is a compound represented by formula (VI-1) or formula (VI-2) or a pharmaceutically acceptable salt thereof,

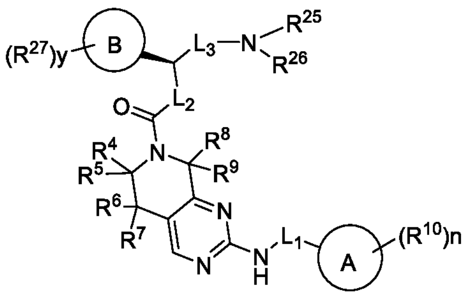

(VI-1)

or

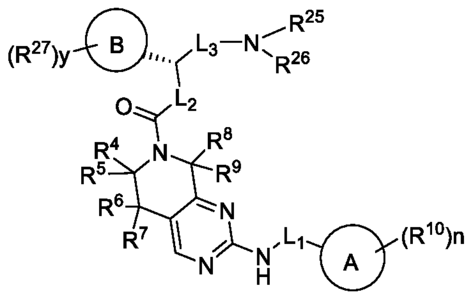

(VI-2)

,

wherein $L_2$ is selected from the group consisting of -NH- and -O-;

ring B is selected from the group consisting of 5- to 6-membered heteroaryl and 5- to 6-membered aryl;

$L_3$ is selected from the group consisting of $C_{1-3}$ alkylene (e.g., methylene, ethylene, or propylene), wherein the $C_{1-3}$ alkylene is optionally substituted with one or more substituents independently selected from the group consisting of $R^I$, and $R^I$ is selected from the group consisting of deuterium, halogen, oxo, hydroxy, amino, and $C_{1-6}$ alkyl (e.g., methyl, ethyl, *n*-propyl, isopropyl, *n*-butyl, isobutyl, or *tert*-butyl);

$R^{25}$ and $R^{26}$ are each independently selected from the group consisting of hydrogen and $C_{1-6}$ alkyl (e.g., methyl, ethyl, *n*-propyl, isopropyl, *n*-butyl, isobutyl, or *tert*-butyl), wherein the $C_{1-6}$ alkyl is optionally substituted with one or more deuterium atoms;

each $R^{27}$ is independently selected from the group consisting of deuterium, halogen, hydroxy, carboxyl, $C_{1-6}$ alkyl (e.g., methyl, ethyl, *n*-propyl, isopropyl, *n*-butyl, isobutyl, or *tert*-butyl), $C_{1-6}$ haloalkyl, $C_{1-6}$ hydroxyalkyl, $C_{2-6}$ alkenyl, $C_{2-6}$ alkynyl, $C_{1-6}$ alkylene-3- to 6-membered cycloalkyl (e.g., cyclopropyl or cyclobutyl), 3- to 6-membered cycloalkyl (e.g., cyclopropyl or cyclobutyl), -S-$C_{1-6}$ alkyl, $C_{1-6}$ alkoxy, $C_{1-6}$ haloalkoxy, - NH-$C_{1-6}$ alkyl, and -NH($C_{1-6}$ alkyl)$_2$;

y is selected from the group consisting of 0, 1, 2, 3, and 4;

$R^4$ is $C_{1-6}$ alkyl;

$R^5$, $R^6$, $R^7$, $R^8$, and $R^9$ are each independently selected from the group consisting of hydrogen, deuterium, halogen,

and $C_{1-6}$ alkyl;

$L_1$ is selected from the group consisting of a linking bond; or $L_1$ is selected from the group consisting of $C_{1-6}$ alkylene (e.g., methylene, ethylene, or propylene); or $L_1$ is selected form $C_{1-6}$ alkylene, wherein the $C_{1-6}$ alkylene is substituted with one or more substituents independently selected from the group consisting of $R^B$, and $R^B$ is selected from the group consisting of deuterium, halogen, hydroxy, $C_{1-6}$ alkyl (e.g., methyl, ethyl, *n*-propyl, isopropyl, *n*-butyl, isobutyl, or *tert*-butyl), and oxo;

ring A is selected from the group consisting of 5- to 6-membered aryl, 5- to 6-membered heteroaryl, and 3- to 7-membered heterocyclyl;

$R^{10}$ is selected from the group consisting of deuterium, cyano, $C_{2-6}$ alkynyl, -S-$C_{1-6}$ alkyl, $C_{1-6}$ alkoxy, $C_{1-6}$ alkyl (e.g., methyl, ethyl, *n*-propyl, isopropyl, *n*-butyl, isobutyl, or *tert*-butyl), $C_{1-6}$ alkylene-3- to 6-membered cycloalkyl, 3- to 6-membered cycloalkyl, 5- to 6-membered aryl, 5- to 6-membered heteroaryl, 5- to 12-membered heterocycloalkyl, -NH(C=O)-O$C_{1-6}$ alkyl, -(C=O)NH-$C_{1-6}$ alkyl, and (C=O)$NH_2$, wherein

the $C_{2-6}$ alkynyl, -S-$C_{1-6}$ alkyl, $C_{1-6}$ alkoxy, $C_{1-6}$ alkyl, 5- to 6-membered aryl, 5- to 6-membered heteroaryl, or 5- to 12-membered heterocycloalkyl is optionally substituted with one or more substituents independently selected from the group consisting of $R^C$ and $R^C$ is selected from the group consisting of deuterium, halogen, $C_{1-6}$ alkoxy, $C_{1-6}$ alkyl (e.g., methyl, ethyl, *n*-propyl, isopropyl, *n*-butyl, isobutyl, or *tert*-butyl), hydroxy, amino, oxo, and $C_{2-6}$ alkynyl;

n is selected from the group consisting of 0, 1, 2, and 3.

In some embodiments, the compound represented by formula (VI) or the pharmaceutically acceptable salt thereof provided in the present disclosure is a compound represented by formula (VI-1) or a pharmaceutically acceptable salt thereof.

In some embodiments, provided in the present disclosure is the compound represented by formula (VI-1) or formula (VI-2) or the pharmaceutically acceptable salt thereof, wherein $L_2$ is -NH-.

In some embodiments, provided in the present disclosure is the compound represented by formula (VI-1) or formula (VI-2) or the pharmaceutically acceptable salt thereof, wherein ring B is selected from the group consisting of pyridyl and phenyl, and each $R^{27}$ is independently selected from the group consisting of deuterium, halogen, hydroxy, carboxyl, $C_{1-6}$ alkyl, $C_{2-6}$ alkenyl, $C_{2-6}$ alkynyl, and $C_{1-6}$ alkylene-3- to 6-membered cycloalkyl.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of deuterium, halogen, hydroxy, carboxyl, $C_{1-6}$ alkyl, $C_{2-6}$ alkenyl, $C_{2-6}$ alkynyl, and $C_{1-6}$ alkylene-3- to 6-membered cycloalkyl.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of deuterium.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of halogen.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of $C_{1-6}$ alkyl.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of methyl and ethyl.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of methylene-cyclopropyl.

In some embodiments, provided in the present disclosure is the compound represented by formula (VI-1) or formula (VI-2) or the pharmaceutically acceptable salt thereof, wherein ring B is phenyl, and each $R^{27}$ is independently selected from the group consisting of cyclopropyl.

In some embodiments, provided in the present disclosure is the compound represented by formula (VI-1) or formula (VI-2) or the pharmaceutically acceptable salt thereof, wherein $L_3$ is selected from the group consisting of methylene, wherein the methylene is optionally substituted with one or more substituents independently selected from the group consisting of $R^I$, and $R^I$ is selected from the group consisting of deuterium, halogen, oxo, hydroxy, amino, and $C_{1-6}$ alkyl (e.g., methyl, ethyl, *n*-propyl, isopropyl, *n*-butyl, isobutyl, or *tert*-butyl).

In some embodiments, provided in the present disclosure is the compound represented by formula (VI-1) or formula (VI-2) or the pharmaceutically acceptable salt thereof, wherein $L_3$ is selected from the group consisting of ethylene, wherein the ethylene is optionally substituted with one or more substituents independently selected from the group consisting of $R^I$, and $R^I$ is selected from the group consisting of deuterium, halogen, oxo, hydroxy, amino, and $C_{1-6}$

alkyl (e.g., methyl, ethyl, *n*-propyl, isopropyl, *n*-butyl, isobutyl, or *tert*-butyl).

In some embodiments, provided in the present disclosure is the compound represented by formula (VI-1) or formula (VI-2) or the pharmaceutically acceptable salt thereof, wherein $L_3$ is selected from the group consisting of propylene, wherein the propylene is optionally substituted with one or more substituents independently selected from the group consisting of $R^I$, and $R^I$ is selected from the group consisting of deuterium, halogen, oxo, hydroxy, amino, and $C_{1-6}$ alkyl (e.g., methyl, ethyl, *n*-propyl, isopropyl, *n*-butyl, isobutyl, or *tert*-butyl).

In some embodiments, the compound represented by formula (VI) or the pharmaceutically acceptable salt thereof provided in the present disclosure is a compound represented by formula (VI-1-A) or formula (VI-1-B) or a pharmaceutically acceptable salt thereof,

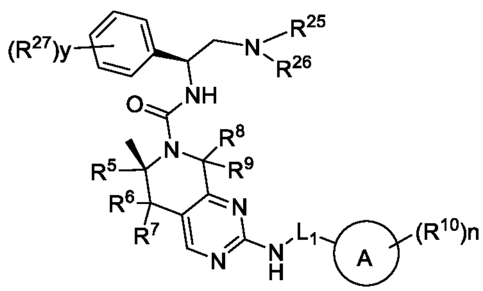

(VI-1-A)

or

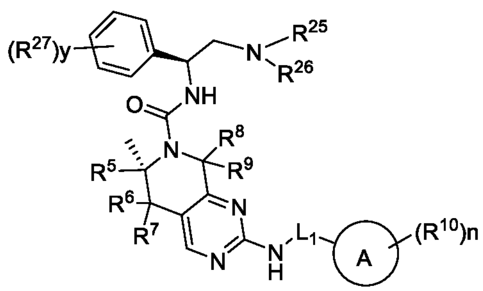

(VI-1-B)

,

wherein $R^5$, $R^6$, $R^7$, $R^8$, $R^9$, $L_1$, ring A, $R^{10}$, n, $R^{25}$, $R^{26}$, $R^{27}$, and y are each a compound represented by formula (VI-1-A) or formula (VI-1-B) or a pharmaceutically acceptable salt thereof.

In an optional embodiment, the compound represented by formula (VI) or the pharmaceutically acceptable salt thereof provided in the present disclosure is a compound represented by formula (VI-1-A) or a pharmaceutically acceptable salt thereof.

In some embodiments, provided in the present disclosure is the compound represented by formula (VI), (VI-1), (VI-2), (VI-1-A), or (VI-1-B) or the pharmaceutically acceptable salt thereof, wherein $L_1$ is a linking bond.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene; or $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of deuterium, $C_{1-6}$ alkyl, and oxo.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene, specifically methylene or ethylene.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of deuterium, methyl, ethyl, and propyl.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of deuterium.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of methyl.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of oxo.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of methylene; or $L_1$ is selected from the group consisting of methylene, wherein the methylene is substituted with one or more substituents independently selected from the group consisting of $R^B$, and $R^B$ is selected from the group consisting of deuterium and $C_{1-6}$ alkyl (e.g., methyl or ethyl).

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of methylene; or $L_1$ is selected from the group consisting of methylene, wherein the methylene is substituted with one or more substituents independently selected from the group consisting of $R^B$, and $R^B$ is selected from the group consisting of deuterium and methyl.

In some embodiments, provided in the present disclosure is the compound represented by formula (VI), (VI-1), (VI-2), (VI-1-A), or (VI-1-B) or the pharmaceutically acceptable salt thereof, wherein $L_1$ is selected from the group consisting of methylene, wherein the methylene is substituted with one or more substituents independently selected from the group consisting of $R^B$, and $R^B$ is selected from the group consisting of oxo.

In some embodiments, provided in the present disclosure is the compound represented by formula (VI), (VI-1), (VI-2), (VI-1-A), or (VI-1-B) or the pharmaceutically acceptable salt thereof, wherein $R^5$, $R^6$, $R^7$, $R^8$, and $R^9$ are each independently selected from the group consisting of hydrogen and deuterium.

In some embodiments, provided in the present disclosure is the compound represented by formula (VI), (VI-1), (VI-2), (VI-1-A), or (VI-1-B) or the pharmaceutically acceptable salt thereof, wherein ring A is selected from the group consisting of pyrazolyl, imidazolyl, pyridyl, phenyl, tetrahydropyranyl, pyrimidinyl, and cyclohexyl.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of deuterium, $C_{1-6}$ alkyl, $C_{1-6}$ alkylene-3- to 6-membered cycloalkyl, 5- to 6-membered aryl, 5- to 6-membered heteroaryl, -NH(C=O)-O$C_{1-6}$ alkyl or -(C=O)NH-$C_{1-6}$ alkyl, and (C=O)$NH_2$, wherein

the $C_{1-6}$ alkyl, 5- to 6-membered aryl, or 5- to 6-membered heteroaryl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of $C_{1-6}$ alkyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of methyl, ethyl, and propyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of methyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of $C_{1-6}$ alkylene-3- to 6-membered cycloalkyl (e.g., cyclopropyl, cyclobutyl, cyclopentyl, or cyclohexyl);
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of methylene-cyclopropyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of 3- to 6-membered cycloalkyl (e.g., cyclopropyl, cyclobutyl, cyclopentyl, or cyclohexyl);
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of cyclopropyl;
n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of phenyl, wherein the phenyl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl;

n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of 5- to 6-membered heteroaryl (e.g., pyridine, imidazole, pyrazole, thiazole, or oxazole), wherein the 5- to 6-membered heteroaryl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl;

n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of -NH(C=O)-O$C_{1-6}$ alkyl (e.g., methyl, ethyl, n-propyl, isopropyl, n-butyl, or tert-butyl); n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of -(C=O)NH-$C_{1-6}$ alkyl (e.g., methyl, ethyl, *n*-propyl, isopropyl, *n*-butyl, or *tert*-butyl); n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of (C=O)$NH_2$; n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of 5- to 10-membered heterocycloalkyl, wherein the heterocycloalkyl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl;

n is selected from the group consisting of 1, 2, and 3.

In an optional embodiment, provided in the present disclosure is the compound represented by formula (VI), (VI-1), (VI-2), (VI-1-A), or (VI-1-B) or the pharmaceutically acceptable salt thereof, wherein $R^{10}$ is selected from the group consisting of 5- to 10-membered bridged-heterocyclyl, wherein the bridged-heterocyclyl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl; the heterocyclyl contains 1, 2, or 3 heteroatoms independently selected from the group consisting of nitrogen, oxygen, and sulfur;

n is selected from the group consisting of 1, 2, and 3.

In some embodiments, provided in the present disclosure is the compound represented by formula (VI), (VI-1), (VI-2), (VI-1-A), or (VI-1-B) or the pharmaceutically acceptable salt thereof, wherein

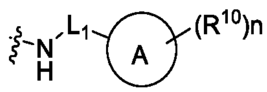

is selected from the group consisting of

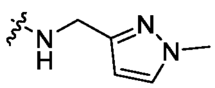 , 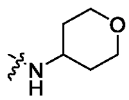 , 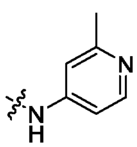 , 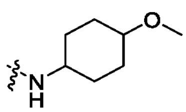 , 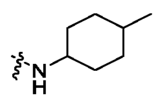 ,

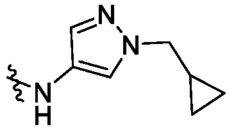 , 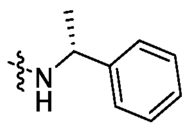 , 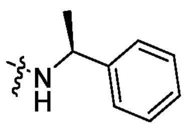 , 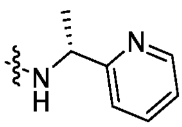 , 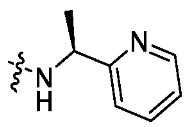 ,

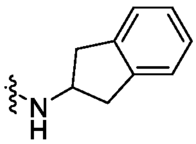, 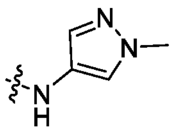, 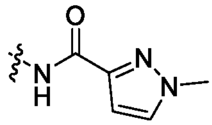, 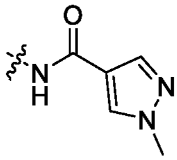, 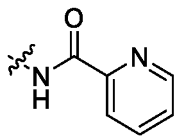,

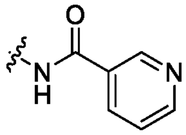, 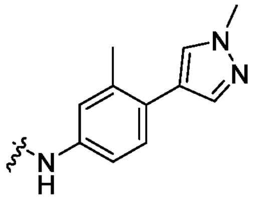, 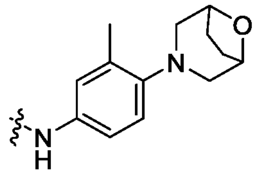, 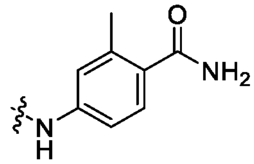,

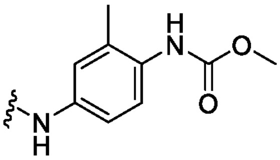, 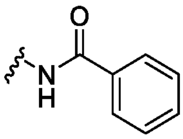, 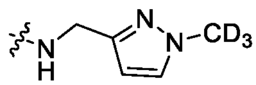, and 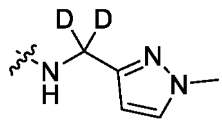.

In some embodiments, provided in the present disclosure is the compound represented by formula (VI), (VI-1), (VI-2), (VI-1-A), or (VI-1-B) or the pharmaceutically acceptable salt thereof, wherein each $R^{27}$ is independently selected from the group consisting of deuterium, halogen, $C_{1-6}$ alkyl, $C_{1-6}$ haloalkyl, $C_{2-6}$ alkynyl, $C_{1-6}$ alkylene-3- to 6-membered cycloalkyl, 3- to 6-membered cycloalkyl, $C_{1-6}$ haloalkoxy, -NH-$C_{1-6}$ alkyl, and -NH($C_{1-6}$ alkyl)$_2$.

In some embodiments, provided in the present disclosure is the compound represented by formula (VI), (VI-1), (VI-2), (VI-1-A), or (VI-1-B) or the pharmaceutically acceptable salt thereof, wherein each $R^{27}$ is independently selected from the group consisting of halogen and $C_{1-6}$ alkyl.

In some embodiments, provided in the present disclosure is the compound represented by formula (VI), (VI-1), (VI-2), (VI-1-A), or (VI-1-B) or the pharmaceutically acceptable salt thereof, wherein each $R^{27}$ is independently selected from the group consisting of fluorine and chlorine.

In some embodiments, provided in the present disclosure is the compound represented by formula (VI), (VI-1), (VI-2), (VI-1-A), or (VI-1-B) or the pharmaceutically acceptable salt thereof, wherein each $R^{27}$ is independently selected from the group consisting of methyl.

In some embodiments, provided in the present disclosure is the compound represented by formula (VI), (VI-1), (VI-2), (VI-1-A), or (VI-1-B) or the pharmaceutically acceptable salt thereof, wherein $R^{25}$ and $R^{26}$ are each independently selected from the group consisting of hydrogen, methyl, ethyl, *n*-propyl, isopropyl, *n*-butyl, isobutyl, and *tert*-butyl, and the methyl, ethyl, *n*-propyl, isopropyl, *n*-butyl, isobutyl, or *tert*-butyl is optionally substituted with one or more deuterium atoms.

In some embodiments, provided in the present disclosure is the compound represented by formula (VI), (VI-1), (VI-2), (VI-1-A), or (VI-1-B) or the pharmaceutically acceptable salt thereof, wherein $R^{25}$ and $R^{26}$ are each independently selected from the group consisting of hydrogen, methyl, and ethyl, and the methyl or ethyl is optionally substituted with one or more deuterium atoms.

In some embodiments, provided in the present disclosure is the compound represented by formula (VI), (VI-1), (VI-2), (VI-1-A), or (VI-1-B) or the pharmaceutically acceptable salt thereof, wherein $R^{25}$ and $R^{26}$ are each independently selected from the group consisting of methyl, and the methyl is optionally substituted with one or more deuterium atoms.

The present disclosure provides the following compounds or pharmaceutically acceptable salts thereof selected from the group consisting of:

Table a

| Group A: |
| --- |

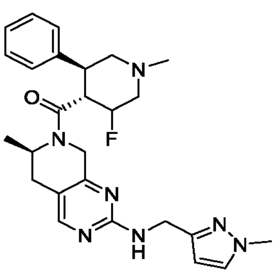,
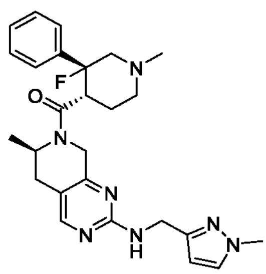,
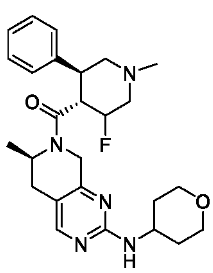,
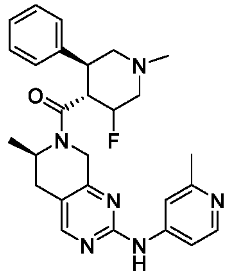,

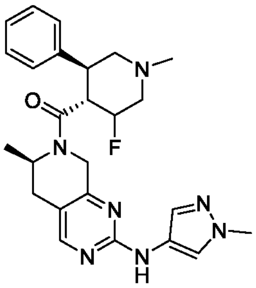,
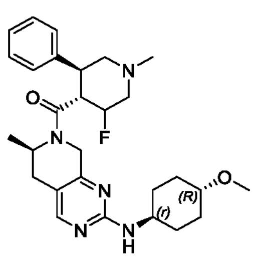,
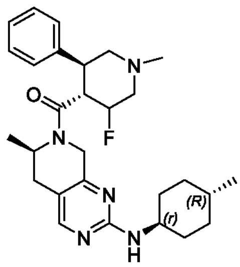,
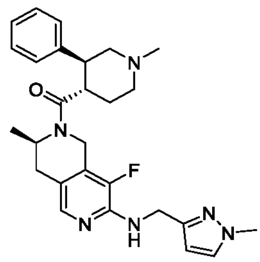,

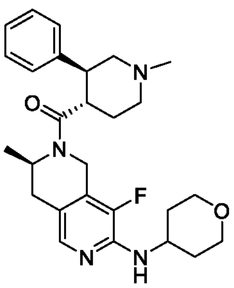,
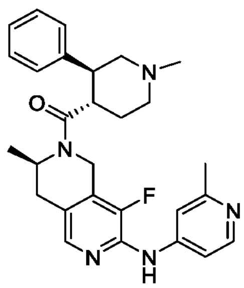,
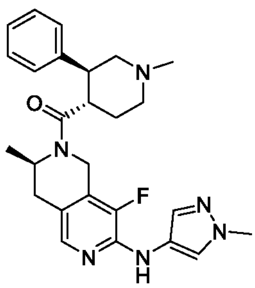,
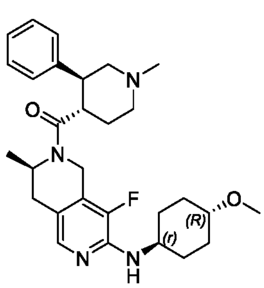,

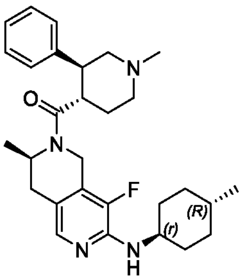,
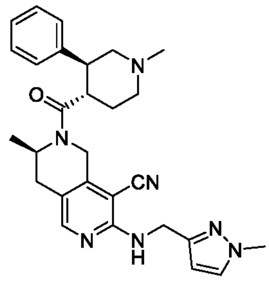,
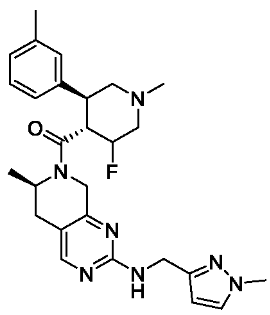,
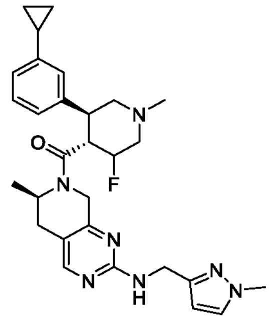,

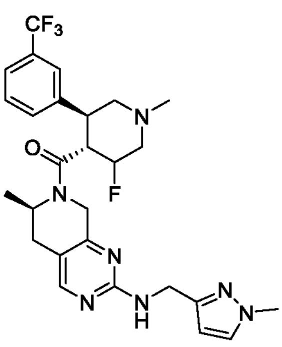,
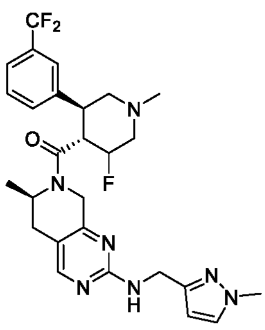,
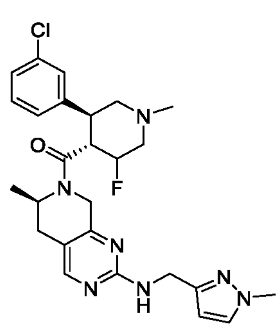,
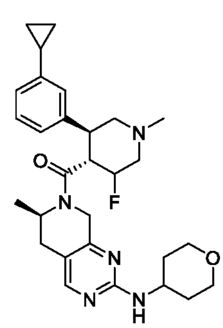,

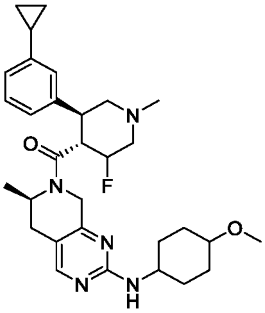,
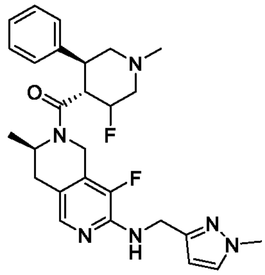,
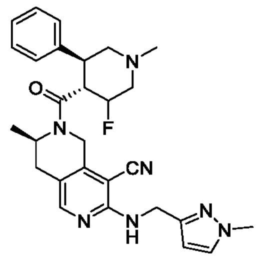,
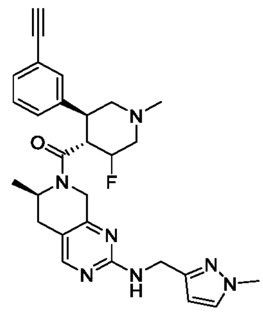,

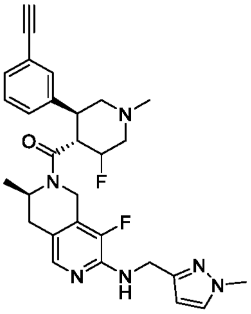,
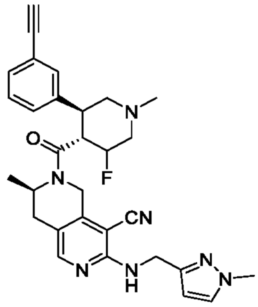,
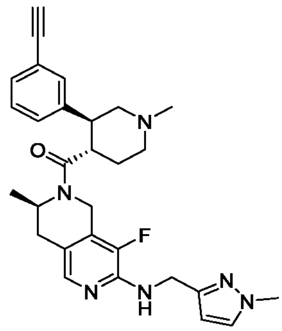,

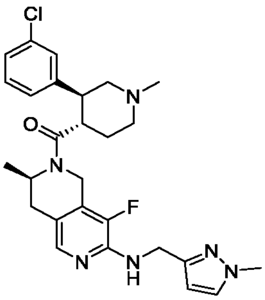,

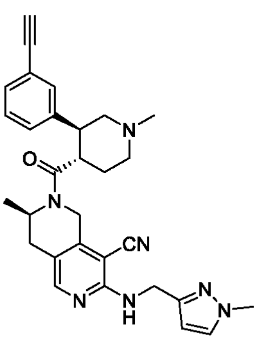,
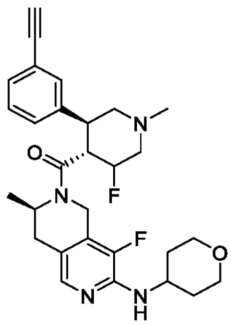,
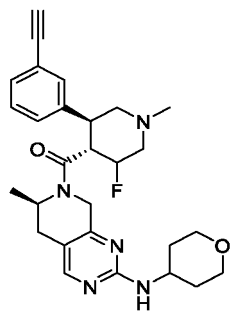,
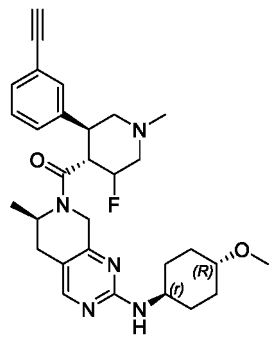,

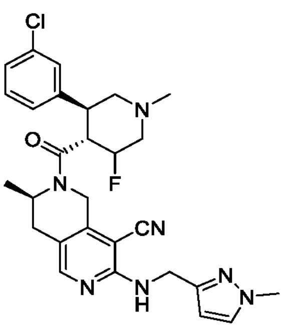,
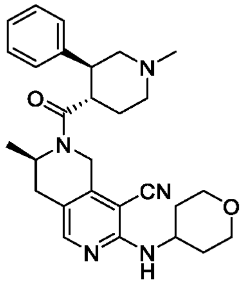,
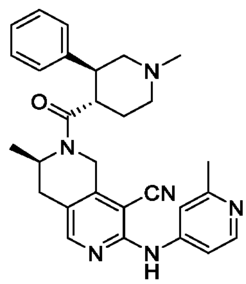,
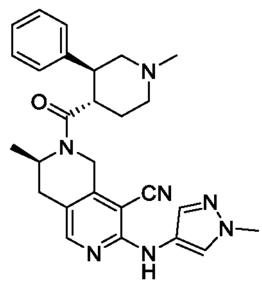,

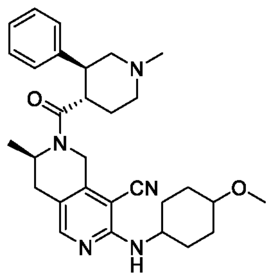,
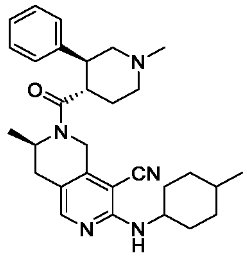,
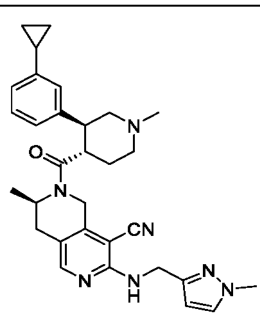,
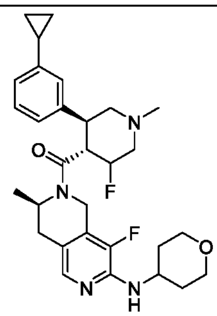,
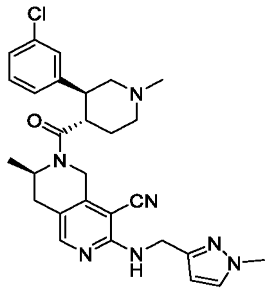,
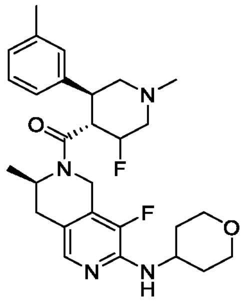,
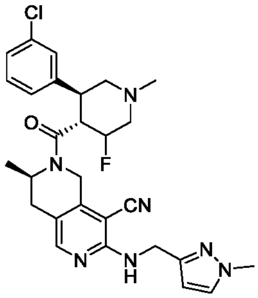
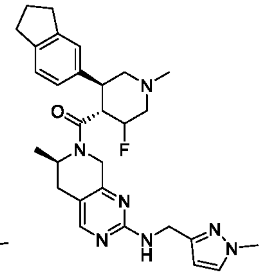,
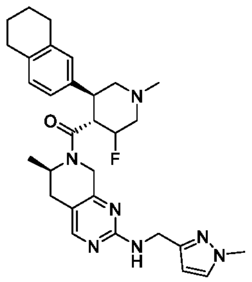,
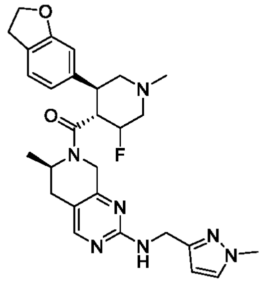,
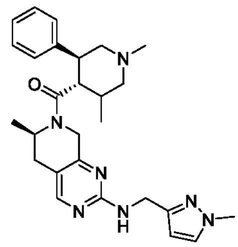,
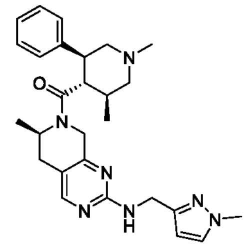,
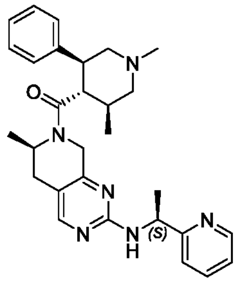,
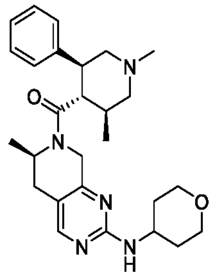, or
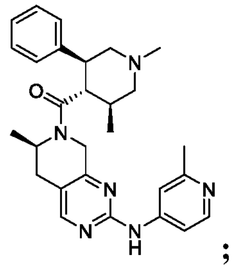;

Group B:

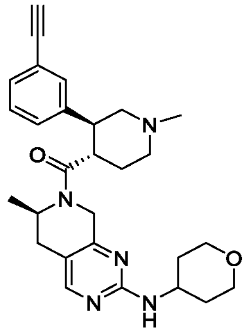,
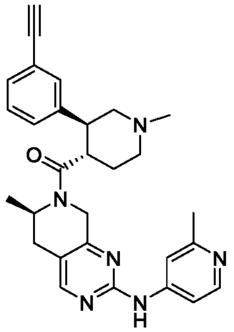,
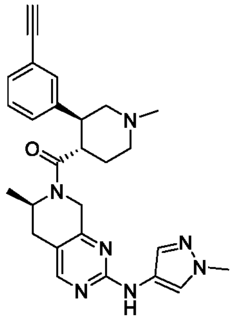,
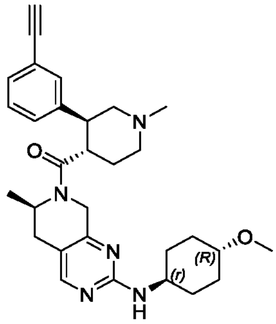,

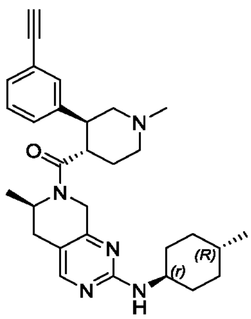,
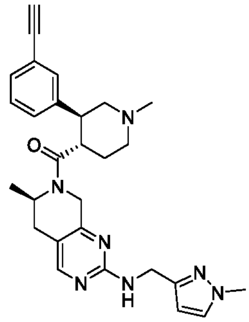,
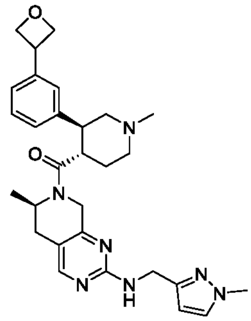,
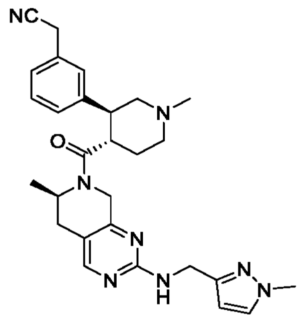,

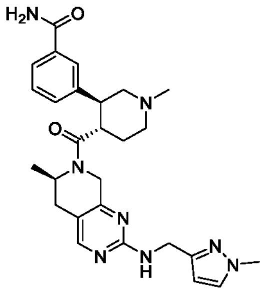,
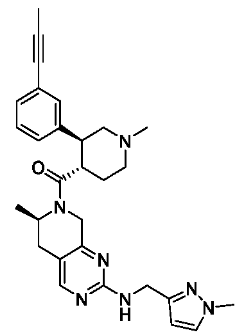,
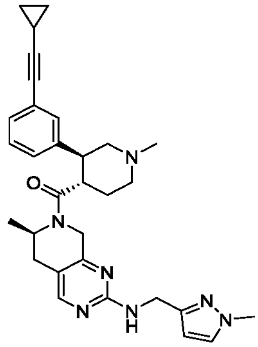,
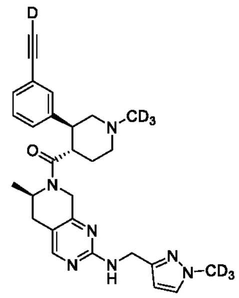 or

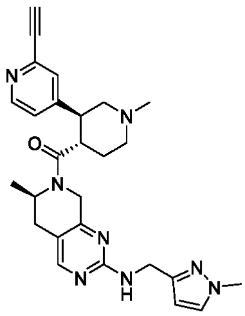,
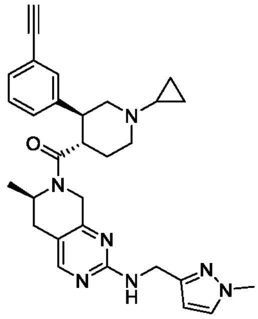,
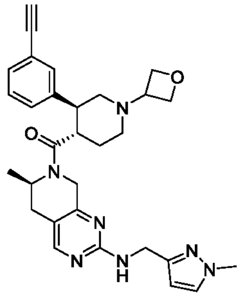, or
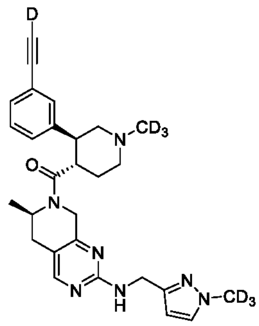;

Group C:

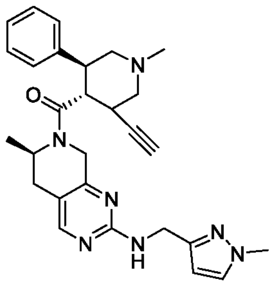 or
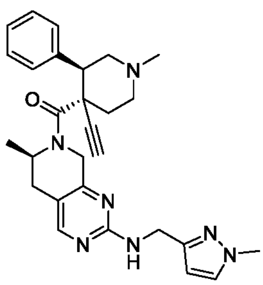;

Group D:

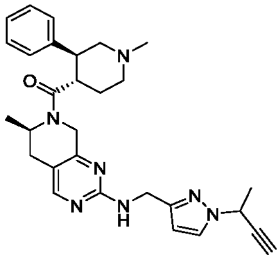, 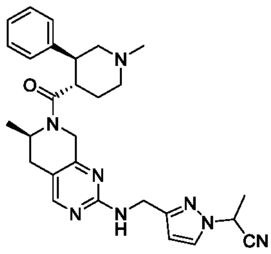, 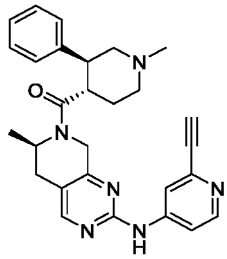, 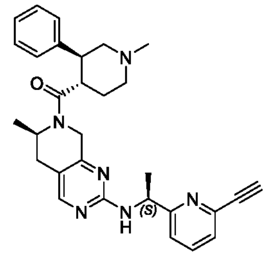,

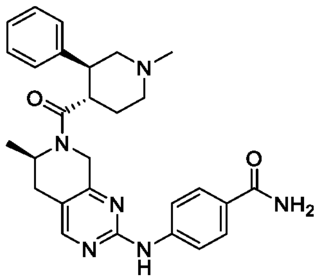, or 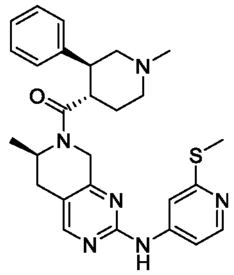;

Group E

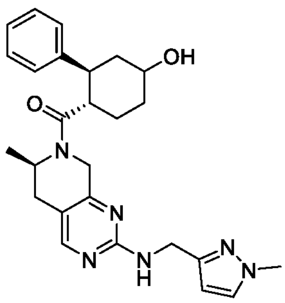, 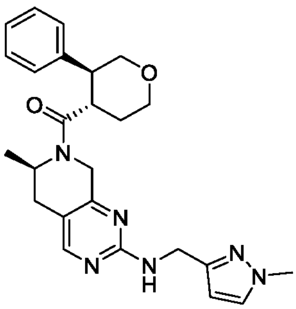, or 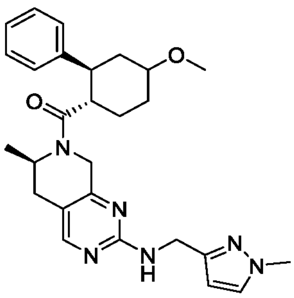;

Group F

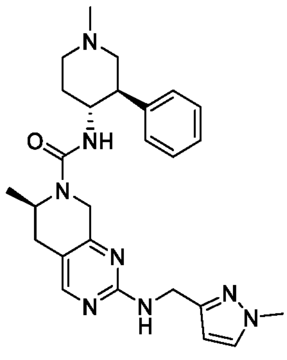, 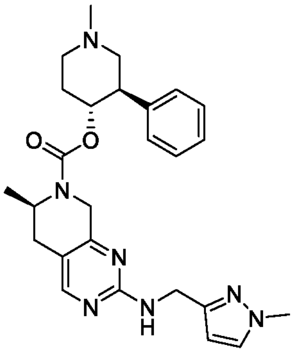, 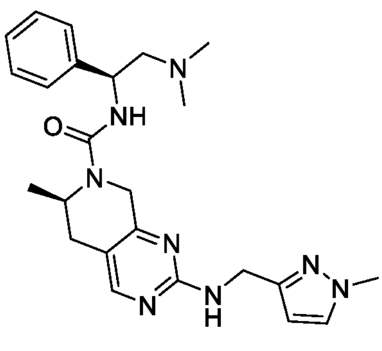,

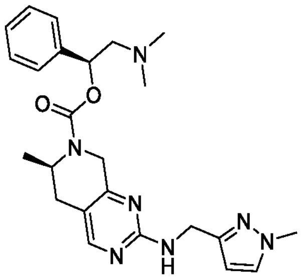, 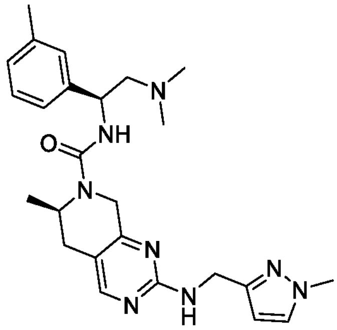, 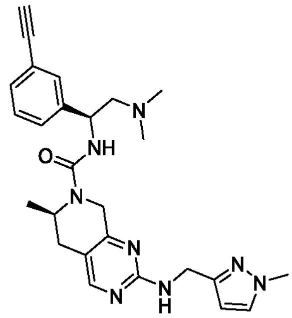,

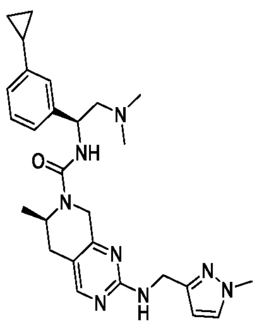, 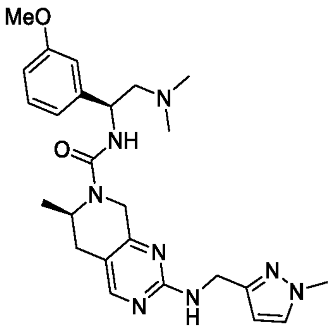, 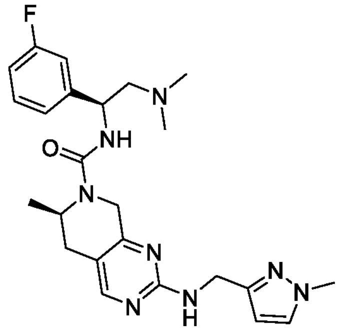,

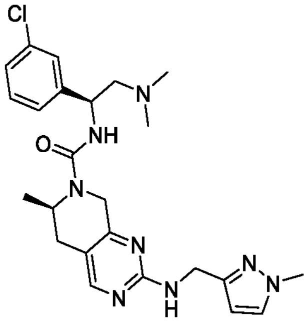, 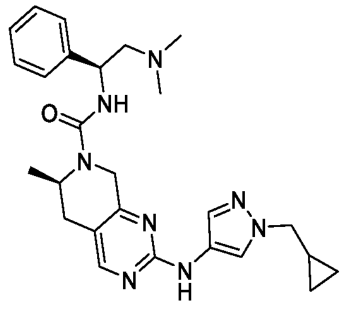, 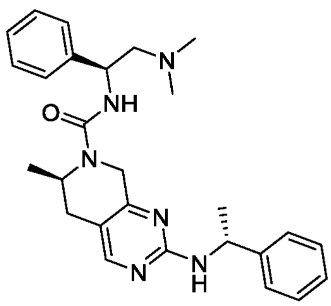,

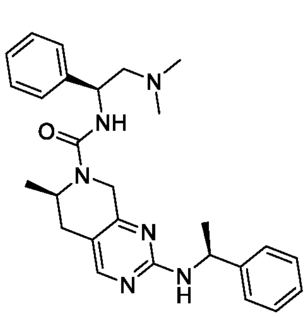, 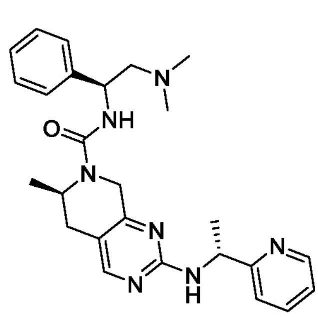, 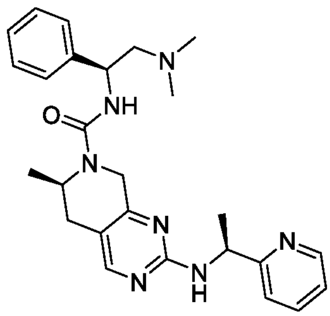,

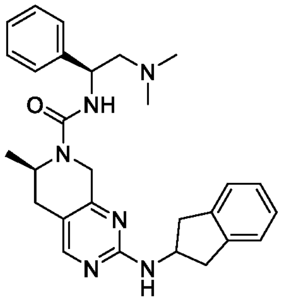, 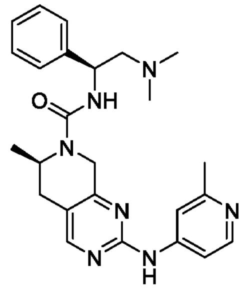, 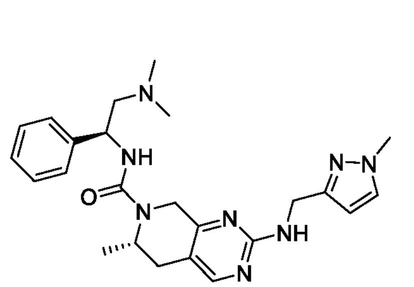,

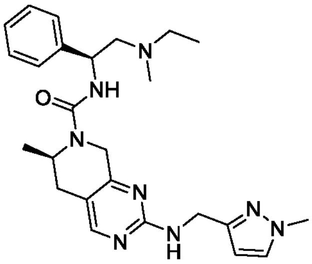,

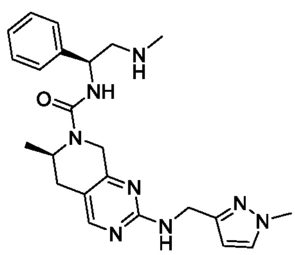,

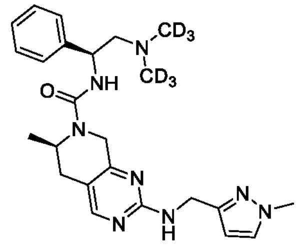,

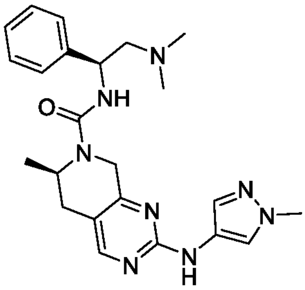,

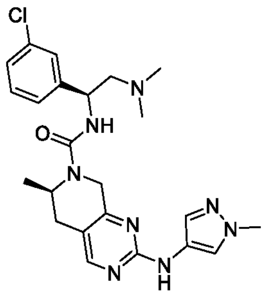,

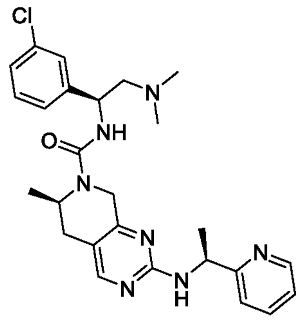,

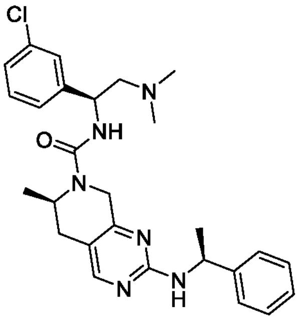,

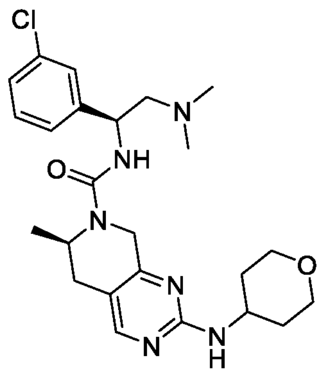,

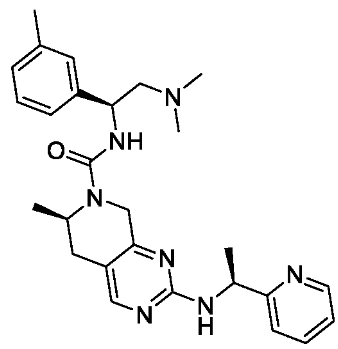,

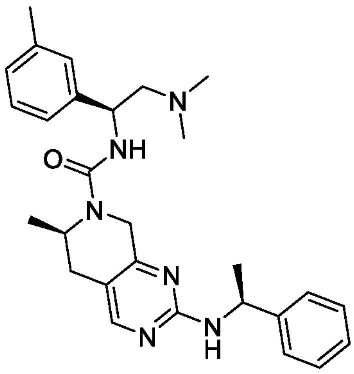,

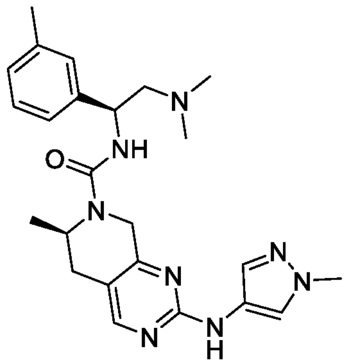,

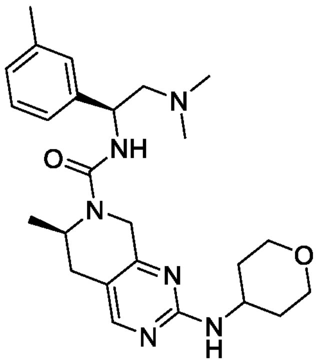,

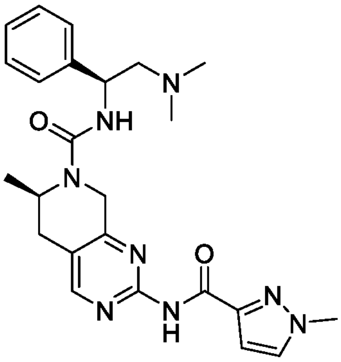,
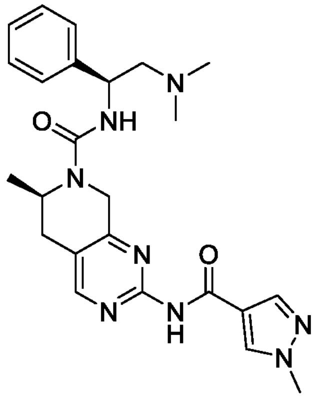,
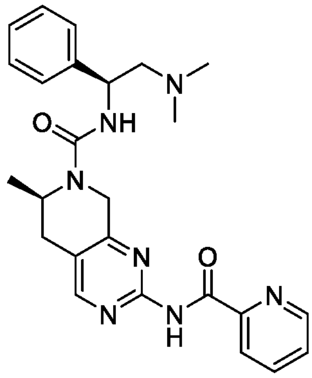,
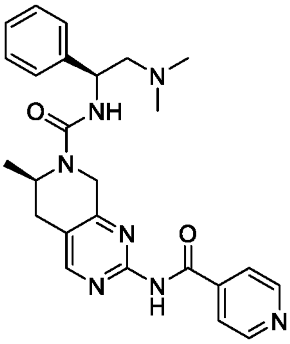,
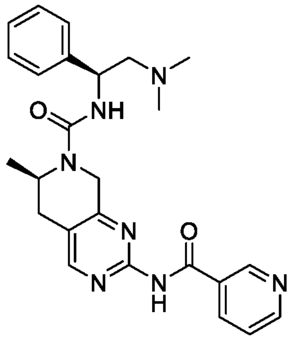,
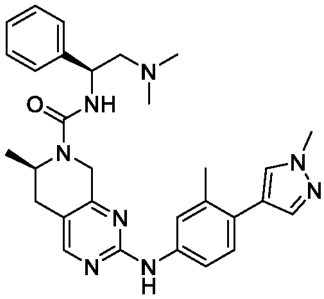,
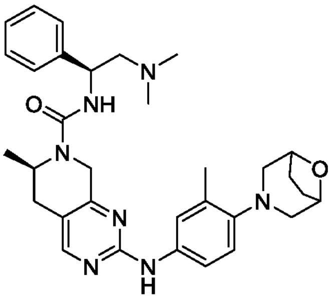,
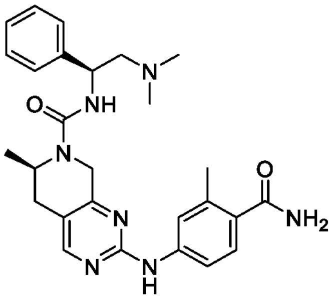,
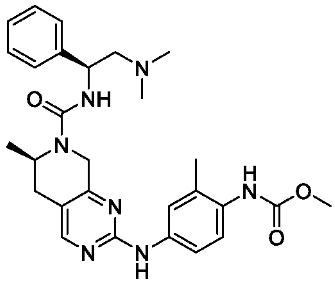,
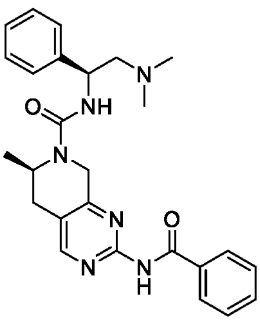,
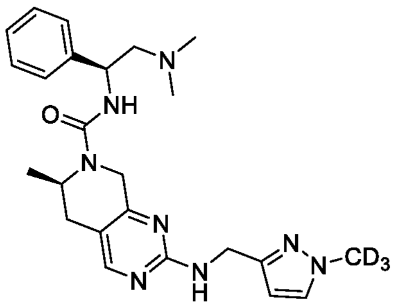,
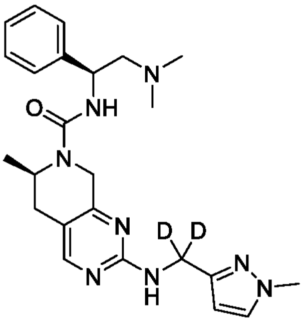,

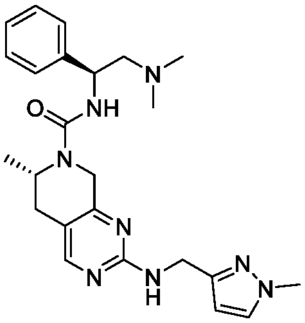,
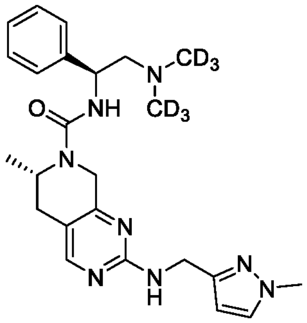,
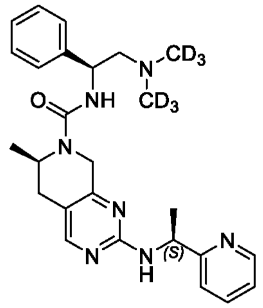,
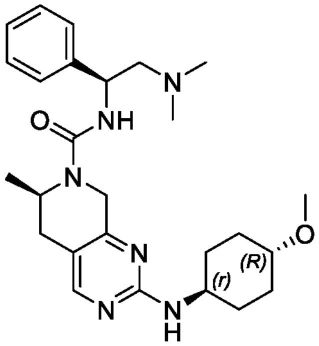,
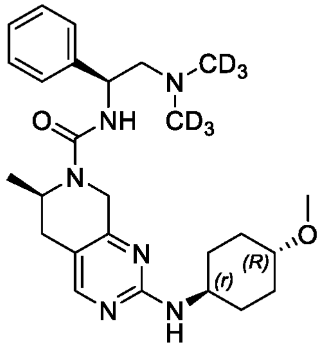,
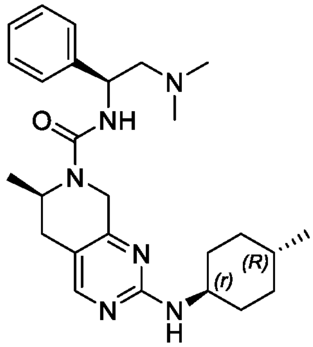,
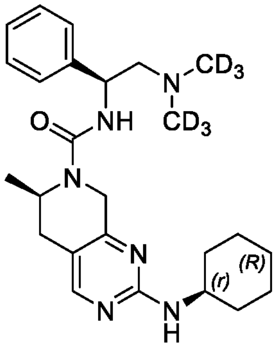,
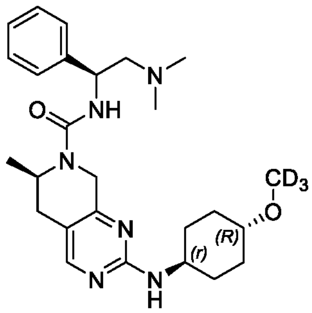,
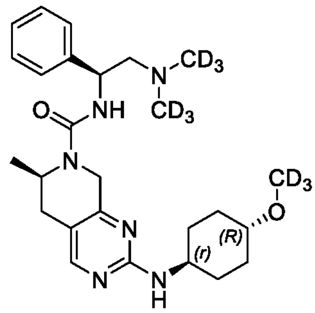,
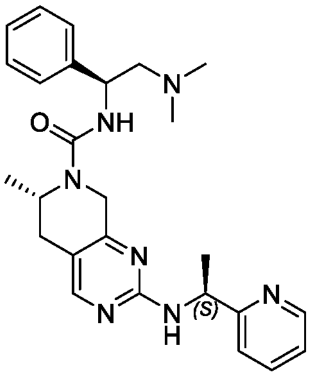,
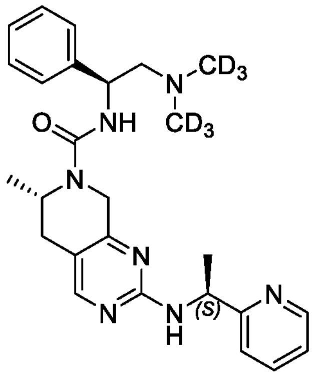,
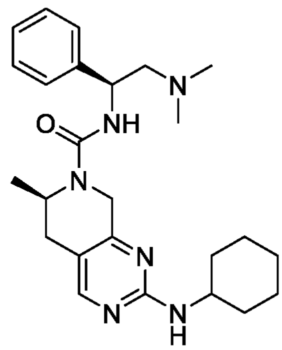, or

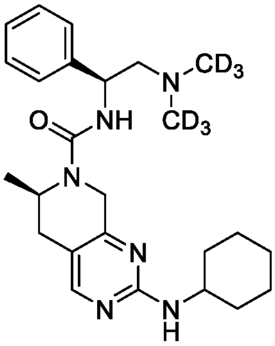
;

Group G

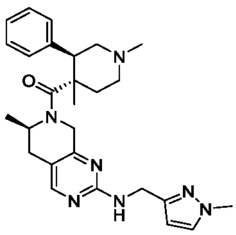
,
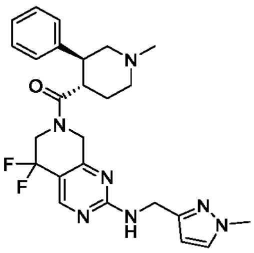
,
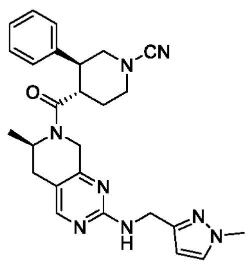
, or
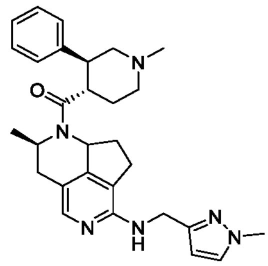
.

In another aspect, the present disclosure provides a compound represented by formula (I), (VI), (VI-1), (VI-2), (VI-1-A), or (VI-1-B) or a pharmaceutically acceptable salt thereof, a compound in Table a, and an isotopically substituted form. In an optional embodiment, the isotopically substituted form is obtained by substitution with a deuterium atom. The present disclosure also provides a pharmaceutical composition comprising at least one compound represented by formula (I), (VI), (VI-1), (VI-2), (VI-1-A), or (VI-1-B) or a pharmaceutically acceptable salt thereof, a compound in Table a, and an isotopically substituted form, or a compound prepared by the aforementioned method or a pharmaceutically acceptable salt thereof, and a pharmaceutically acceptable excipient. In some embodiments, a unit dose of the pharmaceutical composition is 0.001 mg-1000 mg.

In certain embodiments, the pharmaceutical composition comprises 0.01-99.99% of the aforementioned compound or pharmaceutically acceptable salt thereof based on the total weight of the composition. In certain embodiments, the pharmaceutical composition comprises 0.1-99.9% of the aforementioned compound or pharmaceutically acceptable salt thereof. In certain embodiments, the pharmaceutical composition comprises 0.5%-99.5% of the aforementioned compound or pharmaceutically acceptable salt thereof. In certain embodiments, the pharmaceutical composition comprises 1%-99% of the aforementioned compound or pharmaceutically acceptable salt thereof. In certain embodiments, the pharmaceutical composition comprises 2%-98% of the aforementioned compound or pharmaceutically acceptable salt thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure provides use of the aforementioned compound represented by formula (I), (VI), (VI-1), (VI-2), (VI-1-A), or (VI-1-B) or pharmaceutically acceptable salt thereof, compound in Table a, and isotopically substituted form, or a compound prepared by the aforementioned method or a pharmaceutically acceptable salt thereof, and the aforementioned pharmaceutical composition in the preparation of a medicament for treating or preventing a disease or disorder associated with abnormal activity of serine/threonine kinases.

In another aspect, the present disclosure provides use of the aforementioned compound represented by formula (I), (VI), (VI-1), (VI-2), (VI-1-A), or (VI-1-B) or pharmaceutically acceptable salt thereof, compound in Table a, and isotopically substituted form, or a compound prepared by the aforementioned method or a pharmaceutically acceptable salt thereof, and the aforementioned pharmaceutical composition in the preparation of a medicament for treating and/or

preventing a disease or disorder associated with abnormal activity of CDK7.

In an optional embodiment, the disease or disorder associated with abnormal activity of CDK7 is selected from the group consisting of a proliferative disease, an inflammatory disease, an autoinflammatory disease, an autoimmune disease, and an infectious disease. In another aspect, the present disclosure provides use of the aforementioned compound represented by formula (I), (VI), (VI-1), (VI-2), (VI-1-A), or (VI-1-B) or pharmaceutically acceptable salt thereof, compound in Table a, and isotopically substituted form, or a compound prepared by the aforementioned method or a pharmaceutically acceptable salt thereof, and the aforementioned pharmaceutical composition in the preparation of a medicament for treating and/or preventing a disease or disorder, wherein the disease or disorder is selected from the group consisting of a proliferative disease, an inflammatory disease, an autoinflammatory disease, an autoimmune disease, and an infectious disease.

In an optional embodiment, the disease or disorder is a proliferative disease.

In an optional embodiment, the proliferative disease is cancer.

In an optional embodiment, the cancer is selected from the group consisting of a hematological tumor and a solid tumor.

In an optional embodiment, the hematological tumor is selected from the group consisting of leukemias, specifically including: chronic lymphocytic leukemia (CLL), acute lymphocytic leukemia (ALL), T-cell acute lymphocytic leukemia (T-ALL), chronic myelogenous leukemia (CML), acute myeloid leukemia (AML), and the like.

In an optional embodiment, the solid tumor is selected from the group consisting of breast cancer, intestinal cancer, lung cancer, pancreatic cancer, prostate cancer, Ewing's sarcoma, osteoma, neuroblastoma, cervical cancer, ovarian cancer, gastric cancer, and liver cancer.

In an optional embodiment, the breast cancer is triple-negative breast cancer.

In an optional embodiment, the breast cancer is ER/PR+ HER2- breast cancer.

In an optional embodiment, the breast cancer is ER/PR+ HER2- breast cancer resistant to a CDK4/6 inhibitor.

In an optional embodiment, the CDK4/6 inhibitor is palbociclib.

In an optional embodiment, the lung cancer is non-small cell lung cancer.

In an optional embodiment, the lung cancer is small cell lung cancer.

In an optional embodiment, the intestinal cancer is colon cancer.

In an optional embodiment, the intestinal cancer is rectal cancer.

In another aspect, the present disclosure provides a method for treating and/or preventing a disease or disorder associated with abnormal activity of serine/threonine kinases in a patient, the method comprising administering to the patient a therapeutically effective amount of a compound represented by formula (I), (VI), (VI-1), (VI-2), (VI-1-A), or (VI-1-B) or a pharmaceutically acceptable salt thereof, a compound in Table a, and an isotopically substituted form, or a compound prepared by the aforementioned method or a pharmaceutically acceptable salt thereof, and the aforementioned pharmaceutical composition.

In another aspect, the present disclosure provides a method for treating and/or preventing a disease or disorder associated with abnormal activity of CDK7 in a patient, the method comprising administering to the patient a therapeutically effective amount of a compound represented by formula (I), (VI), (VI-1), (VI-2), (VI-1-A), or (VI-1-B) or a pharmaceutically acceptable salt thereof, a compound in Table a, and an isotopically substituted form, or a compound prepared by the aforementioned method or a pharmaceutically acceptable salt thereof, and the aforementioned pharmaceutical composition.

In an optional embodiment, the disease or disorder associated with abnormal activity of CDK7 is selected from the group consisting of a proliferative disease, an inflammatory disease, an autoinflammatory disease, an autoimmune disease, and an infectious disease. In another aspect, the present disclosure provides a method for treating and/or preventing a disease or disorder in a patient, the method comprising administering to the patient a therapeutically effective amount of a compound represented by formula (I), (VI), (VI-1), (VI-2), (VI-1-A), or (VI-1-B) or a pharmaceutically acceptable salt thereof, a compound in Table a, and an isotopically substituted form, or a compound prepared by the aforementioned method or a pharmaceutically acceptable salt thereof, and the aforementioned pharmaceutical composition, wherein the disease or disorder is selected from the group consisting of a proliferative disease, an inflammatory disease, an autoinflammatory disease, an autoimmune disease, and an infectious disease; in an optional embodiment, the disease or disorder is a proliferative disease.

In an optional embodiment, the proliferative disease is cancer.

In an optional embodiment, the cancer is selected from the group consisting of a hematological tumor and a solid tumor.

In an optional embodiment, the hematological tumor is selected from the group consisting of leukemias, specifically including: chronic lymphocytic leukemia (CLL), acute lymphocytic leukemia (ALL), T-cell acute lymphocytic leukemia (T-ALL), chronic myelogenous leukemia (CML), acute myeloid leukemia (AML), and the like.

In an optional embodiment, the solid tumor is selected from the group consisting of breast cancer, intestinal cancer, lung cancer, pancreatic cancer, prostate cancer, Ewing's sarcoma, osteoma, neuroblastoma, cervical cancer,

ovarian cancer, gastric cancer, and liver cancer.

In an optional embodiment, the breast cancer is triple-negative breast cancer.

In an optional embodiment, the breast cancer is ER/PR+ HER2- breast cancer.

In an optional embodiment, the breast cancer is ER/PR+ HER2- breast cancer resistant to a CDK4/6 inhibitor.

In an optional embodiment, the CDK4/6 inhibitor is palbociclib.

In an optional embodiment, the lung cancer is non-small cell lung cancer.

In an optional embodiment, the lung cancer is small cell lung cancer.

In an optional embodiment, the intestinal cancer is colon cancer.

In an optional embodiment, the intestinal cancer is rectal cancer.

In another aspect, the present disclosure provides use of a compound represented by formula (I), (VI), (VI-1), (VI-2), (VI-1-A), or (VI-1-B) or a pharmaceutically acceptable salt thereof, a compound in Table a, and an isotopically substituted form, or a compound prepared by the aforementioned method for use as a medicament.

The compounds or the pharmaceutically acceptable salts thereof provided in the present disclosure can be prepared using synthesis methods common in the art, and exemplary methods are provided below:
The compound represented by formula (VI) or the pharmaceutically acceptable salt thereof provided in the present disclosure may be prepared by referring to the method in WO 2022064009 A1.

The compound represented by formula (VI) provided in the present disclosure is resolved into 2 fragments, namely A and B, during the synthesis process,

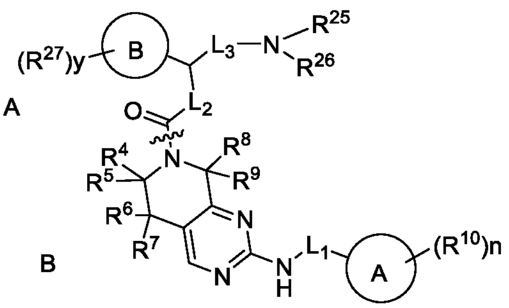

(VI)

.

The present disclosure provides a preparation method for the compound represented by formula (VI-1-A) or the pharmaceutically acceptable salt thereof, or the isotopically substituted form thereof, the preparation method comprising a step of subjecting formula (VI-1-A-01) or a pharmaceutically acceptable salt thereof to a condensation reaction with formula (VI-1-A-02) or a pharmaceutically acceptable salt thereof under the action of carbonyldiimidazole, phosgene, or triphosgene,

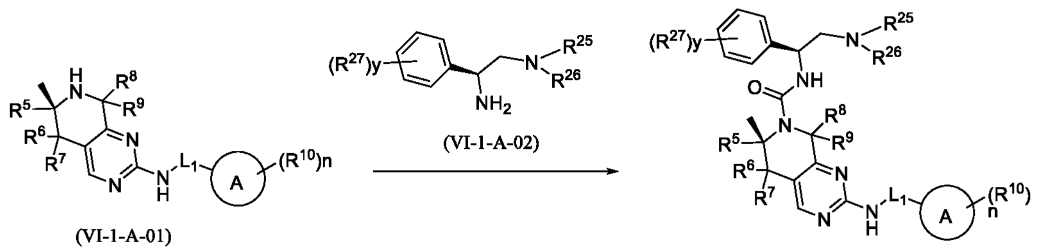

(VI-1-A-01) (VI-1-A-02) (VI-1-A)

,

wherein $R^5$, $R^6$, $R^7$, $R^8$, $R^9$, $L_1$, ring A, $R^{10}$, n, $R^{25}$, $R^{26}$, $R^{27}$, and y are each as defined in the foregoing.

In an optional embodiment, the condensation reaction takes place in an alkaline environment provided by an inorganic base (sodium hydroxide) or an organic base (e.g., triethylamine, pyridine, piperidine, or N,N-diisopropylethylamine), and the solvent in which the reaction takes place is a common solvent (e.g., DMF, DCM, or DMSO).

The present disclosure provides a preparation method for the compound represented by formula (VI-1-B) or the pharmaceutically acceptable salt thereof, or the isotopically substituted form thereof, the preparation method comprising a step of subjecting formula (VI-1-B-01) or a pharmaceutically acceptable salt thereof to a condensation reaction with formula (VI-1-A-02) or a pharmaceutically acceptable salt thereof under the action of carbonyldiimidazole, phosgene, or triphosgene,

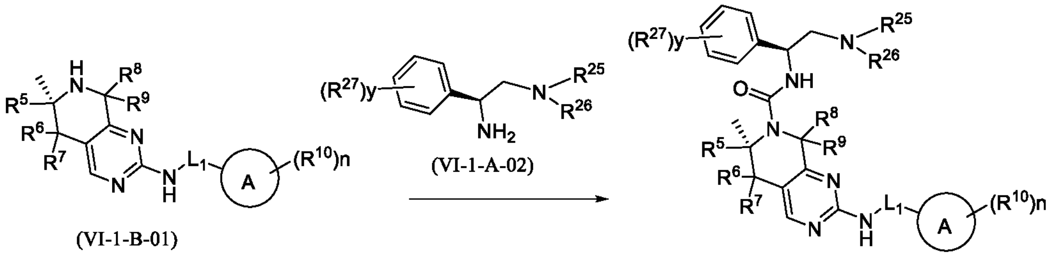

(VI-1-B-01) (VI-1-A-02) (VI-1-B)

wherein $R^5$, $R^6$, $R^7$, $R^8$, $R^9$, $L_1$, ring A, $R^{10}$, n, $R^{25}$, $R^{26}$, $R^{27}$, and y are each as defined in the foregoing.

In an optional embodiment, the condensation reaction takes place in an alkaline environment provided by an inorganic base (sodium hydroxide) or an organic base (e.g., triethylamine, pyridine, piperidine, or N,N-diisopropylethylamine), and the solvent in which the reaction takes place is a common solvent (e.g., DMF, DCM, or DMSO).

The pharmaceutically acceptable salts of the compounds described in the present disclosure are selected from the group consisting of inorganic salts and organic salts. The compounds described in the present disclosure can react with acidic or basic substances to form corresponding salts.

The present disclosure provides a compound represented by formula (I), (VI), (VI-1), (VI-2), (VI-1-A), or (VI-1-B) or a pharmaceutically acceptable salt thereof, a compound in Table a, and an isotopically substituted form having excellent selectivity for CDK7 enzymatic activity; in an optional embodiment, the $IC_{50}$ is less than 100 nM; in an optional embodiment, the $IC_{50}$ is less than 50 nM; in an optional embodiment, the $IC_{50}$ is less than 30 nM; in an optional embodiment, the $IC_{50}$ is less than 15 nM.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures, and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms may be separated in optically active pure form or racemic form. The optically active pure form may be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

In the chemical structures of the compounds described in the present disclosure, a bond "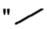" represents an unspecified configuration; that is, if chiral isomers exist in the chemical structures, the bond "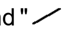" may be "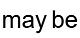" or "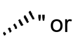", or both the configurations of "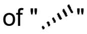" and "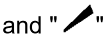" are included simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

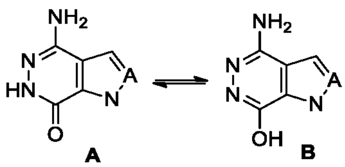

A B

All compounds in the present disclosure can be drawn as form A or form B. All tautomeric forms are within the

scope of the present disclosure. The names of the compounds do not exclude any tautomers.

The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, a solution of tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "$C_{1-6}$ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

Terms and definitions:

"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

"Effective amount" or "therapeutically effective amount" described in the present disclosure includes an amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

The prefix "$C_{u-v}$" indicates that the following group has from u to v carbon atoms. For example, "$C_{1-6}$ alkyl" indicates that the alkyl group has 1 to 6 carbon atoms. Specifically, it may be an alkyl group having 1, 2, 3, 4, 5, or 6 carbon atoms.

The term "alkyl" refers to an unbranched or branched saturated hydrocarbon chain. As used herein, alkyl has 1 to 20 carbon atoms (i.e., $C_{1-20}$ alkyl), 1 to 8 carbon atoms (i.e., $C_{1-8}$ alkyl), 1 to 6 carbon atoms (i.e., $C_{1-6}$ alkyl), or 1 to 4 carbon atoms (i.e., $C_{1-4}$ alkyl). Examples of alkyl groups include methyl, ethyl, propyl, isopropyl, *n*-butyl, *sec*-butyl, isobutyl, *tert*-butyl, pentyl, 2-pentyl, isopentyl, neopentyl, hexyl, 2-hexyl, 3-hexyl, and 3-methylpentyl. When an alkyl residue having a specific number of carbon atoms is named by a chemical name or identified by a molecular formula, all positional isomers having that number of carbon atoms may be included; thus, for example, "butyl" includes *n*-butyl (i.e., $-(CH_2)_3CH_3$), *sec*-butyl (i.e., $-CH(CH_3)CH_2CH_3$), isobutyl (i.e., $-CH_2CH(CH_3)_2$), and *tert*-butyl (i.e., $-C(CH_3)_3$); and "propyl" includes *n*-propyl (i.e., $-(CH_2)_2CH_3$) and isopropyl (i.e., $-CH(CH_3)_2$).

The term "alkenyl" refers to an alkyl group containing at least one carbon-carbon double bond and having 2 to 20 carbon atoms (i.e., $C_{2-20}$ alkenyl), 2 to 8 carbon atoms (i.e., $C_{2-8}$ alkenyl), 2 to 6 carbon atoms (i.e., $C_{2-6}$ alkenyl), or 2 to 4 carbon atoms (i.e., $C_{2-4}$ alkenyl). Examples of alkenyl groups include ethenyl, propenyl, and butadienyl (including 1,2-butadienyl and 1,3-butadienyl).

The term "alkynyl" refers to an alkyl group containing at least one carbon-carbon triple bond and having 2 to 20 carbon atoms (i.e., $C_{2-20}$ alkynyl), 2 to 8 carbon atoms (i.e., $C_{2-8}$ alkynyl), 2 to 6 carbon atoms (i.e., $C_{2-6}$ alkynyl), or 2 to 4

carbon atoms (i.e., $C_{2-4}$ alkynyl). Examples of "alkynyl" groups include ethynyl, propynyl (e.g., 1-propynyl, 2-propynyl), 3-butynyl, pentynyl, hexynyl, and 1-methylpent-2-ynyl.

The term "cycloalkyl" or "carbocycle" refers to a saturated or partially unsaturated cyclic alkyl group having a single ring or multiple rings (including fused, bridged, and spiro ring systems). The term "cycloalkyl" includes cycloalkenyl (that is, the cyclic group has at least one double bond). As used herein, cyclic alkyl has 3 to 20 ring carbon atoms (i.e., $C_{3-20}$ cycloalkyl), 3 to 12 ring carbon atoms (i.e., $C_{3-12}$ cycloalkyl), 3 to 10 ring carbon atoms (i.e., $C_{3-10}$ cycloalkyl), 3 to 8 ring carbon atoms (i.e., $C_{3-8}$ cycloalkyl), or 3 to 7 ring carbon atoms (i.e., $C_{3-7}$ cycloalkyl), or 3 to 6 ring carbon atoms (i.e., $C_{3-6}$ cycloalkyl). Examples of cycloalkyl groups include cyclopropyl, cyclobutyl, cyclopentyl, and cyclohexyl, cyclohexenyl, and cyclohexadienyl. The cycloalkyl ring may be fused to an aryl or heteroaryl ring, wherein the ring attached to the parent structure is a cycloalkyl group; non-limiting examples include indanyl, tetrahydronaphthyl, benzocycloheptyl, etc.

The term "heterocyclyl" or "heterocycloalkyl" refers to a saturated or unsaturated cycloalkyl group having one or more ring heteroatoms independently selected from the group consisting of nitrogen, oxygen, sulfur, and phosphorus. The term "heterocycloalkyl" includes heterocycloalkenyl groups (i.e., heterocyclyl groups having at least one double bond), bridged-heterocyclyl groups, fused-heterocyclyl groups, and spiro-heterocyclyl groups. A heterocyclyl group may be a single ring or multiple rings, wherein the multiple rings may be fused, bridged, or spiro. Any non-aromatic ring containing at least one heteroatom is considered a heterocyclyl group, regardless of the attachment (i.e., can be bound through a carbon atom or a heteroatom). Further, the term heterocyclyl is intended to include any non-aromatic ring containing at least one heteroatom, and the ring may be fused to an aryl or heteroaryl ring, regardless of the attachment to the remainder of the molecule. As used herein, heterocyclyl has 3 to 20 ring atoms (i.e., 3- to 20-membered heterocyclyl), 3 to 12 ring atoms (i.e., 3- to 12-membered heterocyclyl), 3 to 10 ring atoms (i.e., 3- to 10-membered heterocyclyl), 3 to 8 ring atoms (i.e., 3- to 8-membered heterocyclyl), 3 to 7 ring atoms (i.e., 3- to 7-membered heterocyclyl), or 3 to 6 ring atoms (i.e., 3- to 6-membered heterocyclyl) and has 1 to 5 ring heteroatoms, 1 to 4 ring heteroatoms, 1 to 3 ring heteroatoms, 1 to 2 ring heteroatoms, or 1 ring heteroatom, and the ring heteroatoms are independently selected from the group consisting of nitrogen, sulfur, phosphorus, and oxygen. Examples of heterocyclyl groups include pyrrolidinyl, imidazolidinyl, oxetanyl, dioxolanyl, azetidinyl, tetrahydrofuranyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, and homopiperazinyl.

As used herein, the term "bridged-heterocyclyl" refers to a 4- to 10-membered cyclic moiety that is attached at two non-adjacent atoms of the heterocyclyl to one or more (e.g., 1 or 2) 4- to 10-membered cyclic moieties having at least one heteroatom, wherein each heteroatom is independently selected from the group consisting of nitrogen, oxygen, sulfur, and phosphorus. As used herein, "bridged-heterocyclyl" includes bicyclic and tricyclic ring systems. Further, as used herein, the term "spiro-heterocyclyl" refers to a ring system in which 3- to 10-membered heterocyclyl has one or more additional rings, wherein the one or more additional rings are 3- to 10-membered cycloalkyl or 3- to 10-membered heterocyclyl, and a single atom of the one or more additional rings is also an atom of the 3- to 10-membered heterocyclyl. Examples of spiro-heterocyclyl rings include bicyclic and tricyclic ring systems, such as 2-oxa-7-azaspiro[3.5]nonyl, 2-oxa-6-azaspiro[3.4]octyl, and 6-oxa-1-azaspiro[3.3]heptyl. Examples of fused heterocyclyl rings include, but are not limited to, 1,2,3,4-tetrahydroisoquinolinyl, 4,5,6,7-tetrahydrothieno[2,3-c]pyridyl, indolinyl, and isoindolinyl, wherein the heterocyclyl may be bound through any ring of the fused system.

Non-limiting examples of "heterocyclyl" groups include:

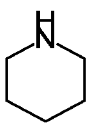 , 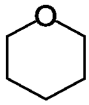 , 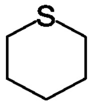 , 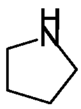 , 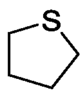 , 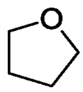 ,

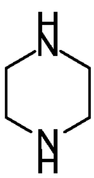 , 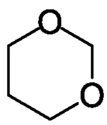 , 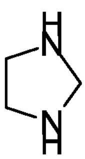 , 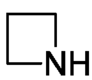 , 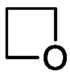 , 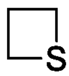 ,

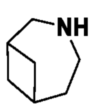 , 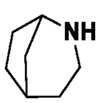 , 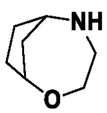 , 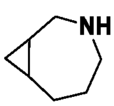 , 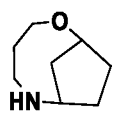 , 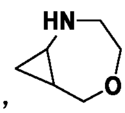

,

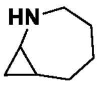, 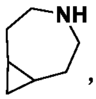, 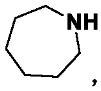, 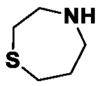, 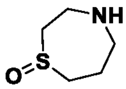, 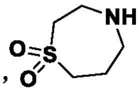,

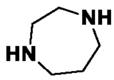, 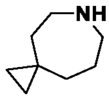, 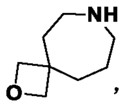, 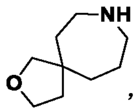, 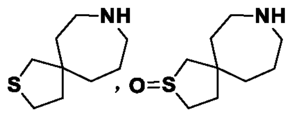,

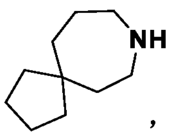, 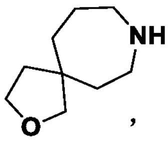, 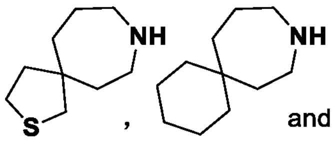 and 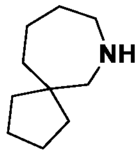,

etc.

The heterocyclyl ring may be fused to an aryl or heteroaryl ring, wherein the ring attached to the parent structure is heterocyclyl; its non-limiting examples include:

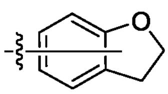, 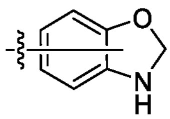, 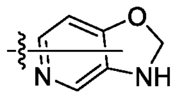, 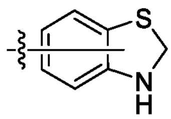,

etc.

The term "aryl" refers to a 6- to 14-membered, preferably 6- to 12-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocyclyl, or cycloalkyl ring, wherein the ring attached to the parent structure is the aryl ring; its non-limiting examples include:

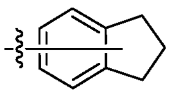, 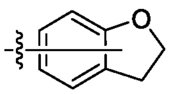, 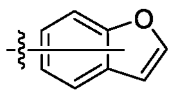, 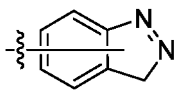, 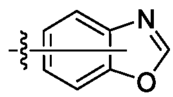,

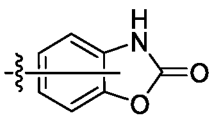, 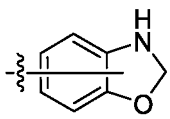, 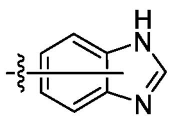, 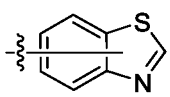, 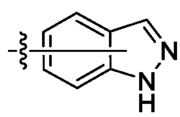,

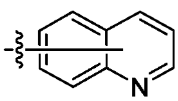, 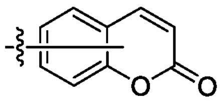, and 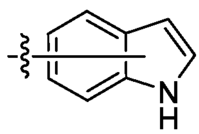.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5- or 6-membered. For example, its non-limiting examples include: imidazolyl, furyl, thienyl, thiazolyl, pyrazolyl, oxazolyl, pyrrolyl, tetrazolyl, pyridyl, pyrimidinyl, thiadiazole, pyrazine,

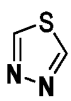, 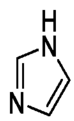, 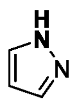,

etc.

The heteroaryl ring may be fused to an aryl, heterocyclyl, or cycloalkyl ring, wherein the ring attached to the parent structure is the heteroaryl ring; its non-limiting examples include:

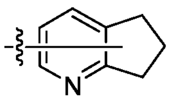 , 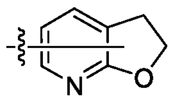 , 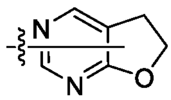 , 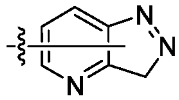 , 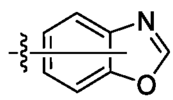 ,

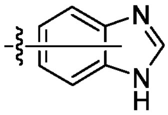 , 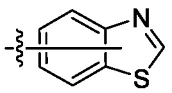 , 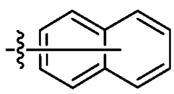 , and 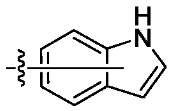 .

The term "alkoxy" refers to the group "alkyl-O-", wherein the alkyl is as defined above. Examples of alkoxy groups include methoxy, ethoxy, *n*-propoxy, isopropoxy, *n*-butoxy, *tert*-butoxy, *sec*-butoxy, *n*-pentoxy, *n*-hexoxy, and 1,2-dimethylbutoxy.

The terms "cycloalkyloxy" and "heterocyclyloxy" are defined in the same manner as the "alkoxy" described above.

The term "haloalkyl" refers to an unbranched or branched alkyl group as defined above, wherein one or more hydrogen atoms are replaced by halogen. For example, when a residue is substituted with more than one halogen, it may be referred to by using a prefix corresponding to the number of halogen moieties attached. Dihaloalkyl and trihaloalkyl refer to alkyl groups substituted with two or three halogen groups, respectively, which may be, but are not necessarily, the same halogen. Examples of haloalkyl groups include difluoromethyl ($-CHF_2$) and trifluoromethyl ($-CF_3$).

The term "haloalkoxy" refers to an alkoxy group as defined above, wherein one or more hydrogen atoms are replaced by halogen.

The term "hydroxyalkyl" refers to an alkyl group substituted with one or more hydroxy groups, wherein the alkyl is as defined above.

The term "hydroxy" refers to the -OH group.

The term "halogen" refers to fluorine, chlorine, bromine, or iodine.

The term "cyano" refers to -CN.

The term "nitro" refers to $-NO_2$.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

**DETAILED DESCRIPTION**

The present disclosure is further described below with reference to examples, but these examples are not intended to limit the scope of the present disclosure.

Examples

The structures of the compounds were determined by nuclear magnetic resonance (NMR) spectroscopy or/and mass spectrometry (MS). The NMR shifts (δ) are given in $10^{-6}$ (ppm). The NMR analyses were performed using a Bruker AVANCE-400 nuclear magnetic resonance instrument or Bruker AVANCE NEO 500M, with dimethyl sulfoxide-D6 (DMSO-$d_6$), chloroform-D (CDCl*3*), and methanol-D4 ($CD_3OD$) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector) and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and a Waters HPLC e2695-2489 high performance liquid chromatograph.

The chiral HPLC analyses were performed using an Agilent 1260 DAD high performance liquid chromatograph.

The preparative high performance liquid chromatography used Waters 2545-2767, Waters 2767-SQ Detecor2, and Shimadzu LC-20AP and Gilson GX-281 preparative chromatographs.

The preparative chiral chromatography used a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The silica gel column chromatography generally used 200-300 mesh Yantai Huanghai silica gel as the carrier.

The kinase mean inhibition rates and $IC_{50}$ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, J&K, Accela ChemBio Inc., Shanghai Bide Pharmatech, and Chembee Chemicals.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples used thin-layer chromatography (TLC). The developing solvent used in the reactions, the eluent systems used in the column chromatography purification, and the developing solvent systems used in the thin-layer chromatography include: A: a dichloromethane/methanol system, B: a *n*-hexane/ethyl acetate system, C: a petroleum ether/ethyl acetate system, and D: petroleum ether/ethyl acetate/methanol, and the volume ratio of the solvents were adjusted depending on the polarity of the compound, or a small amount of basic or acidic reagents such as triethylamine and acetic acid could be added for adjustment.

The abbreviations used in the experiments below have the following meanings:

TFA: trifluoroacetic acid; DCM: dichloromethane; *m*-CPBA: *meta*-chloroperoxybenzoic acid; EtONa: sodium ethoxide; Boc: *tert*-butoxycarbonyl; MeOH: methanol; HBTU: *N*,*N*,*N*',*N*'-tetramethyl-O-(1*H*-benzotriazol-1-yl)uronium hexafluorophosphate; TsOH: *p*-toluenesulfonic acid; EtOAc: ethyl acetate; *t*-BuOH: *tert*-butyl alcohol; $PdCl_2(TPP)_2$: bis(triphenylphosphine)palladium(II) dichloride; XantPhos: 4,5-bis(diphenylphosphino)-9,9-dimethylxanthene; $Pd_2dba_3$: tris(dibenzylideneacetone)dipalladium(0); $PdCl_2(TPP)_2$: bis(triphenylphosphine)palladium(II) dichloride; XantPhos: 4,5-bis(diphenylphosphino)-9,9-dimethylxanthene; $Pd_2dba_3$: tris(dibenzylideneacetone)dipalladium(0); DMF: *N,N*-dimethylformamide; CDI: *N*,*N*-carbonyldiimidazole; ACN: acetonitrile; DMP: Dess-Martin periodinane; DMAP: 4-dimethylaminopyridine.

Example **1** ((3*S*,4*S*)-3-(3-Ethynylphenyl)-1-methylpiperidin-4-yl)((*R*)-6-methyl-2-(((1-methyl-1*H*-pyrazol-3-yl)methyl)amino)-5,8-dihydropyrido[3,4-*d*]pyrimidin-7(6*H*)-yl)methanone; ((3*R*,4*R*)-3-(3-Ethynylphenyl)-1-methylpiperidin-4-yl)((*R*)-6-methyl-2-(((1-methyl-1*H*-pyrazol-3-yl)methyl)amino)-5,8-dihydropyrido[3,4-*d*]pyrimidin-7(6*H*)-yl)methanone

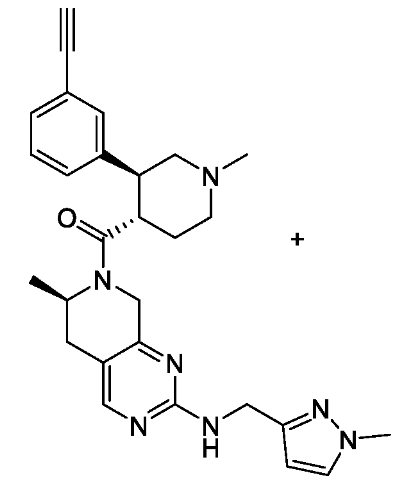

+

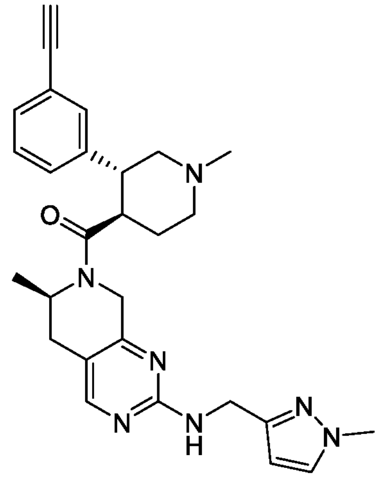

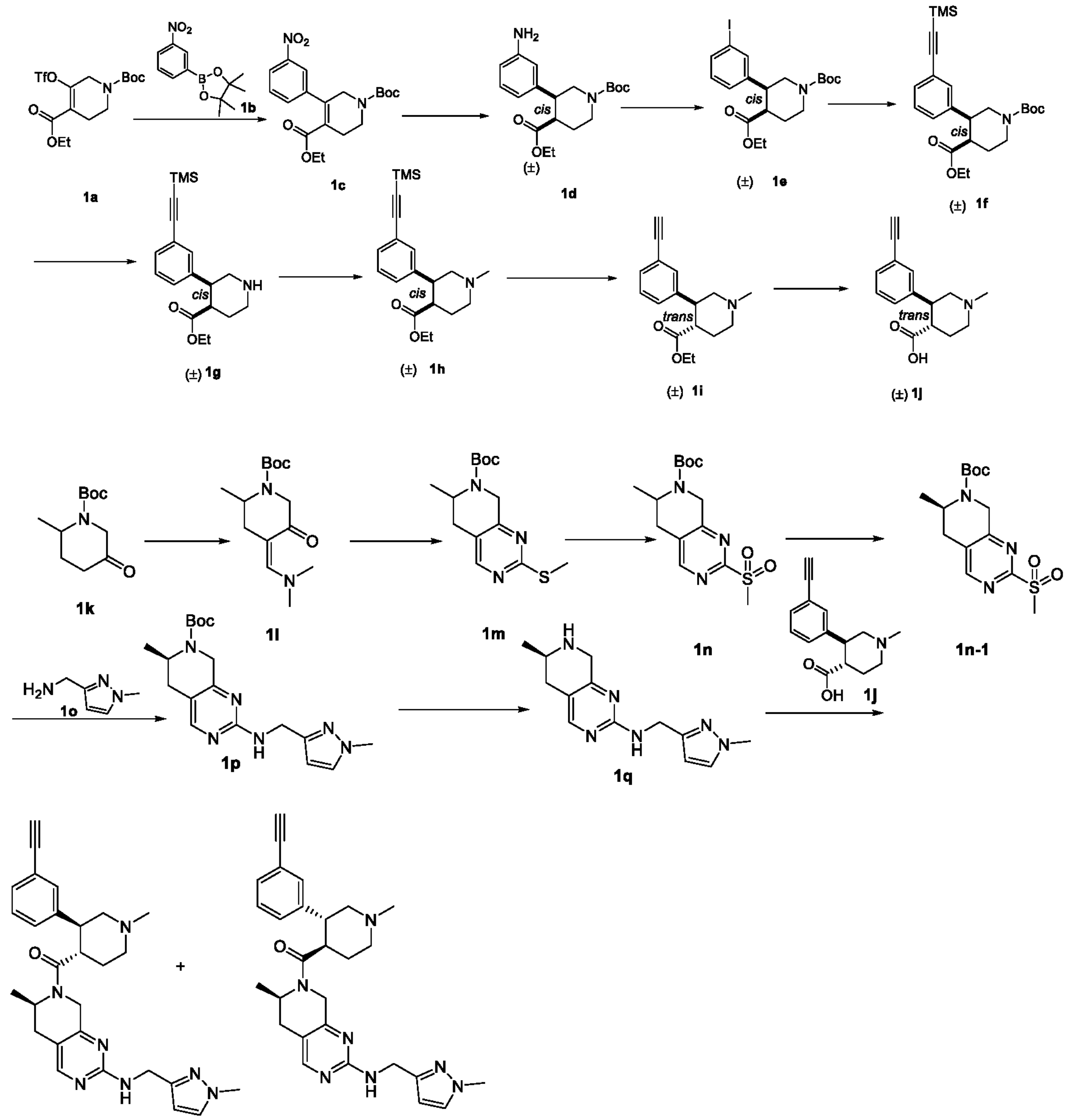

1a 1b 1c 1d 1e 1f 1g 1h 1i 1j 1k 1l 1m 1n 1n-1 1o 1p 1q 1j

+

Step 1

Preparation of compound **1c**

$Na_2CO_3$ (198 mL, 1 M, 198 mmol) and $Pd(PPh_3)_2Cl_2$ (6.96 g, 9.92 mmol) were added to a solution of compounds **1a** (40.0 g, 99.2 mmol) and **1b** (32.6 g, 149 mmol) in 1,4-dioxane (400 mL) under a $N_2$ atmosphere. The reaction mixture was heated to 90 °C and stirred for 12 h under a $N_2$ atmosphere, and then filtered. Water (150 mL) was added to the filtrate, followed by extraction with EtOAc (600 mL). The organic phases were combined, dried over $Na_2SO_4$, and filtered, and the resulting filtrate was concentrated under reduced pressure to give a crude product. The crude product was purified by silica gel flash column chromatography (eluent: 10-30% ethyl acetate in petroleum ether) to give compound **1c** (90.0 mg, yield: 98.1%).

$^1H$ NMR (400 MHz, DMSO-$d_6$) $\delta$ 7.11-6.97 (m, 1H), 6.57 (d, $J$ = 8.0 Hz, 1H), 6.41 (s, 1H), 6.34 (d, $J$ = 7.2 Hz, 1H), 5.15 (s, 2H), 4.09 (s, 2H), 3.92 (q, $J$ = 7.2 Hz, 2H), 2.44 (s, 2H), 1.49 (s, 9H), 0.92 (t, $J$ = 7.2 Hz, 3H).

Step 2

Preparation of compound **1d**

Compound **1c** (35.0 g, 101 mmol) and Pd/C (10%, 24.0 g) were added to a reaction flask containing EtOH (400 mL) at room temperature under a $N_2$ atmosphere. The reaction mixture was purged three times with $H_2$, stirred at 50 °C for 12 h under a $H_2$ (50 psi) atmosphere, and filtered, and the filtrate was concentrated to give **1d** (30.0 g, 86.1 mmol, 92.6%) as a crude product.

Step 3

Preparation of compound **1e**

An aqueous solution (7 mL) of TsOH (11.0 mL, 71.7 mmol) was added to a solution of **1d** (10.0 g, 28.7 mmol) in $CH_3CN$ (100 mL) at 0 °C. The reaction mixture was stirred for 0.5 h. The temperature of the reaction mixture was maintained at 0 °C, and an aqueous solution of $NaNO_2$ (2.30 mL, 43.0 mmol) was added to the reaction mixture. An aqueous solution (7.0 mL) of KI (2.35 mL, 43.0 mmol) was slowly added dropwise. After the addition, the reaction mixture was heated to room temperature, stirred for 16 h, and, after the addition of water, extracted with EtOAc (300 mL × 3). The organic phase was dried over $Na_2SO_4$ and filtered, and the filtrate was concentrated under reduced pressure to give a crude product.

The crude product was purified by silica gel flash column chromatography (eluent: 10-30% ethyl acetate in petroleum ether) to give compound **1e** (8.0 g, 17.4 mmol, yield: 60.7%).

Step 4

Preparation of compound **1f**

Compound 1e (3.00 g, 6.53 mmol), trimethylsilylacetylene (21.4 mL, 150 mmol), CuI (0.111 mL, 3.26 mmol), and $Pd(DTBPF)Cl_2$ (2.13 g, 3.266 mmol) were added to DMF (60 mL) under a $N_2$ atmosphere, and the reaction mixture was heated to 110 °C and stirred for 12 h. Water (260 mL) was added to the reaction mixture, followed by extraction with EtOAc (50 mL × 3). The organic phases were combined, dried over $Na_2SO_4$, and filtered, and the filtrate was concentrated under reduced pressure to give a crude product. The crude product was purified by silica gel flash column chromatography (eluent: 1-5% ethyl acetate in petroleum ether) to give compound **1f** (2.06 g, 2.88 mmol, yield: 44.1%).

Step 5

Preparation of compound **1g**

HCl/EtOAc (15 mL) was added to a solution of compound **1f** (1.00 g, 2.33 mmol) in EtOAc (20 mL) under a $N_2$ atmosphere, and the reaction mixture was stirred at room temperature for 2 h and concentrated under reduced pressure to give **1g** (900 mg) as a crude product. The crude **1g** was dissolved in DCM (60 mL), and NaOAc (0.305 mL, 3.28 mmol) and formaldehyde (399 mg, 4.92 mmol) were added to the solution. The mixture was stirred at room temperature for 1 h, and $NaBH(OAc)_3$ (1440.2 mg, 6.83 mmol) was added. The mixture was left to react at room temperature and stirred for 12 h. The reaction mixture was washed once with 10% $NaHCO_3$ (20 mL), dried over $Na_2SO_4$, and filtered, and the filtrate was concentrated under reduced pressure to give a crude product.

The crude product was separated and purified by preparative HPLC (column: Waters Xbridge BEH C18 250 × 50

mm × 10 μm; mobile phase: [water ($NH_4HCO_3$)-$CH_3CN$]; B%: 65%-90%) to give compound **1h** (400 mg, 1.07 mmol, yield: 39.2%).

MS m/z (ESI): 344.2 $[M+H]^+$.

Step 6

Preparation of intermediate **1j**

EtONa (0.456 mL, 5.822 mmol) was added to a solution of compound **1h** (400 mg, 1.164 mmol) in EtOH (30 mL) at room temperature. The reaction mixture was gradually heated to 85 °C, stirred for 5 h, and concentrated under reduced pressure to give **1i** as a crude product. The crude **1i** was added to a mixed solution of EtOH (15 mL) and $H_2O$ (15 mL) at room temperature, and NaOH (118 mg, 2.95 mmol) was added. The reaction mixture was stirred for 12 h and concentrated under reduced pressure to give a crude product. The crude product was separated and purified by preparative HPLC (column: Phenomenex Luna C18 75 × 30 mm × 3 μm; mobile phase: [water (FA)-$CH_3CN$]; B%: 65%-90%) to give compound **1j** (150 mg, 0.555 mmol, 37.6%).

MS m/z (ESI): 244.2 $[M+H]^+$.

Step 7

Preparation of compound **1l**

DMF-DMA (12.3 g, 103 mmol, 13.7 mL, 2.00 eq) was slowly added dropwise to a solution of compound **1k** (11.0 g, 51.6 mmol, 1.00 eq) in DMF (50 mL) at room temperature, and the reaction mixture was heated to 90 °C and stirred for 18 h. Water (150 mL) was added to the reaction mixture, followed by extraction with EtOAc (100 mL × 3). The organic phases were combined, dried over $Na_2SO_4$, and filtered, and the filtrate was concentrated under reduced pressure to give **1l** (12 g) as a crude product.

MS m/z (ESI): 269.1 $[M+H]^+$.

Step 8

Preparation of compound **1m**

EtONa (6.69 g, 98.4 mmol) was slowly added to a solution of compound **1l** (12.0 g, 44.7 mmol) and 2-methyl-2-thiopseudourea sulfate (24.9 g, 89.4 mmol, 2.00 eq) in EtOH (200 mL) at room temperature, and the reaction mixture was heated to 90 °C, stirred for 12 h, and then cooled to room temperature. $H_2O$ (150 mL) was added, followed by extraction with EtOAc (60 mL × 5). The organic phases were combined, dried over $Na_2SO_4$, and filtered, and the filtrate was concentrated under reduced pressure to give a crude product. The crude product was purified by silica gel flash column chromatography (eluent: 10-30% ethyl acetate in petroleum ether) to give compound **1m** (6.20 g, 21.0 mmol, yield: 46.9%).

MS m/z(ESI): 296.1 $[M+H]^+$.

Step 9

Preparation of intermediate **1n**

*m*-CPBA (10.3 g, 50.7 mmol) was added to a solution of compound **1m** (5.00 g, 17.0 mmol) in DCM (100 mL) at 0 °C, and the reaction mixture was stirred for 2 h, heated to room temperature, and stirred for 10 h. The reaction mixture was cooled to 0 °C, and $Na_2SO_3$ (10%, 40 mL) was added to the reaction mixture, followed by extraction with DCM (30 mL × 3). The organic phases were combined, washed once with saturated brine (50 mL), dried over $Na_2SO_4$, and filtered, and the filtrate was concentrated under reduced pressure to give a crude product.

The crude product was purified by silica gel flash column chromatography (eluent: 10-30% ethyl acetate in petroleum ether) to give compound **1n** (2.7 g, 8.25 mmol, yield: 48.7%).

MS m/z (ESI): 328.1 $[M+H]^+$.

$^1H$ NMR (400 MHz, $CDCl_3$) $\delta$ 8.59 (s, 1H), 5.08, 5.02 (m, 1H), 4.82 (s, 1H), 4.33-4.27 (m, 1H), 3.29 (s, 3H), 3.13-3.06 (m, 1H), 2.67-2.63 (m, 1H), 1.45 (s, 9H), 1.05-1.03 (m, 3H).

Compound **1n** was resolved on a chiral column (column: Chiralcel OD-3, 150 × 4.6 mm I.D., 3 μm; mobile phase: A: supercritical $CO_2$ fluid, B: methanol (0.1% IPAm, v/v)) to give **1n-1** and **1n-2.**

Compound **1n-1** (retention time: 2.770 min)

MS m/z (ESI): 328.1 $[M+H]^+$.

1H NMR (400 MHz, $CDCl_3$) $\delta$ 8.59 (s, 1H), 5.08-5.03 (m, 1H), 4.82 (br s, 1H), 4.32-4.27 (m, 1H), 3.29 (s, 3H), 3.13-3.06 (m, 1H), 2.67-2.63 (m, 1H), 1.43 (s, 9H), 1.05-1.03 (m, 3H).
Compound **1n-2** (retention time: 2.469 min).

Step 10

Preparation of intermediate **1p**

**1o** (380 mg, 3.42 mmol) was added to a solution of compound **1n-1** (140 mg, 0.428 mmol) in t-BuOH (2 mL) at room temperature. The reaction mixture was heated to 100 °C, stirred for 12 h, and concentrated under reduced pressure to give a crude product. The crude product was purified by silica gel flash column chromatography (eluent: 1-5% ethyl acetate in petroleum ether) to give compound **1p** (100 mg, 0.279 mmol, 45.7%).
MS m/z (ESI): 359.1 $[M+H]^+$.

Step 11

Preparation of compound **1**

TFA (0.8 mL, 10.8 mmol) was added to a solution of compound **1p** (100 mg, 0.279 mmol) in DCM at room temperature, and the mixture was stirred for 1 h. The reaction mixture was concentrated to dryness under reduced pressure to give **1q** (96.0 mg, 0.186 mmol, 66.6%) as a crude product.
MS (ESI): m/z =259.1 $[M+H]^+$.
Intermediate **1j** (90.0 mg, 0.37 mmol) was added to DMF (3 mL) at room temperature, and HBTU (421 mg, 1.11 mmol), DIEA (0.31 mL, 1.85 mmol), and the crude **1q** (95.6 mg, 0.370 mmol) were added to the reaction mixture. The reaction mixture was stirred at room temperature for 12 h and concentrated under reduced pressure to give compound 1 as a crude product. The crude product was separated and purified by preparative HPLC (column: Phenomenex Luna C18 75 × 30 mm × 3 µm; mobile phase: $[(NH_4HCO_3)\text{-}CH_3CN]$; B%: 65%-90%) to give compound **1-1** (17.4 mg, 0.036 mmol, 9.18%) and compound **1-2** (17.0 mg, 0.035 mmol, 9.50%).

Compound **1-1**

MS (ESI): m/z =484.3 $[M+H]^+$.
$^1H$ NMR (400 MHz, $CD_3OD$): $\delta$: 8.06-7.90 (m, 1H), 7.56-7.17 (m, 4H), 6.93-6.87 (m, 1H), 6.95-6.16 (m, 1H), 4.64-4.44 (m, 4H), 3.88-3.84 (m, 3H), 3.57-3.35 (m, 2H), 3.17-2.86 (m, 4H), 2.80-2.50 (m, 3H), 2.45-2.22 (m, 5H), 2.18-2.04 (m, 1H), 1.96-1.87 (m, 1H), 1.03-0.78 (m, 3H).

Compound **1-2**

MS m/z (ESI): 484.3 $[M+H]^+$.
$^1H$ NMR (400 MHz, $CD_3OD$): $\delta$: 8.06-7.92 (m, 1H), 7.55-7.17 (m, 5H), 6.25-6.15 (m, 1H), 4.73-4.51 (m, 4H), 3.90-3.83 (m, 4H), 3.46-3.45 (m, 1H), 3.04-2.71 (m, 6H), 2.45-2.25 (m, 5H), 2.17-1.86 (m, 3H), 1.15-0.06 (m, 3H).

**Example 2**

(*R*)-*N*-((*S*)-2-(Dimethylamino)-1-phenylethyl)-6-methyl-2-(((1-methyl-1*H*-pyrazol-3-yl)methyl)amino)-5,8-dihydropyrido [3,4-*d*]pyrimidine-7(6*H*)-carboxamide

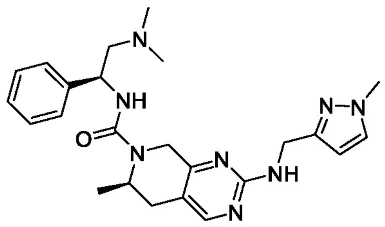

2

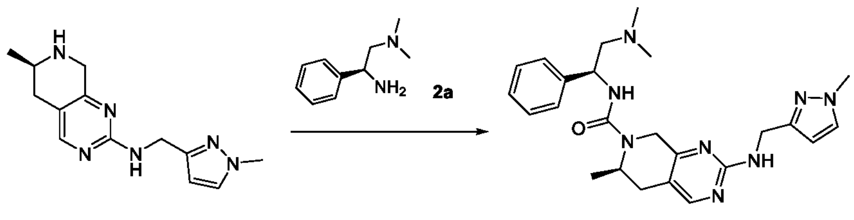

Step 1

Preparation of compound **2**

Compound **2a** (57.2 mg, 348 μmol) and *N*,*N*-carbonyldiimidazole (75.3 mg, 464 μmol) were dissolved in tetrahydrofuran, and triethylamine (1.16 mmol, 162 μL) was added to the solution. Then the mixture was stirred at room temperature for 0.5 h. Compound **1q** (60.0 mg, 232 μmol, synthesized as described in Example 1) was added to the reaction mixture, and the mixture was stirred at room temperature for 12 h. The reaction mixture was concentrated under reduced pressure to give 2 as a crude product. The crude product was separated and purified by preparative HPLC (column: Waters Xbridge Prep OBD C18 150 × 40 mm × 10 μm; mobile phase: [water ($NH_4HCO_3$)-ACN]; B%: 10%-40%) to give compound **2** (11.8 mg, 25.2 μmol, yield: 10.8%).

MS m/z (ESI): 449.3 $[M+H]^+$.

$^1$H NMR (400 MHz, DMSO-$d_6$): δ: 8.10 (s, 1H), 7.53 (d, J = 2.0 Hz, 1H), 7.39-7.25 (m, 4H), 7.23-7.10 (m, 2H), 6.78 (d, J = 7.6 Hz, 1H), 6.10 (d, J = 2.0 Hz, 1H), 4.93-4.83 (m, 1H), 4.72-4.55 (m, 2H), 4.47-4.34 (m, 2H), 3.96 (d, J = 18.4 Hz, 1H), 3.77 (s, 3H), 2.77 (dd, J = 5.4, 15.2 Hz, 1H), 2.69-2.59 (m, 1H), 2.42 (d, J = 15.2 Hz, 1H), 2.34 (dd, J = 5.9, 12.0 Hz, 1H), 2.17 (s, 6H), 0.94 (d, J = 6.8 Hz, 3H).

**Example** 3

(*S*)-*N*-((*S*)-2-(Dimethylamino)-1-phenylethyl)-6-methyl-2-(((1-methyl-1*H*-pyrazol-3-yl)methyl)amino)-5,8-dihydropyrido [3,4-*d*]pyrimidine-7(6*H*)-carboxamide

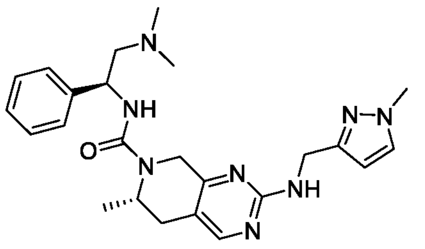

3

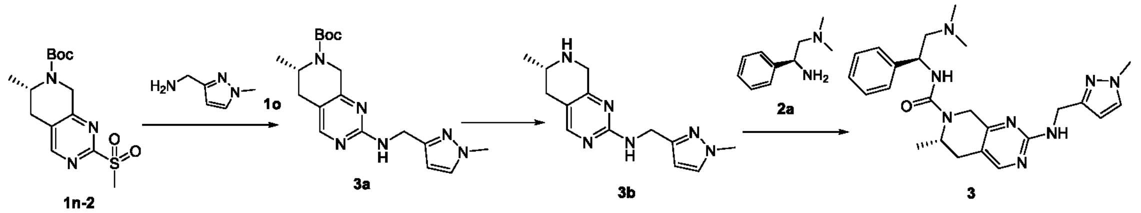


1n-2 3a 3b 3

Step 1

Preparation of compound 3a

**1o** (543 mg, 4.89 mmol) was added to a solution of compound **1n-2** (200 mg, 0.611 mmol) in t-BuOH (2 mL) at room temperature. The reaction mixture was heated to 100 °C, stirred for 12 h, and concentrated under reduced pressure to give a crude product. The crude product was purified by silica gel flash column chromatography (eluent: 1-5% ethyl acetate in petroleum ether) to give compound **3a** (150 mg, 0.418 mmol, yield: 68.51%). MS m/z (ESI): 359.2 $[M+H]^+$.

Step 2

Preparation of compound **3**

TFA (1 mL) was added to a solution of compound **3a** (150 mg, 0.139 mmol) in DCM at room temperature, and the mixture was stirred for 1 h. The reaction mixture was concentrated to dryness under reduced pressure to give **3b** (80.0 mg, 0.135 mmol) as a crude product.

MS m/z (ESI): 259.1 $[M+H]^+$.

Compound **2a** (0.102 mL, 0.609 mmol) and *N*,*N*-carbonyldiimidazole (75.3 mg, 465 μmol) were dissolved in tetrahydrofuran, and triethylamine (118 mg 1.16 mmol, 162 μL) was added to the solution. Then the mixture was stirred at room temperature for 0.5 h. Compound **3b** (60.0 mg, 232 μmol) was added to the reaction mixture, and the mixture was stirred at room temperature for 12 h. The reaction mixture was concentrated under reduced pressure to give **3** as a crude product. The crude product was separated and purified by preparative HPLC (column: Waters Xbridge Prep OBD C18 150 × 40 mm × 10 μm; mobile phase: [water ($NH_4HCO_3$)-ACN]; B%: 10%-40%) to give compound **3** (44.4 mg, 98.9 μmol, yield: 42.6%).

MS m/z (ESI): 449.3 $[M+H]^+$.

$^1H$ NMR (400 MHz, DMSO-$d_6$): δ: 8.09 (s, 1H), 7.53 (d, J = 2.0 Hz, 1H), 7.34-7.30 (m, 2H), 7.30-7.25 (m, 2H), 7.22-7.12 (m, 2H), 6.77 (d, J = 7.2 Hz, 1H), 6.10 (d, J = 2.0 Hz, 1H), 4.93-4.82 (m, 1H), 4.70-4.59 (m, 2H), 4.45-4.37 (m, 2H), 3.93 (d, J = 18.0 Hz, 1H), 3.76 (s, 3H), 2.80-2.60 (m, 2H), 2.34 (dd, J = 6.0, 12.0 Hz, 2H), 2.17 (s, 6H), 0.95 (d, J = 6.8 Hz, 3H).

**Example 4**

8-Fluoro-3-methyl-7-(((1-methyl-1*H*-pyrazol-3-yl)methyl)amino)-3,4-dihydro-2,6-naphthyridin-2(1*H*)-yl)((3*R*,4*R*)-1-methyl-3-phenylpiperidin-4-yl)methanone

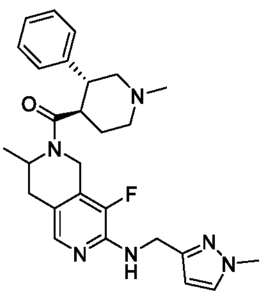

4

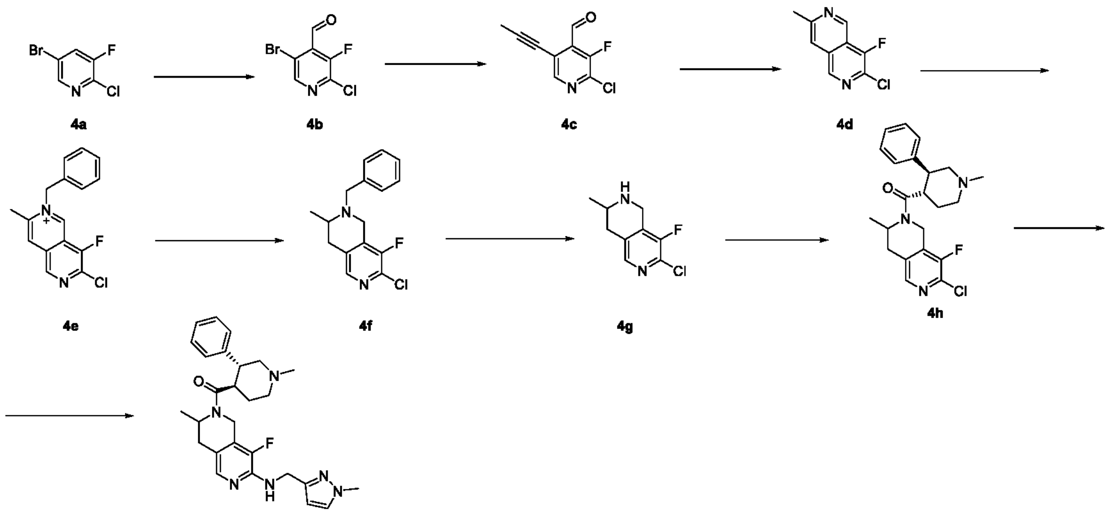

4a 4b 4c 4d 4e 4f 4g 4h 4

Step 1

Preparation of compound **4b**

Compound **4a** (5 g, 23.7 mmol) was dissolved in tetrahydrofuran, and the solution was cooled to -78 °C. n-Butyllithium (11.4 mL, 28.5 mmol) was added dropwise to the solution, and the mixture was stirred at -78 °C for 2 h. A saturated ammonium chloride solution was slowly added to quench the reaction, and after extraction, drying, and concentration, a crude product was obtained. The crude product was separated by normal-phase chromatography to give the title compound **4b** (4.5 g, yield: 79%).

Step 2

Preparation of compound **4c**

Compound **4b** (4.5 g, 18.873 mmol) was dissolved in *N*,*N*-dimethylformamide, and $PdCl_2(TPP)_2$ (0.28 g, 0.47 mmol), cuprous iodide (90 mg, 0.47 mmol), triethylamine (6.5 mL, 47.2 mmol), and propyne (22.6 mL, 22.6 mmol) were added. The reaction mixture was stirred at room temperature for 2 h under a nitrogen atmosphere, extracted, concentrated, and dried to give a crude product. The crude product was purified by normal-phase chromatography to give **4c** (2.6 g, yield: 69%).

Step 3

Preparation of compound **4d**

Compound **4c** (2.6 g, 13.2 mmol) was dissolved in toluene, and then *p*-toluenesulfonic acid (450 mg, 2.6 mmol) and *tert*-butylamine (7 mL, 65.8 mmol) were added. The reaction mixture was left to react at 90 °C for 12 h, extracted, concentrated, and dried to give a crude product. The crude product was purified by normal-phase chromatography to give **4d** (1.3 g, yield: 50%).
MS m/z (ESI): 197.1 $[M+H]^+$.

Step 4

Preparation of compound **4e**

Compound **4d** was dissolved in acetonitrile, and then benzyl bromide was added. The reaction mixture was left to react at 80 °C for 12 h and cooled to room temperature, and diethyl ether was added to the reaction mixture. After filtration, washing, and drying, **4e** was obtained as a crude product, and the crude product was used directly in the next step. MS m/z (ESI): 287.1 $[M+H]^+$.

Step 5

Preparation of compound **4f**

Compound **4e** was dissolved in methanol, and then solid sodium borohydride was slowly added. The reaction mixture was stirred at room temperature for 30 min, extracted, concentrated, and dried to give a crude product. The crude product was purified by normal-phase chromatography to give **4f** (0.4 g, yield: 30%).
MS m/z (ESI): 291.1 $[M+H]^+$.

Step 6

Preparation of compound **4g**

Compound **4f** (130 mg, 0.45 mmol) was dissolved in 1,2-dichloroethane, and then potassium carbonate was added. The reaction mixture was heated at reflux for 3 h. After filtration and concentration, an oily crude product was obtained. The crude product was dissolved in methanol, and the solution was heated at reflux for 1 h. The reaction mixture was concentrated under reduced pressure to give a crude product, and the crude product was purified by normal-phase chromatography to give compound **4g** (70 mg, 78%).
MS m/z (ESI): 201.1 $[M+H]^+$.

Step 7

Preparation of compound **4h**

1-Methyl-3-phenylpiperidine-4-carboxylic acid (76.5 mg, 0.35 mmol) and *N,N,N',N*-tetramethyl-*O*-(1*H*-benzotriazol-1-yl)uronium hexafluorophosphate (396.9 mg, 1.05 mmol) were dissolved in *N*,*N*-dimethylformamide. After 10 min of stirring, *N,N*-diisopropylethylamine (193 μL, 1.05 mmol) and compound **4g** were added. The reaction mixture was separated and purified by preparative HPLC (column: Waters Xbridge Prep OBD C18 150 × 40 mm × 10 μm; mobile phase: [water ($NH_4HCO_3$)-ACN]; B%: 10%-40%, 8 min) to give compound **4h** (9 mg, yield: 6%).
MS m/z (ESI): 402.1 $[M+H]^+$.

Step 8

Preparation of compound **4**

Compound **4h** (9 mg, 0.022 mmol), (1-methyl-1*H*-pyrazol-3-yl)methanamine (4.98 mg, 0.045 mmol), XantPhos (0.65 mg, 0.001 mmol), $Pd_2dba_3$ (1.02 mg, 0.001 mmol), and potassium phosphate (14.26 mg, 0.067 mmol) were dissolved sequentially in dioxane under a nitrogen atmosphere, and the solution was microwaved at 120 °C for 1 h. The reaction mixture was cooled to room temperature and filtered, and the filtrate was collected, concentrated under reduced pressure, and then separated by normal-phase chromatography to give the title compound **4** (2 mg, yield: 18%).
MS m/z (ESI): 478.2 $[M+H]^+$.

**Example 5**

(*R*)-*N*-((*S*)-2-(Dimethylamino)-1-phenylethyl)-6-methyl-2-(((*R*)-1-phenylethyl)amino)-5,8-dihydropyrido[3,4-*d*]pyrimidine-7(6*H*)-carboxamide

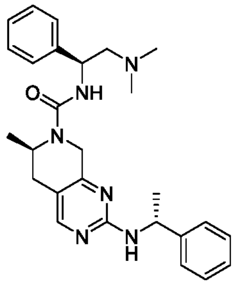

5

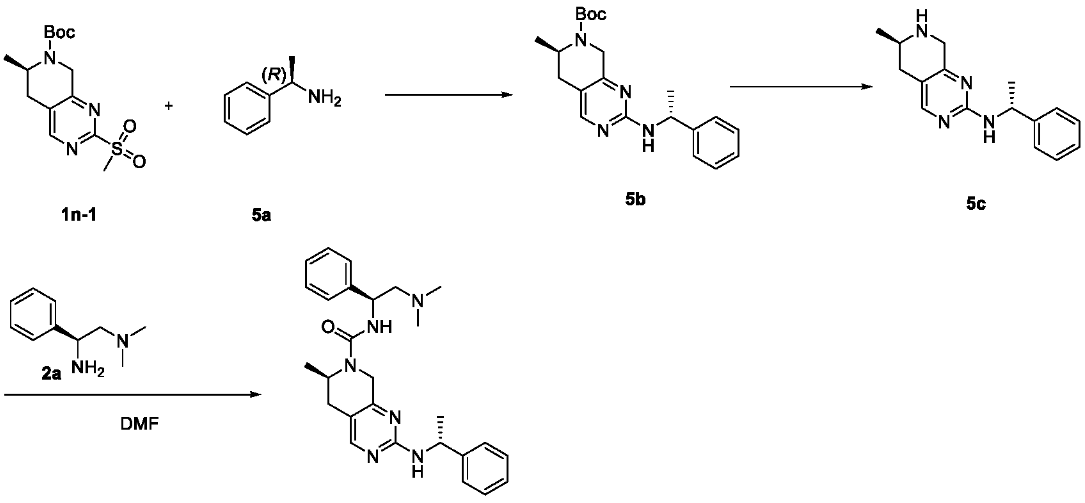


1n-1 5a 5b 5c

2a

5

Step 1

Preparation of compound **5b**

*t*-BuOH (2 mL) and compounds **1n-1** (100 mg, 0.31 mmol) and **5a** (0.39 mL, 3.05 mmol) were added to a microwave tube and microwaved for 8 h under a nitrogen atmosphere. The reaction mixture was concentrated to give a crude product, and the crude product was separated by reversed-phase column chromatography (0-100% water/-acetonitrile) to give the target compound **5b** (89 mg, yield: 79%).

MS m/z (ESI): 369.3 $[M+H]^+$.

Step 2

Preparation of compound **5c**

Compound **5b** (89 mg, 0.24 mmol) was dissolved in DCM (1.0 mL), and TFA (0.18 mL, 2.4 mmol) was slowly added dropwise to the solution. The mixture was stirred at room temperature for 7 h, diluted with water, neutralized with $K_2CO_3$, and extracted with DCM. The organic phases were combined, dried over anhydrous $Na_2SO_4$, and filtered, and the filtrate was concentrated to dryness by rotary evaporation to give the target compound **5c** as a crude product. The crude product was used directly in the next step.

MS m/z (ESI): 269.2 $[M+H]^+$.

Step 3

Preparation of compound **5**

**2a** (30 mg, 0.18 mmol) was dissolved in DMF (0.5 mL), and CDI (60 mg, 0.37 mmol) was added to the solution. The mixture was stirred at room temperature for 5 min. $Et_3N$ (76 μL, 0.55 mmol) and compound **5b** (34.3 mg, 0.13 mmol) were added sequentially, and the mixture was left to react overnight at room temperature. The reaction mixture was separated and purified by preparative HPLC (column: Waters Xbridge Prep OBD C18 150 × 40 mm × 10 μm; mobile

phase: [water ($NH_4HCO_3$)-ACN]) to give compound **5** (20 mg, yield: 23.9%).

MS m/z (ESI): 459.4 $[M+H]^+$.

$^1H$ NMR (400 MHz, $CD_3OD$): δ 8.02 (s, 1H), 7.42-7.34 (m, 6H), 7.32-7.24 (m, 3H), 7.20-7.14 (m, 1H), 5.26 (dd, *J* = 11.4, 4.0 Hz, 1H), 5.11 (q, *J* = 6.9 Hz, 1H), 4.79-4.68 (m, 2H), 4.07 (d, *J* = *18.3* Hz, 1H), 3.37-3.33 (m, 1H), 3.06 (dd, *J* = 13.0, 4.1 Hz, 1H), 2.84 (dd, *J* = 15.6, 5.6 Hz, 1H), 2.73 (s, 6H), 2.48 (d, 16H), 1.50 (d, *J* = 7.0 Hz, 3H), 1.01 (d, 7.2 Hz, 3H).

**Example 6**

(*R*)-*N*-((*S*)-2-(Dimethylamino)-1-phenylethyl)-6-methyl-2-(((*S*)-1-(pyridin-2-yl)ethyl)amino)-5,8-dihydropyrido[3,4-*d*]pyrimidine-7(6*H*)-carboxamide

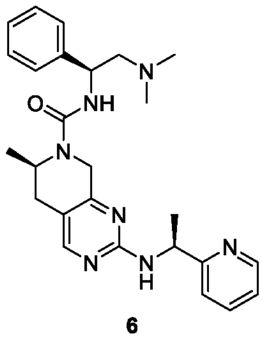

**6**

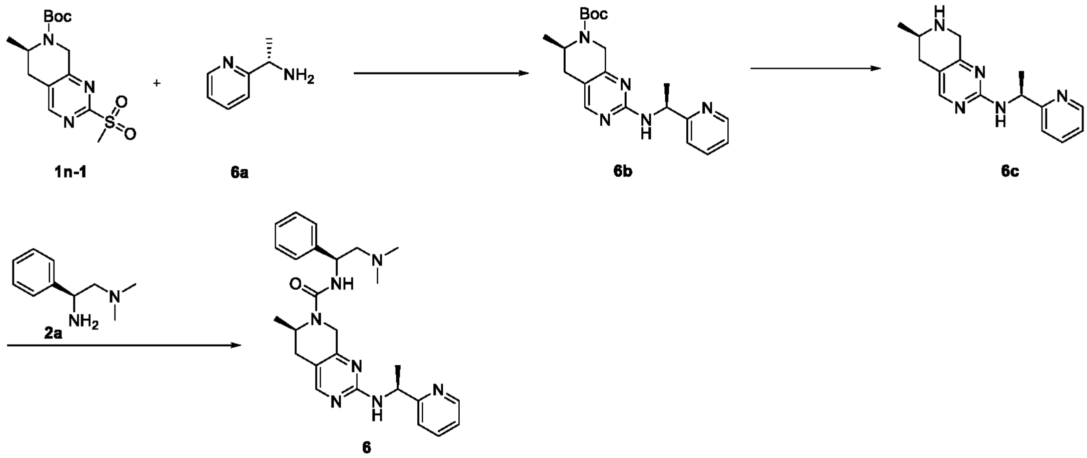

1n-1

6a

6b

6c

2a

6

Step 1

Preparation of compound **6b**

Compounds **1n-1** (100 mg, 0.31 mmol) and **6a** (0.37 mL, 3.05 mmol) were added to a microwave tube containing *t*-BuOH (2.0 mL) and microwaved for 5 h under a nitrogen atmosphere. The reaction mixture was concentrated and separated by reversed-phase column chromatography (0-100% water/acetonitrile) to give the target compound **6b** (97.9 mg, yield: 86.8%).

MS m/z (ESI): 370.3 $[M+H]^+$.

Step 2

Preparation of compound **6c**

Compound **6b** (97.9 mg, 0.27 mmol) was dissolved in DCM (1.0 mL), and TFA (0.19 mL, 2.6 mmol) was slowly added dropwise to the solution. The mixture was stirred at room temperature for 7 h, diluted with water, neutralized with $K_2CO_3$, and extracted with DCM. The organic phases were combined, dried over anhydrous $Na_2SO_4$, and filtered, and the

filtrate was concentrated to dryness by rotary evaporation to give the target compound **6c** as a crude product. The crude product was used directly in the next step.

MS m/z (ESI): 270.2 $[M+H]^+$.

Step 3

Preparation of compound **6**

**2a** (30 mg, 0.18 mmol) was dissolved in DMF (0.5 mL), and CDI (60 mg, 0.37 mmol) was added to the solution. The mixture was stirred at room temperature for 5 min. $Et_3N$ (76 μL, 0.55 mmol) and compound **6c** (34.4 mg, 0.13 mmol) were added sequentially, and the mixture was left to react overnight at room temperature. The reaction mixture was separated and purified by preparative HPLC (column: Waters Xbridge Prep OBD C18 150 × 40 mm × 10 μm; mobile phase: [water ($NH_4HCO_3$)-ACN]) to give compound **6** (40 mg, yield: 47.7%).

MS m/z (ESI): 460.3 $[M+H]^+$.

$^1H$ NMR (400 MHz, DMSO-$d_6$): $^1H$ NMR (400 MHz,) δ 8.50 (d, *J* = 4.4 Hz, 1H), 8.08 (s, 1H), 7.71 (td, *J* = 7.7, 1.8 Hz, 1H), 7.41-7.38 (m, 2H), 7.36-7.26 (m, 4H), 7.23-7.18 (m, 2H), 6.83 (d, *J* = 7.7 Hz, 1H), 5.12 (p, *J* = 7.1 Hz, 1H), 4.92-4.84 (m, 1H), 4.68-4.48 (m, 2H), 3.94 (d, *J* = 18.8 Hz, 1H), 2.77-2.64 (m, 2H), 2.44-2.35 (m, 2H), 2.19 (s, 6H), 1.45 (d, *J* = 7.0 Hz, 3H), 0.93 (d, *J* = 6.7 Hz, 3H).

**Example 7**

(*R*)-2-((2,3-Dihydro-1*H*-inden-2-yl)amino)-*N*-((*S*)-2-(dimethylamino)-1-phenylethyl)-6-methyl-5,8-dihydropyrido[3,4-d] pyrimidine-7(6*H*)-carboxamide

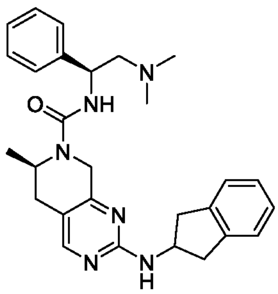

**7**

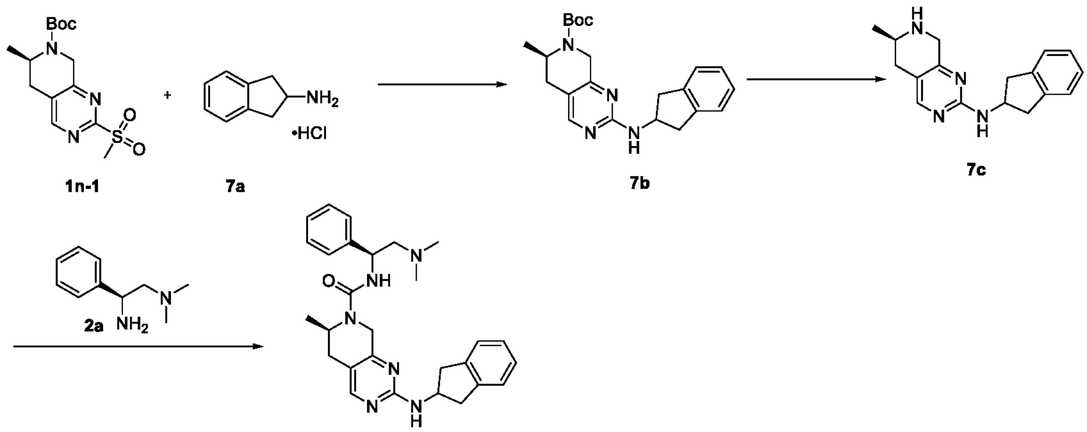

1n-1 7a 7b 7c 2a 7

Compound **7** was prepared by referring to the synthesis method of Example **5.** Compound **7** (35 mg, yield: 40.7%) was obtained through three reactions with **1** (100 mg, 0.31 mmol) and **7a** (414.5 mg, 2.44 mmol) as starting materials. The crude compound 7 was obtained through separation and purification by preparative HPLC (column: Waters Xbridge Prep OBD C18 150 × 40 mm × 10 μm; mobile phase: [water ($NH_4HCO_3$)-ACN]).

MS m/z (ESI): 471.4 $[M+H]^+$.

$^1$H NMR (400 MHz, $CD_3OD$): δ 8.09 (s, 1H), 7.42-7.35 (m, 4H), 7.32-7.27 (m, 1H), 7.22-7.16 (m, 2H), 7.15-7.10 (m, 2H), 5.27 (dd, J = 11.4, 4.0 Hz, 1H), 4.82-4.64 (m, 3H), 4.17 (d, J = 18.2 Hz, 1H), 3.35-3.32 (m, 2H), 3.28 (s, 1H), 3.07 (dd, J = 13.0, 4.1 Hz, 1H), 2.93-2.85 (m, 3H), 2.73 (s, 6H), 2.53 (d, 15.6 Hz, 1H), 1.07 (d, J = 6.8 Hz, 3H).

## Example 8

(*R*)-*N*-((*S*)-2-(Ethyl(methyl)amino)-1-phenylethyl)-6-methyl-2-(((1-methyl-1*H*-pyrazol-3-yl)methyl)amino)-5,8-dihydropyrido[3,4-*d*]pyrimidine-7(6*H*)-carboxamide

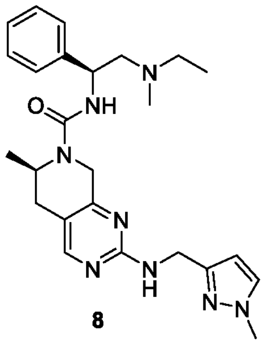

8

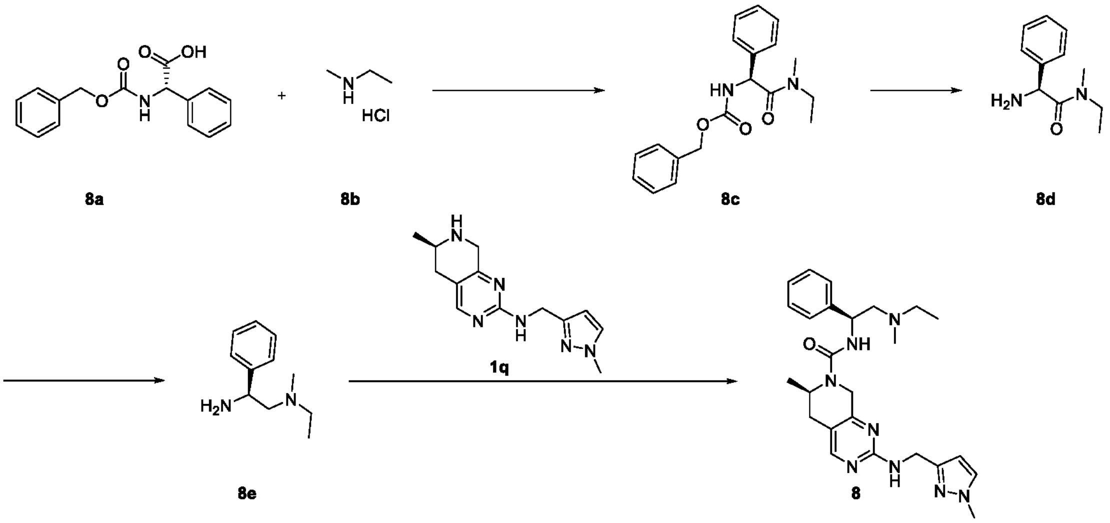

8a 8b 8c 8d

1q

8e 8

Step 1

Preparation of compound **8c**

HBTU (1.33 g, 3.51 mmol) and $K_2CO_3$ (1.45 g, 10.52 mmol) were added to a solution of **8a** (1 g, 3.51 mmol) in DCM (30 mL), and the mixture was left to react at room temperature for 0.5 h. 8b (0.5 g, 5.26 mmol) was added to the reaction mixture, and the mixture was left to react overnight at 40 °C. After the reaction was complete, the reaction mixture was cooled to room temperature, then filtered, and concentrated to give a crude product, and the crude product was separated by column chromatography (0-100% petroleum ether/ethyl acetate) to give the target compound **8c** (973 mg, yield: 85.1%).
MS m/z (ESI): 327.2 [M+H]$^+$.

Step 2

Preparation of compound **8d**

Compound **8c** (973 mg, 2.98 mmol) was dissolved in ethyl acetate (10 mL), and 10% Pd/C (300 mg) was added under a nitrogen atmosphere. The mixture was purged 3 times with $H_2$, left to react at room temperature for 6 h under a $H_2$

atmosphere, and filtered through diatomaceous earth, and the filtrate was concentrated to give compound **8d** as a crude product. The crude product was used directly in the next step.

MS m/z (ESI): 193.2 $[M+H]^+$.

Step 3

Preparation of compound **8e**

THF (6.0 mL) and $LiAlH_4$ (4.7 mL, 1 M, THF) were added sequentially to a dry three-necked flask under a nitrogen atmosphere. The reaction mixture was placed in a dry ice-ethanol bath at -78 °C, and a solution of compound **8d** (400 mg, 2.08 mmol) in THF (2 mL) was slowly added dropwise to the reaction mixture. After the addition, the mixture was refluxed overnight. The reaction mixture was cooled to room temperature and then placed in an ice bath, and 0.5 mL of water and a 15% NaOH solution (0.2 mL) were added sequentially to the reaction mixture. The mixture was dried over anhydrous $Na_2SO_4$ and filtered, and the filtrate was concentrated under reduced pressure to give a crude product. The crude product was separated by reversed-phase column chromatography (0-100% water/acetonitrile) to give **8e** (177 mg, yield: 47.7%) as a yellow liquid.

MS m/z (ESI): 179.2 $[M+H]^+$.

Step 4

Preparation of compound **8**

Compound **8e** (30 mg, 0.17 mmol) was dissolved in DMF (0.5 mL), and CDI (54.6 mg, 0.34 mmol) was added to the solution. The mixture was stirred at room temperature for 5 min. $Et_3N$ (7 μL, 0.51 mmol) and compound **5d** (30.4 mg, 0.12 mmol) were added sequentially, and the mixture was left to react overnight at room temperature. The reaction mixture was separated and purified by preparative HPLC (column: Waters Xbridge Prep OBD C18 150 × 40 mm × 10 μm; mobile phase: [water ($NH_4HCO_3$)-ACN]) to give compound **8** (20 mg, yield: 25.7%).

MS m/z (ESI): 463.4 $[M+H]^+$.

$^1H$ NMR (400 MHz,) δ 8.08 (s, 1H), 7.49-7.36 (m, 6H), 7.32-7.29 (m, 1H), 6.19 (d, J = 2.2 Hz, 1H), 5.41-5.34 (m, 1H), 4.82-4.73 (m, 2H), 4.53 (s, 2H), 4.14 (d, J = 18.2 Hz, 1H), 3.82 (s, 3H), 3.57-3.47 (m, 1H), 3.28-3.09 (m, 3H), 2.90-2.86 (s, 4H), 2.54-2.50 (m, 1H), 1.32 (t, J = 7.3 Hz, 3H), 1.06 (d, J = 7.0 Hz, 3H).

**Example 9**

(*R*)-*N*-((*S*)-2-(Dimethylamino)-1-phenylethyl)-6-methyl-2-(((*S*)-1-phenylethyl)amino)-5,8-dihydropyrido[3,4-d]pyrimidine-7(6*H*)-carboxamide

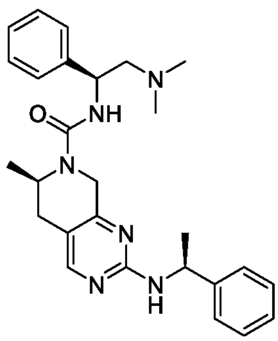

**9**

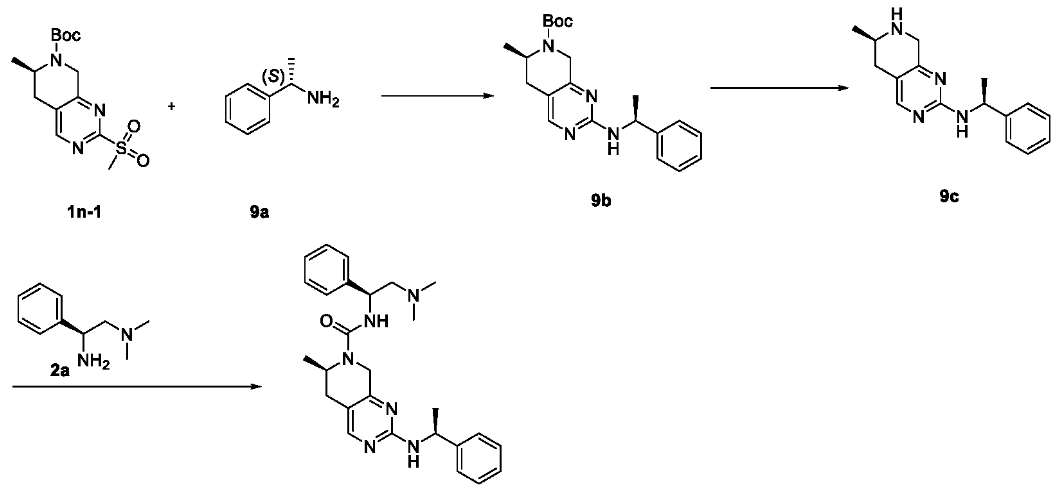

1n-1 9a 9b 9c

2a

9

Compound **9** was prepared by referring to the synthesis method of compound **5.** Compound **9** (12 mg, 0.026 mmol) was obtained through three reactions with **1n-1** (100 mg, 0.306 mmol) as a starting material.

MS m/z (ESI): 471.4 $[M+H]^+$.

$^1H$ NMR (400 MHz, $CD_3OD$): δ 8.03 (s, 1H), 7.37-7.25 (m, 8H), 7.24-7.14 (m, 2H), 5.13 (q, J = 7.0 Hz, 1H), 5.01 (dd, J = 10.7, 4.4 Hz, 1H), 4.77-4.69 (m, 1H), 4.66 (d, J = 18.3 Hz, 1H), 4.11 (d, J = 18.3 Hz, 1H), 2.91-2.78 (m, 2H), 2.48 (dd, J = 15.5, 1.7 Hz, 1H), 2.41 (dd, J = 12.8, 4.5 Hz, 1H), 2.30 (s, 6H), 1.51 (d, J = 7.0 Hz, 3H), 1.02 (d, J = 6.8 Hz, 3H).

## Example 10

*N*-((*S*)-2-(Dimethylamino)-1-phenylethyl)-6-methyl-2-((tetrahydro-2*H*-pyran-4-yl)amino)-5,8-dihydropyrido[3,4-*d*]pyrimidine-7(6*H*)-carboxamide

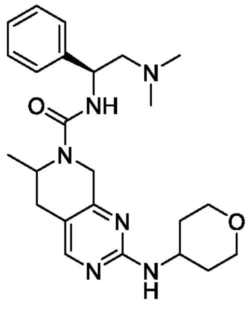

**10**

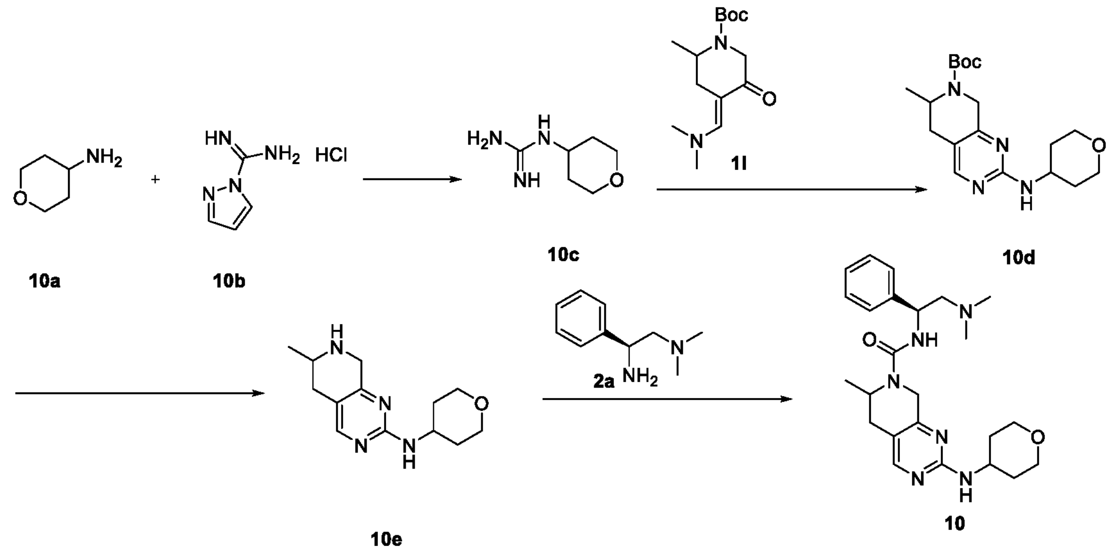
10a 10b 10c 11 10d 10e 2a 10

Step 1

Preparation of compound **10c**

DIPEA (1.63 mL, 9.89 mmol) and **10a** (500 mg, 4.94 mmol) were added sequentially to a solution of **10b** (1.09 g, 7.42 mmol) in DMF (3.0 mL) under a nitrogen atmosphere, and the mixture was left to react at room temperature for 48 h. The reaction mixture was concentrated, and the crude product was separated by reversed-phase column chromatography (mobile phase: [water ($NH_4OH$)-ACN]) to give the target compound **10c** (400 mg, yield: 56.5%).
MS m/z (ESI): 144.2 $[M+H]^+$.

Step 2

Preparation of compound **10d**

Compound **10c** (346.8 mg, 2.42 mmol) and compound **11** (500 mg, 1.86 mmol) were dissolved in EtOH (10 mL), and EtONa (279 mg, 4.1 mmol) was added to the solution. The mixture was heated to 50 °C and left to react overnight. The reaction mixture was cooled to room temperature, diluted with water, and extracted 3 times with DCM. The organic phases were combined, dried over anhydrous $Na_2SO_4$, and filtered, and the filtrate was concentrated and separated by reversed-phase column chromatography (mobile phase: [water ($NH_4OH$)-ACN]) to give the target compound **10d** (258 mg, yield: 39.7%). MS m/z (ESI): 349.2 $[M+H]^+$.

Step 3

Preparation of compound **10e**

Compound **10d** (258 mg, 0.74 mmol) was dissolved in a mixed solution of 1,4-dioxane and MeOH (4 mL, 3:1), and a solution of hydrochloric acid in dioxane (1.8 mL, 4 M) was slowly added dropwise to the solution. The mixture was left to react overnight at room temperature, diluted with water, neutralized with a $K_2CO_3$ solution, and extracted 3 times with DCM. The organic phases were combined, dried over anhydrous $Na_2SO_4$, and filtered. The filtrate was concentrated to dryness by rotary evaporation to give compound **10d** as a crude product, and the crude product was used directly in the next step.
MS m/z (ESI): 249.2 $[M+H]^+$.

Step 4

Preparation of compound **10**

CDI (213.2 mg, 1.32 mmol) was added to a solution of **2a** (108 mg, 0.66 mmol) in DMF (3 mL), and the mixture was stirred at room temperature for 5 min. $Et_3N$ (0.27 mL, 1.97 mmol) and compound **10d** (114.3 mg, 0.46 mmol) were added

sequentially, and the mixture was left to react overnight at room temperature. The reaction mixture was separated and purified by preparative HPLC (column: Waters Xbridge Prep OBD C18 150 × 40 mm × 10 μm; mobile phase: [water ($NH_4HCO_3$)-ACN]) to give compound **10** (80 mg, yield: 27.7%).

MS m/z (ESI): 439.3 $[M+H]^+$.

The compound was resolved on a chiral column (column: DAICEL ChiralPak IC (250 × 30 mm, 10 μm); mobile phase: A: supercritical $CO_2$ fluid, B: ethanol (0.1% $NH_3H_2O$)) to give compounds **10-1** and **10-2,** which have the following structures:

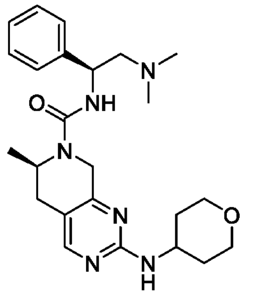

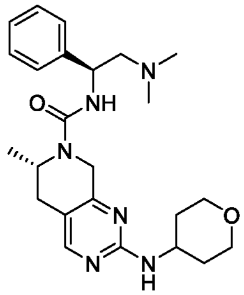

**Compound 10-1** (retention time: 3.43 min)

MS m/z (ESI): 439.3 $[M+H]^+$.

$^1H$ NMR (400 MHz, $CDCl_3$) δ 8.00 (s, 1H), 7.24-7.13 (m, 5H), 5.68 (s, 1H), 4.86 (d, J = 8.0 Hz, 1H), 4.81-4.66 (m, 2H), 4.53 (d, J = 17.9 Hz, 1H), 4.10 (d, J = 17.8 Hz, 1H), 3.99-3.89 (m, 3H), 3.57-3.40 (m, 2H), 2.84 (dd, J = 15.8, 5.8 Hz, 1H), 2.53 (t, J = 11.7 Hz, 1H), 2.37-2.27 (m, 2H), 2.21 (s, 6H), 2.04-1.88 (m, 2H), 1.52-1.44 (m, 2H), 1.01 (d, J = 6.8 Hz, 3H).

**Compound 10-2** (retention time: 6.29 min)

MS m/z (ESI): 439.3 $[M+H]^+$.

$^1H$ NMR (400 MHz, $CDCl_3$) δ 8.00 (s, 1H), 7.27-7.18 (s, 5H), 5.95 (s, 1H), 4.97 (s, 1H), 4.83-4.72 (m, 1H), 4.71-4.62 (m, 1H), 4.55 (d, J = 18.2 Hz, 1H), 4.06 (d, J = 18.2 Hz, 1H), 4.06-3.89 (m, 3H), 3.51-3.41 (m, 2H), 2.91-2.83 (m, 1H), 2.65 (t, J = 11.8 Hz, 1H), 2.39-2.32 (m, 3H), 2.24 (s, 6H), 1.98-1.94 (m, 2H), 1.49-1.45 (m, 2H), 0.99 (d, J = 6.8 Hz, 3H).

**Example 11**

(*R*)-*N*-((*R*)-2-(Dimethylamino)-1-phenylethyl)-6-methyl-2-(((1-methyl-1*H*-pyrazol-3-yl)methyl)amino)-5,8-dihydropyrido [3,4-*d*]pyrimidine-7(6*H*)-carboxamide

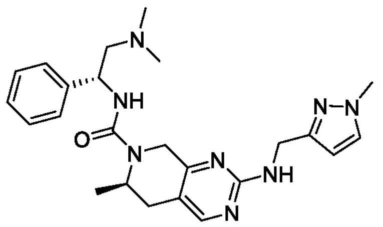

11

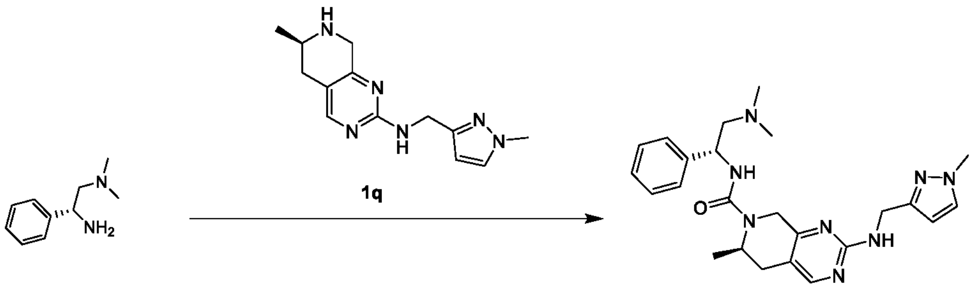

1q

11a

11

Step 1

Preparation of compound **11**

Compound **11a** (9.08 mg, 0.055 mmol) and CDI (18 mg, 0.111 mmol) were dissolved in DMF (2 mL), and $Et_3N$ (0.023 mL, 0.166 mmol) was added to the solution. Then the mixture was stirred at room temperature for 0.5 h. Compound **1q** (10 mg, 0.039 mmol) was added to the reaction mixture, and the mixture was stirred at room temperature for 12 h. The reaction mixture was separated and purified by preparative HPLC to give compound **11** (5.0 mg, 0.011 mmol).

MS m/z (ESI): 449.38 $[M+H]^+$.

$^1H$ NMR (400 MHz, $CD_3OD$): $\delta$ 8.05 (s, 1H), 7.52-7.17 (m, 6H), 6.16 (s, 1H), 5.31 (d, J = 7.8 Hz, 1H), 4.78-4.58 (m, 2H), 4.50 (s, 2H), 4.16 (d, J = 18.4 Hz, 1H), 3.80 (s, 3H), 3.52-3.36 (m, 1H), 3.28 (s, 1H), 3.23-3.12 (m, 1H), 2.81 (s, 7H), 2.49 (d, J = 15.6 Hz, 1H), 1.05 (d, J = 6.8 Hz, 3H).

### Example 12

(*R*)-*N*-((*S*)-2-(Bis(methyl-$d_3$)amino)-1-phenylethyl)-6-methyl-2-(((1-methyl-1*H*-pyrazol-3-yl)methyl)amino)-5,8-dihydropyrido[3,4-*d*]pyrimidine-7(6*H*)-carboxamide

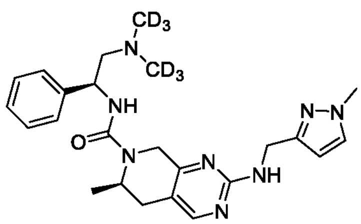

12

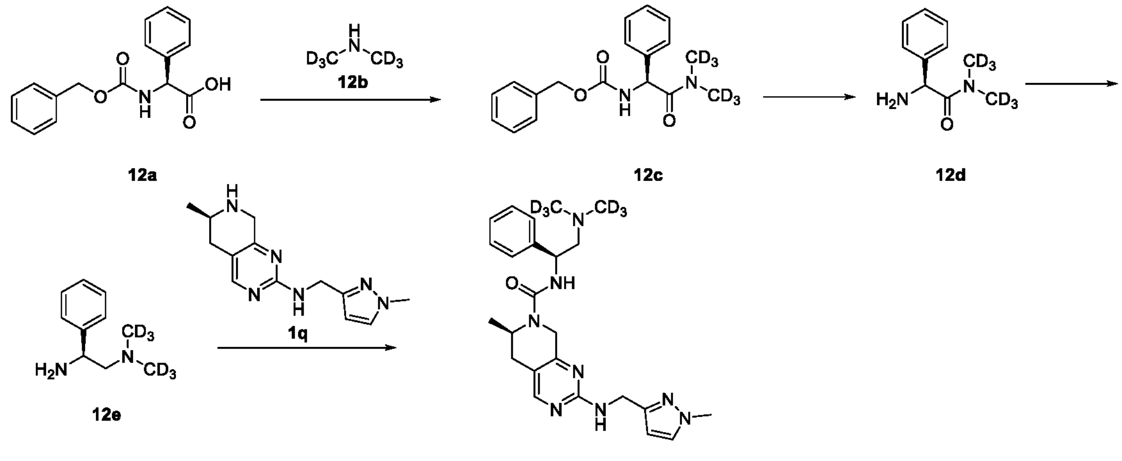

12b

12a 12c 12d

1q

12e 12

Step 1

Preparation of compound **12c**

**12a** (500 mg, 1.753 mmol) and HBTU (1329 mg, 3.5 mmol) were dissolved in DCM (7 mL) at room temperature, and the solution was stirred for 5 min. $K_2CO_3$ (726 mg, 5.26 mmol) and **12b** (134.4 mg, 2.63 mmol) were added. The reaction was stirred at room temperature for 12 h. The reaction mixture was extracted, concentrated, and dried to give a crude product. The crude product was separated and purified by column chromatography to give compound **12c** (900 mg, 2.83 mmol).

MS m/z (ESI): 319.2 $[M+H]^+$.

Step 2

Preparation of compound **12d**

10% palladium on carbon (Pd/C) was added to a solution of compound **12c** (900 mg, 2.83 mmol) in ethyl acetate (15 mL) under a hydrogen atmosphere. The mixture was stirred at room temperature for 2 h. The reaction mixture was filtered and concentrated to give **12d** (260 mg) as a crude product. The crude product (260 mg) was used directly in the next step.

MS m/z (ESI): 185.1 $[M+H]^+$.

Step 3

Preparation of compound **12e**

$LiAlH_4$ (82.0 mg, 2.17 mmol) was added to anhydrous THF (5.0 mL) under a nitrogen atmosphere. After the mixture was cooled to -78 °C, a solution of compound **12d** in THF was added dropwise, and the mixture was stirred for 30 min. The reaction mixture was then heated to 50 °C, left to react for 2 h, and quenched with an ammonium chloride solution. After extraction, concentration, and drying, a crude product was obtained. The crude product was separated and purified by preparative HPLC to give compound **12e** (40.0 mg, yield: 21%).

MS m/z (ESI): 171.1 $[M+H]^+$.

Step 4

Preparation of compound **12**

Compound **12e** (20.0 mg, 0.094 mmol) and CDI (30 mg, 0.188 mmol) were dissolved in

DMF (1.0 mL), and $Et_3N$ (0.039 mL, 0.282 mmol) was added to the solution. Then the mixture was stirred at room temperature for 0.5 h. Compound **1q** (24.3 mg, 0.094 mmol) was added to the reaction mixture, and the mixture was stirred at room temperature for 12 h. The reaction mixture was separated and purified by preparative HPLC to give compound **12** (5.0 mg, 0.011 mmol).

MS m/z (ESI): 455.4 $[M+H]^+$.

$^1H$ NMR (400 MHz, DMSO-$d_6$): δ 8.10 (s, 1H), 7.53 (s, 1H), 7.38-7.27 (m, 4H), 7.24-7.16 (m, 2H), 6.86 (d, J = 7.8 Hz, 1H), 6.09 (s, 1H), 4.93-4.84 (m, 1H), 4.73-4.55 (m, 2H), 4.47-4.33 (m, 2H), 3.97 (d, J = 18.6 Hz, 1H), 3.76 (s, 3H), 2.81-2.71 (m, 2H), 2.45-2.39 (m, 2H), 0.93 (d, J = 6.7 Hz, 3H).

**Example 13**

(*R*)-*N*-((*S*)-1-(3-Chlorophenyl)-2-(dimethylamino)ethyl)-6-methyl-2-(((1-methyl-1*H*-pyrazol-3-yl)methyl)amino)-5,8-dihydropyrido[3,4-*d*]pyrimidine-7(6*H*)-carboxamide

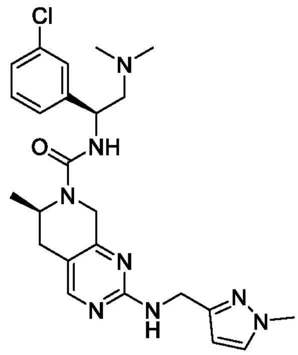

13

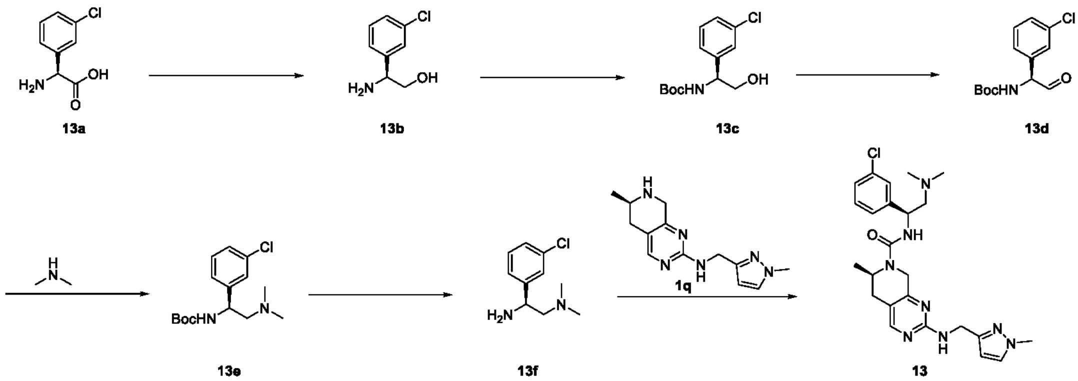

13a

13b

13c

13d

13e

13f

1q

13

Step 1

Preparation of compound **13b**

Compound **13a** (300 mg, 1.616 mmol) was added to a solution of $NaBH_4$ in THF, and the reaction mixture was cooled to 0 °C. A solution of $I_2$ (451 mg, 1.78 mmol) in THF was added dropwise, and after the addition, the mixture was left to react at room temperature for 2 h. After the reaction was complete, the reaction was quenched with an aqueous solution of KOH. After extraction, drying, and concentration, **13b** was obtained as a crude product, and the crude product was used directly in the next step.

MS m/z (ESI): 172.01 $[M+H]^+$.

Step 2

Preparation of compound **13c**

Di-*tert*-butyl dicarbonate (698.3 mg, 3.2 mmol) and $Et_3N$ (485.7 mg, 4.8 mmol) were added to a solution of the crude **13b** in DCM, and the mixture was stirred overnight at room temperature. The reaction mixture was extracted, dried, and concentrated to give a crude product, and the crude product was separated by column chromatography to give the target compound **13c** (170 mg, 0.627 mmol).

MS m/z (ESI): 294.09 $[M+Na]^+$.

Step 3

Preparation of compound **13d**

DMP (212.07 mg, 0.5 mmol) was added to a solution of compound **13c** in DCM, and the solution was stirred at room temperature for 2 h. The reaction mixture was purified by column chromatography to give the target compound **13d** (40 mg, 0.148 mmol).

Step 4

Preparation of compound **13e**

Dimethylamine hydrochloride (57.08 mg, 0.7 mmol) was added to a solution of compound **3d** (40.4 mg, 0.15 mmol) in methanol. After 2 h of stirring at room temperature, $NaBH_3CN$ (18.9 mg, 0.3 mmol) was slowly added, and the mixture was stirred overnight at room temperature. The reaction mixture was directly separated by reversed-phase column chromatography to give compound **13e** (12 mg, 0.04 mmol).
MS m/z (ESI): 299.17 $[M+H]^+$.

Step 5

Preparation of compound **13f**

An excessive amount of TFA (1.0 mL) was added to a solution of compound **13e** (12 mg, 0.04 mmol) in DCM (5.0 mL), and the mixture was stirred at room temperature for 1 h. The reaction mixture was concentrated under reduced pressure to give a crude product, and the crude product was used directly in the next step.
MS m/z (ESI): 199.02 $[M+H]^+$.

Step 6

Preparation of compound **13**

Compound **13f** (11.89 mg, 0.06 mmol) and CDI (19.5 mg, 0.12 mmol) were dissolved in DMF (2.0 mL), and $Et_3N$ (0.18 mmol) was added to the solution. Then the mixture was stirred at room temperature for 0.5 h. Compound **1q** (15.5 mg, 0.06 mmol) was added to the reaction mixture, and the mixture was stirred at room temperature for 12 h. The reaction mixture was separated and purified by preparative HPLC to give compound **13** (2.0 mg, 0.004 mmol).
MS m/z (ESI): 483.28 $[M+H]^+$.
$^1H$ NMR (400 MHz, $CD_3OD$) $\delta$ 8.44 (s, 1H), 8.10 (s, 1H), 7.49-7.42 (m, 2H), 7.40-7.28 (m, 3H), 6.19 (d, J = 2.3 Hz, 1H), 5.24 (dd, J = 11.2, 4.1 Hz, 1H), 4.80-4.69 (m, 2H), 4.54 (s, 2H), 4.18 (d, J = 18.2 Hz, 1H), 3.84 (s, 3H), 3.25 (d, J = 12.0 Hz, 1H), 3.10-3.00 (m, 1H), 2.95-2.85 (m, 1H), 2.72 (s, 6H), 2.54 (dd, J = 15.6, 1.9 Hz, 1H), 2.03 (q, J = 6.4 Hz, 1H), 1.12-1.04 (m, 3H).

**Example 14**

(*R*)-*N*-((*S*)-2-(Dimethylamino)-1-phenylethyl)-6-methyl-2-(((1-(methyl-$d_3$)-1*H*-pyrazol-3-yl)methyl)amino)-5,8-dihydropyrido[3,4-*d*]pyrimidine-7(6*H*)-carboxamide

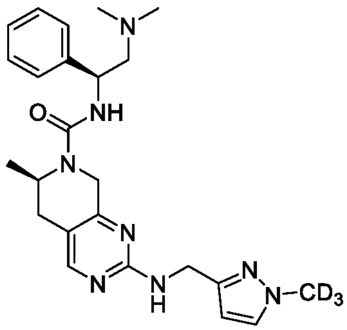

**14**

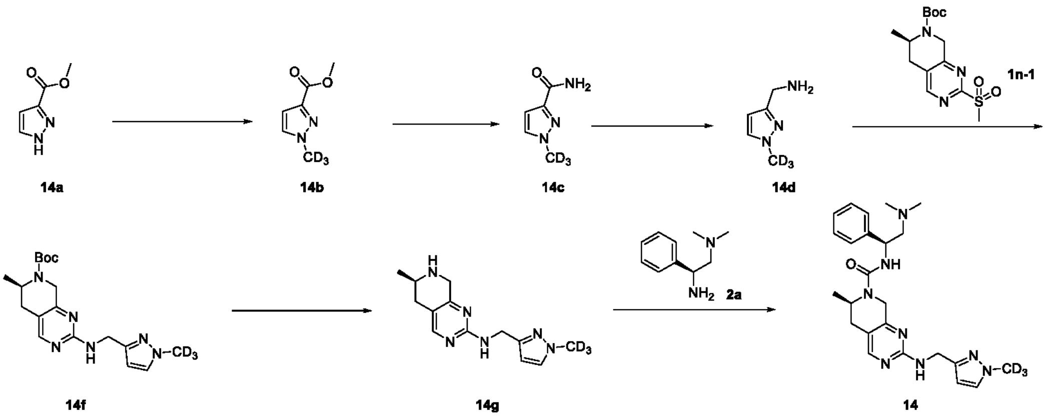


Step 1

Preparation of compound **14b**

$CD_3I$ (2.75 g, 19.0 mmol) was slowly added to a solution of compound **14a** (1.99 g, 15.8 mmol) and cesium carbonate (10.3 g, 31.7 mmol) in DMF (15.0 mL), and the mixture was stirred at room temperature for 2 h. The reaction mixture was extracted, dried, and concentrated to give a crude product, and the crude product was separated by column chromatography to give compound **14b** (1.04 g, 7.26 mmol, yield: 45%).

MS m/z (ESI): 144.07 $[M+H]^+$.

Step 2

Preparation of compound **14c**

Compound **14b** (1.04 g, 7.26 mmol) was dissolved in ammonia water, and the solution was stirred at room temperature for 4 h. The reaction mixture was dried under reduced pressure to give **14c** as a crude product, and the crude product was used directly in the next step.

MS m/z (ESI): 129.00 $[M+H]^+$.

Step 3

Preparation of compound **14d**

$LiAlH_4$ (212.5 mg, 5.6 mmol) was added to anhydrous THF (5.0 mL) under a nitrogen atmosphere. After the mixture was cooled to -78 °C, a solution of compound **14c** (2.8 mmol) in THF was added dropwise, and the mixture was stirred at that temperature for 30 min. The reaction mixture was then left to react at 50 °C for 12 h. After the reaction was complete, the reaction was quenched with an ammonium chloride solution. After extraction, concentration, and drying, **14d** was obtained as a crude product, and the crude product was used directly in the next step.

MS m/z (ESI): 114.99 $[M+H]^+$.

Preparation of compound **14**

**14** (2 mg, 0.0044 mmol) was synthesized through three reactions with compound **14d** by referring to the synthesis of Example **5.**

MS m/z (ESI): 452.37 [M+H]+.

Example **15**

(*R*)-*N*-((*S*)-2-(Dimethylamino)-1-phenylethyl)-6-methyl-2-((2-methylpyridin-4-yl)amino)-5,8-dihydropyrido[3,4-*d*]pyrimidine-7(6*H*)-carboxamide

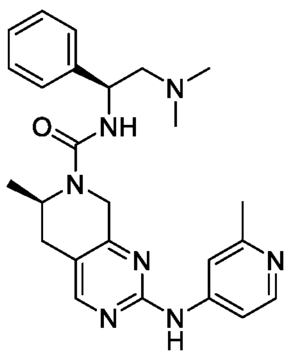

**15**

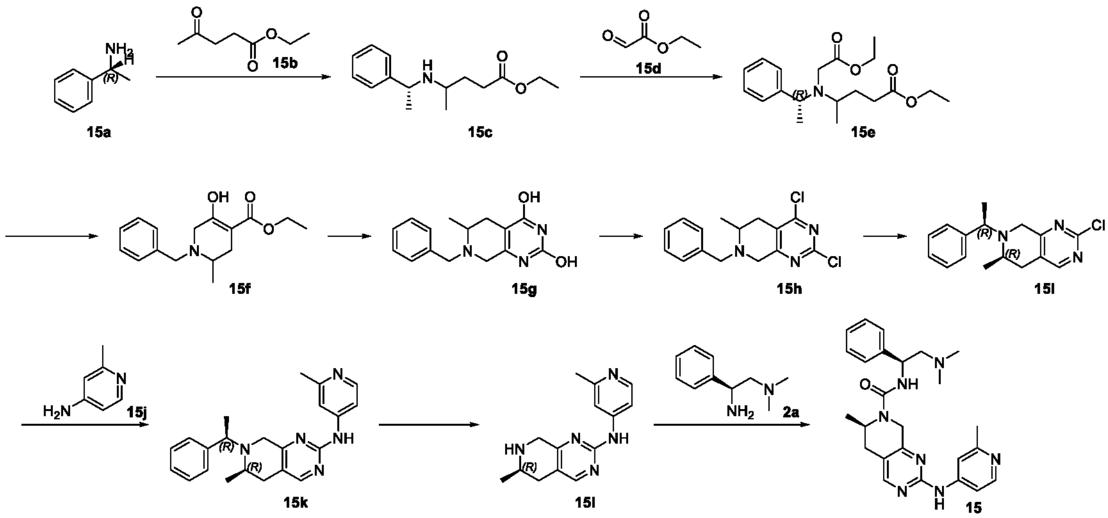


Step 1

Synthesis of compound **15c**

Compound **15a** (50.0 g, 412.6 mmol) and compound **15b** (67.3 mL, 474.5 mmol) were dissolved in DCM (500 mL) at room temperature, and $NaBH(OAc)_3$ (174 g, 825 mmol) was slowly added in batches. The mixture was stirred overnight at room temperature. The reaction mixture was extracted with DCM/MeOH (9:1, 500 × 2 mL) and washed with saturated brine. The organic phase was dried over $Na_2SO_4$ and filtered, and the filtrate was concentrated under reduced pressure to give compound **15c** (80 g, 249 mmol) as a crude product.

MS m/z (ESI): 250.3 [M+H]+.

Step 2

Synthesis of compound **15e**

Compound **15c** (80.0 g, 249 mmol) and compound **15d** (31.7 mL, 320.8 mmol) were dissolved in DCM (1 L) at room temperature, and $NaBH(OAc)_3$ (203 g, 962 mmol) was slowly added in batches. The mixture was stirred at room temperature for 2 days. The reaction mixture was extracted with DCM/MeOH (9:1, 500 mL × 2) and washed with saturated brine. The organic phase was dried over $Na_2SO_4$ and filtered, and the filtrate was concentrated under reduced pressure to give a crude product. The crude product was purified by silica gel flash column chromatography (eluent: 1-10% ethyl acetate in petroleum ether) to give compound **15e** (40 g, 249 mmol).

MS m/z (ESI): 336.3 $[M+H]^+$.

Step 3

Synthesis of compound **15f**

Compound **15e** (40.0 g, 119.2 mmol) was dissolved in toluene (200 mL) at room temperature, and *t*-BuOK (33.5 g, 298 mmol) was slowly added in batches. The mixture was stirred overnight at room temperature. The reaction mixture was extracted with ethyl acetate (100 mL × 2) and washed with saturated brine. The organic phase was dried over $Na_2SO_4$ and filtered, and the filtrate was concentrated under reduced pressure to give a crude product. The crude product was purified by silica gel flash column chromatography (eluent: 1-10% ethyl acetate in petroleum ether) to give compound **15f** (14 g, 48.4 mmol). MS m/z (ESI): 262.2 $[M+H]^+$.

Step 4

Synthesis of compound **15g**

Urea (11.6 mL, 193.5 mmol) was added to a solution of **15f** (14 g, 48.4 mmol) in absolute MeOH (200 mL) at room temperature, and activated 4A MS (10 g) was added. The mixture was heated at reflux and stirred overnight. The reaction mixture was extracted with ethyl acetate (100 mL × 2) and washed with saturated brine. The organic phase was dried over $Na_2SO_4$ and filtered, and the filtrate was concentrated under reduced pressure to give a crude product. The crude product was purified by C18 reversed-phase silica gel column chromatography to give compound **15g** (8 g, 28 mmol).

MS m/z (ESI): 272.2 $[M+H]^+$.

Step 5

Synthesis of compound **15h**

Compound **15g** (8 g, 28 mmol) was dissolved in $POCl_3$ (30 mL) at room temperature, and the reaction mixture was stirred overnight at reflux and concentrated to give a crude product. The crude product was purified by silica gel flash column chromatography (eluent: 1-20% ethyl acetate in petroleum ether) to give compound **15h** (6 g, 18.6 mmol) as a white solid.

MS m/z (ESI): 308.2 $[M+H]^+$.

Step 6

Synthesis of compound **15i**

**15h** (6 g, 18.6 mmol) and ammonia water (8.0 mL) were dissolved in EtOH (100 mL) at room temperature, and activated Zn (15 g) was added. The reaction mixture was heated at reflux, stirred overnight, extracted with ethyl acetate (100 mL × 2), and washed with saturated brine. The organic phase was dried over $Na_2SO_4$ and filtered, and the filtrate was concentrated under reduced pressure to give a crude product. The crude product was purified by silica gel flash column chromatography (eluent: 1-10% ethyl acetate in petroleum ether) to give compound **15i** (3.5 g, 12.2 mmol) as a brownish-yellow solid.

MS m/z (ESI): 288.1 $[M+H]^+$.

Step 7

Synthesis of compound **15k**

At room temperature, to a solution of compound **15i** (240 mg, 0.83 mmol), compound **15j** (108 mg, 1.0 mmol), Xantphos-G3-Pd (79 mg, 0.08 mmol), and $Cs_2CO_3$ (325 mg, 1.25 mmol) in dioxane (5.0 mL). The reaction was heated to 100 °C and stirred for 4 h under a nitrogen atmosphere. The reaction mixture was concentrated to give a crude product. The crude product was purified by silica gel flash column chromatography (eluent: 1-5% ethyl acetate in petroleum ether) to give compound **15k** (100 mg, 33.4%). MS m/z (ESI): 360.3 $[M+H]^+$.

Step 8

Synthesis of compound **151**

10% Pd/C (50 mg) was added to a solution of compound **15k** (100 mg, 0.28 mmol) in MeOH (5.0 mL) at room temperature, and the reaction was stirred overnight at room temperature under a hydrogen atmosphere. The reaction mixture was concentrated to give a crude product. The crude product was purified by silica gel flash column chromatography (eluent: 1-5% ethyl acetate in petroleum ether) to give compound **151** (60 mg, yield: 84.5%).
MS m/z (ESI): 256.3 $[M+H]^+$.

Step 9

Synthesis of compound **15**

CDI (38 mg, 0.24 mmol) and $Et_3N$ (0.05 mL, 0.36 mmol) were added to a solution of **151** (30 mg, 0.12 mmol) and compound **2a** (20 mg, 0.12 mmol) in DMF (1.0 mL) at room temperature, and the reaction mixture was stirred overnight at room temperature and extracted with ethyl acetate (10 mL × 3). The organic phase was dried over $Na_2SO_4$ and filtered, and the filtrate was concentrated under reduced pressure to give a crude product. The crude product was purified by HPLC to give compound **15** (20 mg, 48.3%).
MS m/z (ESI): 446.5 $[M+H]^+$.
$^1H$ NMR (400 MHz, $CD_3OD$): δ 8.54 (s, 2H), 8.41(s, 1H), 8.25 (d, *J* = 6.7 Hz, 1H), 7.97 (dd, *J* = 6.7, 2.3 Hz, 1H), 7.92 (d, *J* = 2.2 Hz, 1H), 7.51-7.46 (m, 2H), 7.43-7.37 (m, 2H), 7.34-7.29 (m, 1H), 5.43 (dd, *J* = 12.1, 4.0 Hz, 1H), 5.05-4.97 (m, 1H), 4.91-4.82 (m, 1H), 4.33 (d, *J* = 18.6 Hz, 1H), 3.71-3.58 (m, 1H), 3.39-3.33 (m, 1H), 3.02-2.88 (m, 7H), 2.66 (d, *J*= 16.1, 1.5 Hz, 1H), 2.59 (s, 3H), 1.09 (d, *J* = 6.8 Hz, 3H).

Example **16**

(*R*)-2-((1-(Cyclopropylmethyl)-1*H*-pyrazol-4-yl)amino)-*N*-((*S*)-2-(dimethylamino)-1-phenylethyl)-6-methyl-5,8-dihydropyrido[3,4-*d*]pyrimidine-7(6*H*)-carboxamide

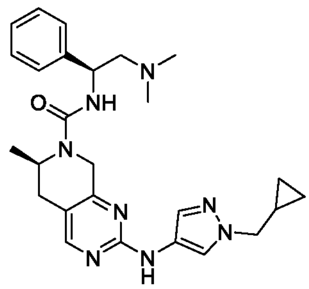

**16**

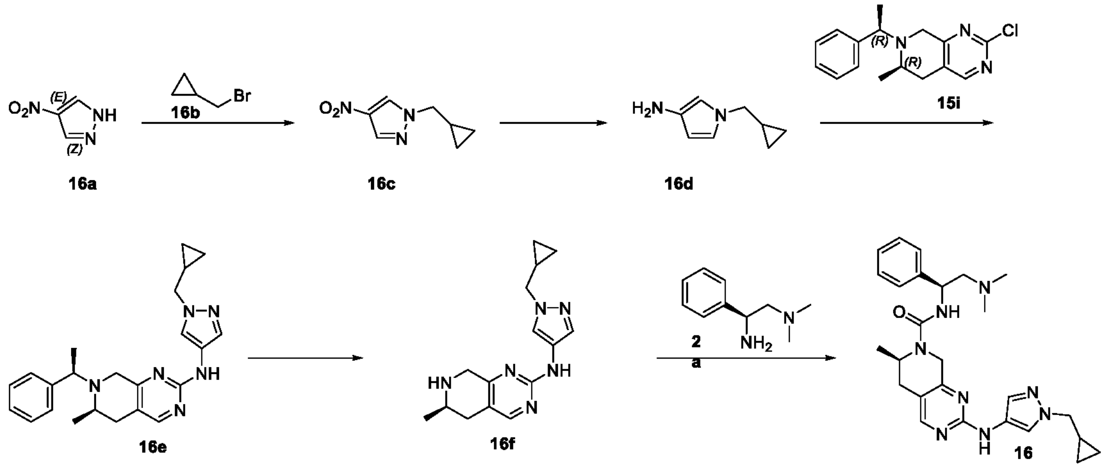


Step 1

Synthesis of compound **16c**

A solution of compound **16a** (1.0 g, 8.84 mmol), compound **16b** (1.0 mL), and $Cs_2CO_3$ (5.7 g, 17.68 mmol) in DMF (10 mL) was stirred overnight at room temperature. The reaction mixture was concentrated to give a crude product. The crude product was purified by silica gel flash column chromatography (eluent: 1-5% ethyl acetate in petroleum ether) to give compound **16c** (1.0 g, yield: 67.6%).

Step 2

Synthesis of compound **16d**

Pd/C (100 mg) was added to a solution of compound **16c** (1.0 g, 6.0 mmol) in MeOH (20 mL) at room temperature, and the reaction was stirred overnight at room temperature under a hydrogen atmosphere. The reaction mixture was concentrated to give a crude product. The crude product was purified by silica gel flash column chromatography (eluent: 1-5% ethyl acetate in petroleum ether) to give compound **16d** (800 mg, yield: 97.5%).

MS m/z (ESI): 138.3 $[M+H]^+$.

Compound **16** was synthesized by referring to compound **15. 16** (20.8 mg, 0.044 mmol) was obtained with compound **16d** (33.9 mg, 0.25 mmol) through three reactions and purification;

MS m/z (ESI): 475.4 $[M+H]^+$.

$^1H$ NMR (400 MHz, $CD_3OD$) δ 8.17 (s, 1H), 8.00 (s, 1H), 7.61 (s, 1H), 7.45-7.32 (m, 4H), 7.28-7.23 (m, 1H), 5.17 (dd, *J* = 11.0, 4.3 Hz, 1H), 4.88-4.72 (m, 2H), 4.22 (d, *J* = 18.3 Hz, 1H), 3.94 (d, *J* = 7.0 Hz, 2H), 3.11 (t, *J* = 12.9, 11.0 Hz, 1H), 3.01-2.83 (m, 1H), 2.78-2.71 (m, 1H), 2.61-2.48 (m, 7H), 1.33-1.21 (m, 1H), 1.06 (d, *J* = 6.8 Hz, 3H), 0.66-0.53 (m, 2H), 0.48-0.35 (m, 2H).

Example **17**

(R)-N-((S)-2-(Dimethylamino)-1-phenylethyl)-6-methyl-2-((1-methyl-1*H*-pyrazol-4-yl)amino)-5,8-dihydropyrido[3,4-*d*]pyrimidine-7(6*H*)-carboxamide

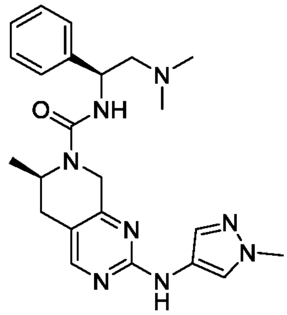

**17**

Compound **17** (10 mg, 0.023 mmol) was obtained with compound **15i** (80 mg, 0.278 mmol) through three reactions by referring to the preparation method of compound **15.** MS m/z (ESI): 435.4 $[M+H]^+$.

Example **18**

(*R*)-6-Methyl-2-(((1-methyl-1*H*-pyrazol-3-yl)methyl)amino)-*N*-((*S*)-2-(methylamino)-1-phenylethyl)-5,8-dihydropyrido[3,4-*d*]pyrimidine-7(6*H*)-carboxamide

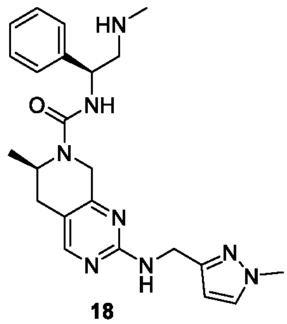

**18**

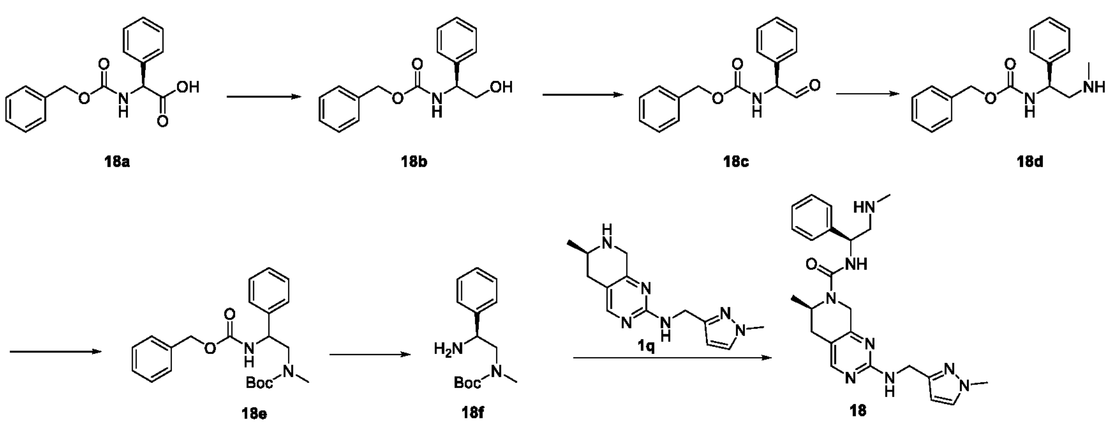
18a 18b 18c 18d 18e 18f 1q 18

Step 1

Synthesis of compound **18b**

Compound **18a** (1.1 g, 3.86 mmol) was dissolved in borane/tetrahydrofuran (20 mL, 20 mmol) at room

temperature, and the solution was stirred overnight. The reaction mixture was quenched and then concentrated to give a crude product. The crude product was purified by silica gel flash column chromatography (eluent: 1-5% ethyl acetate in petroleum ether) to give compound **18b** (700 mg, yield: 66.9%).
MS m/z (ESI): 272.3 $[M+H]^+$.

Step 2

Synthesis of compound **18c**

DMP (930 mg, 2.2 mmol) was added to a solution of compound **18a** (300 mg, 1.1 mmol) in DCM (5.0 mL) at room temperature, and the mixture was stirred at room temperature for 1 h. The reaction mixture was quenched, then extracted with ethyl acetate (100 mL × 2), and washed with saturated brine. The organic phase was dried over $Na_2SO_4$ and filtered, and the filtrate was concentrated under reduced pressure to give a crude product. The crude product was purified by silica gel flash column chromatography (eluent: 1-20% ethyl acetate in petroleum ether) to give compound **18c** (100 mg, yield: 33.6%).
MS m/z (ESI): 270.3 $[M+H]^+$.

Step 3

Synthesis of compound **18d**

Compound **18c** (100 mg, 0.37 mmol) and methylamine hydrochloride (50 mg, 0.74 mmol) were dissolved in DCM (5 mL) at room temperature, and $NaBH(OAc)_3$ (235 mg, 1.1 mmol) was slowly added in batches. The mixture was stirred overnight at room temperature. The reaction mixture was extracted with DCM/MeOH (9:1, 500 mL × 2) and washed with saturated brine. The organic phase was dried over $Na_2SO_4$ and filtered, and the filtrate was concentrated under reduced pressure to give a crude product. The crude product was purified by silica gel flash column chromatography (eluent: 1-10% ethyl acetate in petroleum ether) to give compound **18c** (60 mg, yield: 56.8%).
MS m/z (ESI): 285.3 $[M+H]^+$.

Step 4

Synthesis of compound **18e**

Di-*tert*-butyl dicarbonate (69 mg, 0.32 mmol), DMAP (2 mg), and a saturated $Na_2CO_3$ solution (1 mL) were added to a solution of compound **18c** (60 mg, 0.21 mmol) in DCM (5.0 mL) at room temperature, and the mixture was stirred at room temperature for 1 h. The reaction mixture was quenched, then extracted with ethyl acetate (100 mL × 2), and washed with saturated brine. The organic phase was dried over $Na_2SO_4$ and filtered, and the filtrate was concentrated under reduced pressure to give a crude product. The crude product was purified by silica gel flash column chromatography (eluent: 1-20% ethyl acetate in petroleum ether) to give compound **18e** (70 mg, yield: 86.3%).
MS m/z (ESI): 385.4 $[M+H]^+$.

Step 5

Synthesis of compound **18f**

10% Pd/C (30 mg) was added to a solution of compound **18e** (70 mg, 0.18 mmol) in MeOH (10 mL) at room temperature, and the reaction was stirred overnight at room temperature under a hydrogen atmosphere. The reaction mixture was concentrated to give a crude product. The crude product was purified by silica gel flash column chromatography (eluent: 1-5% ethyl acetate in petroleum ether) to give compound **18f** (30 mg, yield: 65.8%).
MS m/z (ESI): 251.5 $[M+H]^+$.
Compound **18** (22 mg, 0.049 mmol) was obtained through two reactions, urea formation and Boc removal, by referring to the synthesis of Example **11.**
MS m/z (ESI): 251.5 $[M+H]^+$.
$^1H$ NMR (400 MHz, $CD_3OD$) δ 8.09 (s, 1H), 7.48-7.46 (m, 1H), 7.41-7.34 (m, 4H), 7.32-7.26 (m, 1H), 6.19 (d, J = 2.3 Hz, 1H), 5.22-5.12 (m, 1H), 4.81-4.68 (m, 2H), 4.54 (s, 2H), 4.17 (d, J = 18.3 Hz, 1H), 3.84 (s, 3H), 3.25-3.13 (m, 2H), 2.95-2.85 (m, 1H), 2.61 (s, 3H), 2.53 (d, J = 15.8, 1.6 Hz, 1H), 1.07 (d, J = 6.8 Hz, 3H).

Example **19**

(*R*)-*N*-((*S*)-2-(Bis(methyl-$d_3$)amino)-1-phenylethyl)-6-methyl-2-(((*S*)-1-(pyridin-2-yl)ethyl)amino)-5,8-dihydropyrido[3,4-*d*]pyrimidine-7(6*H*)-carboxamide

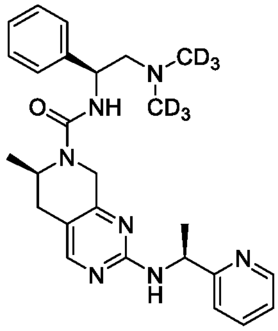

**19**

Compound **19** (15 mg, 0.032 mmol) was synthesized by referring to the synthesis of Example **6.**
MS m/z (ESI): 465.4 $[M+H]^+$.
$^1H$ NMR (400 MHz, $CDCl_3$): δ 8.49 (d, J = 4.4 Hz, 1H), 7.99 (s, 1H), 7.56 (td, J = 7.7, 1.8 Hz, 1H), 7.26-7.15 (m, 6H), 7.11-7.02 (m, 1H), 6.11-5.86 (m, 2H), 5.19-5.10 (m, 1H), 4.81-4.75 (m, 1H), 4.70-4.65 (m, 1H), 4.58-4.53 (d, J = 18.1 Hz, 1H), 4.08 (d, J = 18.1 Hz, 1H), 2.85 (dd, J = 15.4, 5.5 Hz, 1H), 2.70-2.65 (m, 1H), 2.42-2.32 (m, 2H), 1.49 (d, J = 6.8 Hz, 3H), 0.97 (d, J = 6.7 Hz, 3H).

## Biological Evaluations

The present disclosure is further described and explained below with reference to test examples, but these examples are not intended to limit the scope of the present disclosure.

### Test Example 1

Test Example 1. Assay for Inhibitory Activity of Compounds of Present Disclosure Against Ovarian Cancer Cells (OVCAR3)

1.1. Experimental materials and instruments (see Table 1)

Table 1. Experimental materials and instruments

| Instrument name | Equipment manufacturer | Model |
|---|---|---|
| RPMI 1640 meduim | Gibco | A1049101 |
| FBS | Gibco | 11965118 |
| CellTiter-Glo® kits | Promega | G7572 |
| White CulturePlate 96-well | Corning | 3610 |
| Envision | PerkinElmer | 2015 |
| Plate shaker | Beijing Jiayuan | MB-100-2A |

1.2. Experimental procedure

OVCAR3 ovarian cancer cells were cultured in a cell incubator at 37% with 5% $CO_2$ using RPMI 1640 medium with 10% FBS. On the first day, the cells were plated in a 96-well plate at a cell concentration of 2500 cells/well and cultured overnight in the incubator. On the second day, the cells were treated with compounds. The maximum compound concentration was 10 μM and was 3-fold diluted to obtain 9 concentrations, and the final concentration of DMSO was 0.1%. After the cells were cultured in the incubator for another 5 days, cell viability was tested using the Celltiter Glo assay

kit (Promega), and the testing method was consistent with the operational method provided by the kit. Data were processed using GraphPad Prism 8, and $IC_{50}$ was calculated.

$$\text{Calculation formula: } Y = \text{Bottom} + (\text{Top} - \text{Bottom})/(1 + 10\string^((\text{LogIC50} - X) \times \text{HillSlope})).$$

X: the logarithm of the compound concentration; Y: % inhibition.

Table 2. The $IC_{50}$ (nM) of the compounds of the present disclosure against OVCAR3

| Example No. | $IC_{50}$ (nM) |
|---|---|
| Compound **1-2** | 11.49 |
| Example **2** | 24.25 |
| **5** | 81.24 |
| **6** | 27.74 |
| **7** | 6.44 |
| **8** | 58.26 |
| **9** | 13.81 |
| **10** | 42.63 |
| **12** | 33.92 |
| **13** | 21.7 |
| **14** | 40.53 |
| **15** | 26.69 |
| **16** | 9.99 |
| **17** | 22.49 |
| **19** | 26.15 |

The activity of the compounds of Examples **2, 5-10, 12-17,** and **19** of the present disclosure is superior to that of the compound of Example **18.**

**Test Example 2**

Test Example 2. Assay for Inhibitory Activity of Compounds of Present Disclosure Against Tumor Cells

2.1. Experimental materials and instruments (see Table 3)

Table 3. Experimental materials and instruments

| Name | Manufacturer | Model |
|---|---|---|
| RPMI 1640 meduim | Gibco | 11875093 |
| DMEM | Gibco | 11965092 |
| IMDM | Gibco | 12440053 |
| MC'5A | Gibco | 16600082 |
| FBS | Gibco | 10091148 |
| CellTiter-Glo® kits | Promega | G7572 |
| White CulturePlate 384-well | Corning | 781098 |
| Envision | PerkinElmer | 2015 |

2.2. Experimental procedure

MCF7 (ATCC# HTB-22) and T47D (ATCC# HTB-133) breast cancer cells and PANC-1 (ATCC# CRL-1469) pancreatic cancer cells were purchased from ATCC. MDA-MB-231 (Cobioer# CBP60382), HCC1806 (Cobioer# CBP60373), and MDA-MB-468 (Cobioer# CBP60387) breast cancer cells, HCT-116 (Cobioer# CBP60028) colorectal cancer cells, and OCI-AML-3 (Cobioer# CBP60817) and MV-4-11 (Cobioer# CBP60522) leukemia cells were purchased from Nanjing Cobioer Biosciences Co., Ltd.

OCI-AML-3 was cultured in RPMI 1640 medium with 20% FBS; HCC1806 and T47D were cultured in RPMI 1640 medium with 10% FBS; MCF7, MDA-MB-231, MDA-MB-468, and PANC-1 were cultured in DMEM medium with 10% FBS; MV-4-11 was cultured in IMDM medium with 20% FBS; HCT-116 was cultured in MC'5A medium with 10% FBS.

Palbociclib-resistant cells were constructed on MCF7 parent cells, designated MCF7 Palbo-R, and the culture conditions were consistent with those of the parent cells. All tumor cells were cultured in a cell incubator at 37 °C with 5% $CO_2$. The cells were seeded at corresponding cell densities (600 cells/well for MDA-MB-231; 500 cells/well for MDA-MB-468, PANC-1, and HCT-116; 200 cells/well for MCF7 and HCC1806; 800 cells/well for MCF7 Palbo-R; 3000 cells/well for OCI-AML-3 and MV-4-11) in 384-well plates and cultured overnight in the incubator. On the second day, the cells were treated with compounds. The maximum compound concentration was 10 μM and was 3-fold diluted to obtain 9 concentrations, each tested in duplicate, and the final concentration of DMSO was 0.1%. The cells continued to be cultured in the incubator, with MDA-MB-231, PANC-1, MDA-MB-468, OCI-AML-3, and MV-4-11 being treated with drugs for 5 days and HCC1806, MCF7, MCF7 Palbo-R, and HCT116 being treated with drugs for 7 days. Cell viability was tested using the Celltiter Glo assay kit (Promega), and the testing method was consistent with the operational method provided by the kit. Readings were taken using an Envision multimode microplate reader. Data were processed using GraphPad Prism 8, and $IC_{50}$ was calculated.

$$\text{Calculation formula: } Y = \text{Bottom} + (\text{Top} - \text{Bottom})/(1 + 10\text{^}((\text{LogIC}_{50} - X) \times \text{HillSlope})).$$

X: the logarithm of the compound concentration; Y: % inhibition.

Table 4. The $IC_{50}$ (nM) of the compounds of the present disclosure against test tumor cells

| Example | MCF7 (nM) | MCF7/Palbo-R (nM) | T47D (nM) | PANC-1 (nM) | MDA-MB-231 (nM) |
|---|---|---|---|---|---|
| **2** | 14.64 (n = 3) | 40.63 (n = 3) | 31.02 | 45.91 (n = 2) | 25.44 (n = 5) |
| **6** | 11.75 (n = 3) | 24.63 (n = 3) | 22.21 | 17.68 (n = 2) | 23.23 (n = 4) |
| **12** | 21.67 (n = 3) | 43.27 (n = 3) | 41.49 | 50.32 | 25.8 (n = 2) |
| **19** | NT | NT | 14.10 | NT | NT |

Table 5. The $IC_{50}$ (nM) of the compounds of the present disclosure against test tumor cells

| Example | HCC1806 (nM) | MDA-MB-468 (nM) | HCT-116 (nM) | OCI-AML-3 (nM) | MV-4-11 (nM) |
|---|---|---|---|---|---|
| **2** | 28.55 (n = 4) | 30.02 | 75.62 | 33.24 (n = 2) | 18.24 (n = 2) |
| **6** | 23.68 (n = 2) | 21.25 | 46.93 | 33.44 (n = 2) | 19.12 |
| **12** | 28.54 (n = 4) | 32.38 | 65.95 (n = 3) | 47.1 (n = 2) | 27.28 |
| **19** | NT | NT | 29.24 | NT | NT |

The data in Tables 4 and 5 show that Examples **2, 6, 12,** and **19** all exhibited high inhibitory activity against the various tumor cell lines.

**Test 3**

Test Example 3. Assay for Inhibitory Activity of Compounds of Present Disclosure Against CDKs

3.1. Experimental procedure

The ADP-Glo kinase assay was used for testing the activity of CDKs. The compounds were diluted in 384-well plates using Echo. The starting concentration was 10 μM and was 3-fold diluted to obtain 10 concentration points, each

tested in duplicate, and the final concentration of DMSO in the assay system was 1%. CDK solutions (the final concentrations were 16.5 nM CDK1/CyclinB, 1 nM CDK2/CyclinE1, 16.3 nM CDK4/CyclinD1, 11.5 nM CDK5/p25, 15.7 nM CDK6/CyclinD3, 80 nM CDK7/Cyclin H/MAT1, 15.3 nM CDK9/Cyclin T1, and 150 nM CDK12/CyclinK) prepared in assay buffer were added at 2.5 μL, and the enzymes and compounds were pre-incubated at room temperature for 10 min. ATP (the concentration was $K_m$) & substrate solutions (20 μM ATP & 0.1 mg/mL Histone H1 Protein for CDK1/CyclinB, 15 μM ATP & 0.1 mg/mL Histone H1 Protein for CDK2/CyclinE1, 200 μM ATP & 0.2 mg/mL DYRKtide peptide for CDK4/CyclinD1, 10 μM ATP & 0.1 mg/mL Histone H1 Protein for CDK5/p25, 200 μM ATP & 0.1 mg/mL Histone H1 Protein for CDK6/CyclinD3, 70 μM ATP & 0.2 mg/mL MBP for CDK7/Cyclin H/MAT1, 60 μM ATP & 0.2 mg/mL PDKtide for CDK9/Cyclin T1, and 20 μM ATP & 80 μM pS7-CTD peptide for CDK12/CyclinK) prepared in assay buffer were added at 2.5 μL. After thorough mixing, the plates were incubated at room temperature, with CDK1/4/9/12/13 incubated for 120 min and CDK2/5/6/7 incubated for 60 min. ADP-Glo reagents were added at 4 μL, and the plates were incubated at room temperature for 40 min. Kinase detection reagents were added at 8 μL, and the plates were incubated at room temperature for 40 min. Readings were taken using an Envision multimode microplate reader. Data were processed using GraphPad Prism 8, and $IC_{50}$ was calculated.

$$\text{Calculation formula: } Y = \text{Bottom} + (\text{Top} - \text{Bottom})/(1 + 10\text{^}((\text{LogIC50} - X) \times \text{HillSlope})).$$

X: the logarithm of the compound concentration; Y: % inhibition.

Table 6. The inhibitory activity ($IC_{50}$, nM) of the compounds of the present disclosure against CDKs

| Example | CDK1 | CDK2 | CDK4 | CDK 6 | CDK5 | CDK7 | CDK9 | CDK12 |
|---|---|---|---|---|---|---|---|---|
| **2** | >10000 | 2334 | >10000 | >10000 | 6309 | 21.87 | 1582 | >10000 |
| **6** | >10000 | 1903 | >10000 | >10000 | 6415 | 28.42 | 4274 | >10000 |
| **Janssen-01** | NT | 932.8 | NT | NT | NT | 25.19 | NT | NT |

The data in Table 6 show that Examples **2** and **6** exhibited significantly better selectivity for CDK2/CDK7 than **Janssen-01. Janssen-01**

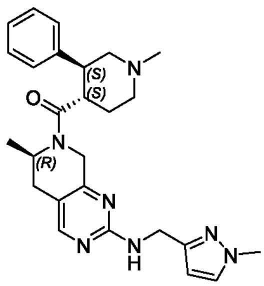

was prepared by referring to the method provided in WO2022064009A.

**Test 4**

Test Example 4. Human Liver Microsome Metabolism Study

222.5 μL of human liver microsomes (protein concentration: 1 mg/mL) and 25 μL of NADPH (10 nM) were added to an incubation plate and pre-heated for 10 min. 2.5 μL of control compound and test compound (100 μM) were added.
30-μL aliquots were taken from the reaction solution at 0.5 min, 5 min, 10 min, 15 min, 20 min, and 30 min. The reaction was stopped by adding 5 volumes of cold acetonitrile containing IS (100 nM alprazolam, 200 nM caffeine, and 100 nM tolbutamide). The reaction mixture was centrifuged, and 100 μL of the supernatant was collected and mixed with 100 μL of ultrapure $H_2O$. Then the mixture was analyzed by LC-MS/MS.
The slope value k was determined by linear regression of the natural logarithm of a curve of the parent drug remaining percentage versus incubation time.
The *in-vitro* half-life (*in-vitro* $T_{1/2}$) was determined by the slope value:

$$T1/2 = -(0.693/k)$$

Using the following equation (the average of repeated measurements), the *in-vitro* T1/2 (min) was converted to the *in-vitro* intrinsic clearance rate (*in-vitro* $CL_{int}$, in µL/min/mg protein):

$$CL_{int} = \left(\frac{0.693}{T1/2}\right) * \left(\frac{\text{Incubation amount (µL)}}{\text{Protein amount (mg)}}\right)$$

Table 7. The residues of the test compounds at various time points during the incubation of human liver microsomes

| Time / Example | 0.5 min | 5 min | 15 min | 30 min | 60 min |
|---|---|---|---|---|---|
| | Remaining amount % | | | | |
| **2** | 100.00 | 105.55 | 96.07 | 100.48 | 81.83 |
| **Janssen-01** | 100.00 | 91.32 | 78.60 | 68.95 | 55.59 |

Table 8. The metabolic clearance half-lives and clearance rates of the test compounds in human liver microsomes

| Parameter / Example | $T_{1/2}$(min) | CLint (µL/min/mg) |
|---|---|---|
| **2** | > 184.78 | < 7.50 |
| **Janssen-01** | 72.97 | 19.00 |

Tables 7 and 8 show that, compared to **Janssen-01,** Example **2** resulted in more parent drug remaining, as detected at the various time points during the incubation of human liver microsomes, and exhibited a significantly longer clearance half-life and a significantly slower clearance rate, indicating a more stable metabolism.

## Claims

1. A compound represented by formula (I) or a pharmaceutically acceptable salt thereof,

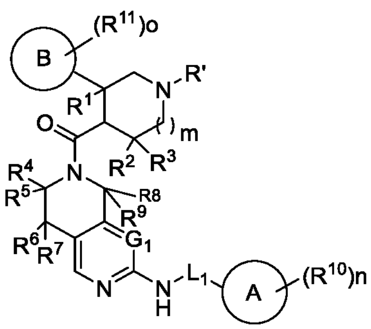

(I)

,

wherein $G_1$ is N or $CR^{1a}$;

$R^1$, $R^2$, $R^3$, and $R^{1a}$ are each independently selected from the group consisting of hydrogen, deuterium, cyano, hydroxy, $C_{1-6}$ alkyl, and halogen (e.g., fluorine, chlorine, bromine, or iodine), and at least one thereof is not

hydrogen or deuterium;

$R^4$, $R^5$, $R^6$, $R^7$, $R^8$, and $R^9$ are each independently selected from the group consisting of hydrogen, deuterium, halogen, $C_{1-6}$ alkyl, cyano, $C_{2-6}$ alkenyl, $C_{2-6}$ alkynyl, and 3- to 6-membered cycloalkyl, wherein the $C_{1-6}$ alkyl, $C_{2-6}$ alkenyl, $C_{2-6}$ alkynyl, or 3- to 6-membered cycloalkyl is optionally substituted with one or more substituents independently selected from the group consisting of $R^A$, and $R^A$ is selected from the group consisting of deuterium, halogen, hydroxy, cyano, and 3- to 6-membered cycloalkyl;

$L_1$ is selected from the group consisting of a chemical bond and $C_{1-6}$ alkylene, wherein the $C_{1-6}$ alkylene is optionally substituted with one or more substituents independently selected from the group consisting of $R^B$, and $R^B$ is selected from the group consisting of deuterium, halogen, hydroxy, $C_{1-6}$ alkoxy, amino, and oxo, or two $R^B$ attached to the same carbon atom, together with the carbon atom to which they are both attached, form 3- to 6-membered cycloalkyl or 3- to 7-membered heterocyclyl containing at least one heteroatom selected from the group consisting of N, O, and S;

ring A is selected from the group consisting of 3- to 6-membered cycloalkyl, 6- to 10-membered aryl, 5- to 12-membered heteroaryl, and 3- to 12-membered heterocyclyl; each $R^{10}$ is independently selected from the group consisting of deuterium, cyano, halogen, hydroxy, amino, $C_{2-6}$ alkynyl, $C_{2-6}$ alkenyl, -S-$C_{1-6}$ alkyl, $C_{1-6}$ alkoxy, oxo, $C_{1-6}$ alkyl, 3- to 6-membered cycloalkyl, 6- to 10-membered aryl, 5- to 12-membered heteroaryl, 3- to 12-membered heterocyclyl, -NH-(C=O)-$C_{1-6}$ alkyl, -NH-(C=O)-$C_{3-6}$ cycloalkyl, -NH(C=O)-O$C_{1-6}$ alkyl, -NH(C=O)-O$C_{3-6}$ cycloalkyl, -O(C=O)NH$C_{1-6}$ alkyl, -O(C=O)NH-$C_{3-6}$ cycloalkyl, -(C=O)NH-$C_{1-6}$ alkyl, -(C=O)-NH-$C_{3-6}$ cycloalkyl, -(C=O)-$C_{1-6}$ alkyl, -(C=O)-$C_{3-6}$ cycloalkyl, -$SO_2$-$C_{1-6}$ alkyl, -$SO_2$-$C_{3-6}$ cycloalkyl, -$SO_2$-$NH_2$, - $SO_2$-NH-$C_{1-6}$ alkyl, -$SO_2$-NH-$C_{3-6}$ cycloalkyl, -$SO_2$-N($C_{1-6}$ alkyl)$_2$, -$SO_2$-NH($C_{3-6}$ cycloalkyl)$_2$, -S(O)(NH)-$C_{1-6}$ alkyl, and -S(O)(NH)-$C_{3-6}$ cycloalkyl, wherein

the $C_{1-6}$ alkyl, 3- to 6-membered cycloalkyl, 6- to 10-membered aryl, 5- to 12-membered heteroaryl, or 3- to 12-membered heterocyclyl is optionally substituted with one or more substituents independently selected from the group consisting of $R^C$, and $R^C$ is selected from the group consisting of deuterium, halogen, $C_{1-6}$ alkoxy, hydroxy, amino, oxo, $C_{2-6}$ alkynyl, $C_{2-6}$ alkenyl, cyano, $C_{1-6}$ hydroxyalkyl, 3- to 12-membered heterocyclyl, $C_{1-6}$ alkyl, $C_{3-6}$ cycloalkyl, 6- to 10-membered aryl, and 5- to 12-membered heteroaryl;

$R^{11}$ is selected from the group consisting of hydrogen, deuterium, halogen, cyano, $C_{1-6}$ alkyl, $C_{1-6}$ alkoxy, 3- to 6-membered cycloalkyl, 3- to 12-membered heterocyclyl, $C_{2-6}$ alkenyl, and $C_{2-6}$ alkynyl, wherein the $C_{1-6}$ alkyl, $C_{1-6}$ alkoxy, 3- to 6-membered cycloalkyl, 3- to 12-membered heterocyclyl, $C_{2-6}$ alkenyl, or $C_{2-6}$ alkynyl is optionally substituted with one or more substituents independently selected from the group consisting of $R^D$, and $R^D$ is selected from the group consisting of hydrogen, deuterium, halogen, cyano, hydroxy, alkynyl, $C_{1-6}$ alkoxy, $C_{1-6}$ hydroxyalkyl, and 3- to 6-membered cycloalkyl;

ring B is selected from the group consisting of 5- to 12-membered heteroaryl and 6- to 12-membered aryl;

R' is selected from the group consisting of hydrogen, cyano, $C_{1-6}$ alkyl, 3- to 6-membered cycloalkyl, and 3- to 12-membered heterocyclyl, wherein the $C_{1-6}$ alkyl, 3- to 6-membered cycloalkyl, or 3- to 12-membered heterocyclyl is optionally substituted with one or more substituents independently selected from the group consisting of $R^E$, and $R^E$ is selected from the group consisting of hydrogen, deuterium, halogen, cyano, hydroxy, alkynyl, $C_{1-6}$ alkoxy, $C_{1-6}$ hydroxyalkyl, 3- to 6-membered cycloalkyl, and 3- to 7-membered heterocyclyl;

m is selected from the group consisting of 0 and 1;

n is selected from the group consisting of 0, 1, 2, 3, 4, and 5;

o is selected from the group consisting of 0, 1, 2, 3, and 4.

**2.** A compound represented by formula (VI) or a pharmaceutically acceptable salt thereof,

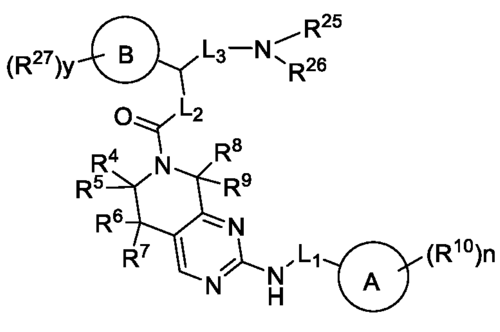

(VI)

wherein $L_2$ is selected from the group consisting of -NH- and -O-;

ring B is selected from the group consisting of 5- to 12-membered heteroaryl and 6- to 12-membered aryl;
$L_3$ is selected from the group consisting of $C_{0-6}$ alkylene, wherein the $C_{0-6}$ alkylene is optionally substituted with one or more substituents independently selected from the group consisting of $R^I$, and $R^I$ is selected from the group consisting of deuterium, halogen, oxo, hydroxy, amino, and $C_{1-6}$ alkyl;
$R^{25}$ and $R^{26}$ are each independently selected from the group consisting of hydrogen, $C_{1-6}$ alkyl, and $C_{1-6}$ haloalkyl, or $R^{25}$ and $R^{26}$, together with the nitrogen atom to which they are attached, form a 4- to 7-membered nitrogen-containing heterocyclic ring, wherein the 4- to 7-membered nitrogen-containing heterocyclic ring is optionally substituted with one or more substituents independently selected from the group consisting of $R^J$, and $R^J$ is selected from the group consisting of halogen, hydroxy, cyano, nitro, and amino;
each $R^{27}$ is independently selected from the group consisting of deuterium, halogen, hydroxy, carboxyl, $C_{1-6}$ alkyl, $C_{1-6}$ haloalkyl, $C_{1-6}$ hydroxyalkyl, $C_{2-6}$ alkenyl, $C_{2-6}$ alkynyl, 3- to 6-membered cycloalkyl, -S-$C_{1-6}$ alkyl, $C_{1-6}$ alkoxy, $C_{1-6}$ haloalkoxy, -NH-$C_{1-6}$ alkyl, -NH($C_{1-6}$ alkyl)$_2$, -(C=O)-$NH_2$, alkyl-(C=O)-NH-$C_{1-6}$ alkyl, -(C=O)-NH-($C_{1-6}$ alkyl)$_2$, - (C=O)$C_{1-6}$ alkyl, and -NH-(C=O)$C_{1-6}$ alkyl;
y is selected from the group consisting of 0, 1, 2, 3, and 4;
$R^4$, $R^5$, $R^6$, $R^7$, $R^8$, $R^9$, $L_1$, ring A, $R^{10}$, and n are each as defined in claim 1, provided that $R^4$ and $R^5$ are not simultaneously hydrogen.

**3.** The compound represented by formula (VI) or the pharmaceutically acceptable salt thereof according to claim 2, wherein $L_2$ is -NH-.

**4.** The compound represented by formula (VI) or the pharmaceutically acceptable salt thereof according to claim 2, wherein $L_2$ is -O-.

**5.** The compound represented by formula (VI) or the pharmaceutically acceptable salt thereof according to any one of claims 2 to 4, wherein $L_3$ is -$CH_2CH_2$-.

**6.** The compound represented by formula (VI) or the pharmaceutically acceptable salt thereof according to any one of claims 2 to 5, wherein ring B is phenyl or pyridyl, preferably phenyl.

**7.** The compound represented by formula (VI) or the pharmaceutically acceptable salt thereof according to any one of claims 2 to 6, wherein each $R^{27}$ is independently selected from the group consisting of hydrogen, deuterium, halogen, cyano, hydroxy, carboxyl, $C_{1-6}$ alkyl, $C_{1-6}$ haloalkyl, $C_{1-6}$ hydroxyalkyl, $C_{2-6}$ alkynyl, 3- to 6-membered cycloalkyl, 3- to 12-membered heterocycloalkyl, 5- to 12-membered aryl, and heteroaryl, preferably from the group consisting of halogen, $C_{1-6}$ alkyl, $C_{1-6}$ haloalkyl, and $C_{2-6}$ alkynyl, and most preferably from the group consisting of chlorine, fluorine, trifluoromethyl, and ethynyl.

**8.** The compound represented by formula (VI) or the pharmaceutically acceptable salt thereof according to any one of claims 2 to 7, wherein $R^{25}$ and $R^{26}$ are each independently selected from the group consisting of hydrogen and $C_{1-6}$ alkyl.

**9.** The compound or the pharmaceutically acceptable salt thereof according to any one of claims 1 to 8, wherein $R^4$, $R^5$, $R^6$, $R^7$, $R^8$, and $R^9$ are each independently selected from the group consisting of hydrogen, deuterium, halogen, and $C_{1-6}$ alkyl.

**10.** The compound or the pharmaceutically acceptable salt thereof according to claim 9, wherein $R^4$ is methyl.

**11.** The compound or the pharmaceutically acceptable salt thereof according to claim 10, wherein $R^5$, $R^6$, $R^7$, $R^8$, and $R^9$ are each independently selected from the group consisting of hydrogen and deuterium.

**12.** The compound or the pharmaceutically acceptable salt thereof according to any one of claims 1 to 11, wherein $L_1$ is a chemical bond.

**13.** The compound or the pharmaceutically acceptable salt thereof according to any one of claims 1 to 12, wherein $L_1$ is selected from the group consisting of $C_{1-6}$ alkylene, wherein the $C_{1-6}$ alkylene is optionally substituted with one or more substituents independently selected from the group consisting of $R^B$, and $R^B$ is selected from the group consisting of deuterium, halogen, hydroxy, and $C_{1-6}$ alkoxy; preferably, $L_1$ is methylene, wherein the methylene is optionally substituted with one or more substituents independently selected from the group consisting of $R^B$, and $R^B$ is selected from the group consisting of deuterium, halogen, hydroxy, and $C_{1-6}$ alkoxy.

**14.** The compound or the pharmaceutically acceptable salt thereof according to any one of claims 1 to 13, wherein ring A is selected from the group consisting of a pyrazole ring, an imidazole ring, a tetrahydropyran ring, a pyrimidine ring, and cyclohexyl.

**15.** The compound or the pharmaceutically acceptable salt thereof according to any one of claims 1 to 14, wherein $R^{10}$ is selected from the group consisting of deuterium, cyano, $C_{2-6}$ alkynyl, -S-$C_{1-6}$ alkyl, $C_{1-6}$ alkoxy, and $C_{1-6}$ alkyl, wherein the $C_{2-6}$ alkynyl, -S-$C_{1-6}$ alkyl, $C_{1-6}$ alkoxy, or $C_{1-6}$ alkyl is optionally substituted with one or more substituents independently selected from the group consisting of $R^C$, and $R^C$ is selected from the group consisting of deuterium, halogen, $C_{1-6}$ alkoxy, hydroxy, amino, oxo, and $C_{2-6}$ alkynyl.

**16.** The compound or the pharmaceutically acceptable salt thereof according to any one of claims 1 to 15, wherein

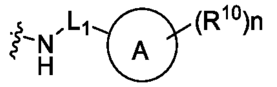

is selected from the group consisting of

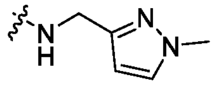

,

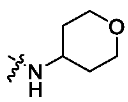

,

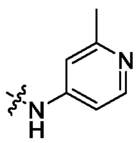

,

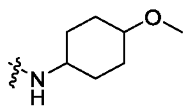

,

and

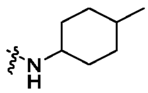

.

**17.** The compound represented by formula (VI) or the pharmaceutically acceptable salt thereof according to claim 2, being a compound represented by formula (VI-1) or formula (VI-2) or a pharmaceutically acceptable salt thereof, preferably a compound represented by formula (VI-1) or a pharmaceutically acceptable salt thereof,

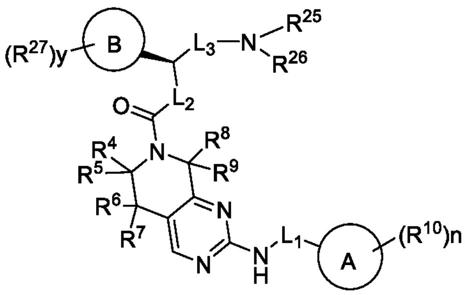

(VI-1)

or

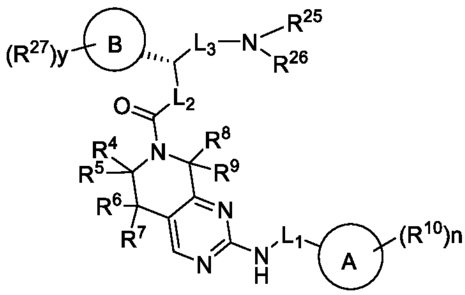

(VI-2)

,

wherein $L_2$ is selected from the group consisting of -NH- and -O-;
ring B is selected from the group consisting of 5- to 6-membered heteroaryl and 5- to 6-membered aryl;
$L_3$ is selected from the group consisting of $C_{1-3}$ alkylene (e.g., methylene), wherein the $C_{1-3}$ alkylene is optionally substituted with one or more substituents independently selected from the group consisting of $R^I$, and $R^I$ is selected from the group consisting of deuterium, halogen, oxo, hydroxy, amino, and $C_{1-6}$ alkyl;
$R^{25}$ and $R^{26}$ are each independently selected from the group consisting of hydrogen and $C_{1-6}$ alkyl (e.g., methyl or

ethyl), wherein the $C_{1-6}$ alkyl is optionally substituted with one or more deuterium atoms;

each $R^{27}$ is independently selected from the group consisting of deuterium, halogen, hydroxy, carboxyl, $C_{1-6}$ alkyl (e.g., methyl), $C_{1-6}$ haloalkyl, $C_{1-6}$ hydroxyalkyl, $C_{2-6}$ alkenyl, $C_{2-6}$ alkynyl, $C_{1-6}$ alkylene-3- to 6-membered cycloalkyl, 3- to 6-membered cycloalkyl, -S-$C_{1-6}$ alkyl, $C_{1-6}$ alkoxy, $C_{1-6}$ haloalkoxy, -NH-$C_{1-6}$ alkyl, and -NH($C_{1-6}$ alkyl)$_2$;

y is selected from the group consisting of 0, 1, 2, 3, and 4;

$R^4$ is $C_{1-6}$ alkyl;

$R^5$, $R^6$, $R^7$, $R^8$, and $R^9$ are each independently selected from the group consisting of hydrogen, deuterium, halogen, and $C_{1-6}$ alkyl;

$L_1$ is selected from the group consisting of a linking bond; or $L_1$ is selected from the group consisting of $C_{1-6}$ alkylene (e.g., methylene), wherein the $C_{1-6}$ alkylene is substituted with one or more substituents independently selected from the group consisting of $R^B$, and $R^B$ is selected from the group consisting of deuterium, halogen, hydroxy, $C_{1-6}$ alkyl (e.g., methyl), and oxo;

ring A is selected from the group consisting of 5- to 6-membered aryl, 5- to 6-membered heteroaryl, and 3- to 7-membered heterocyclyl;

$R^{10}$ is selected from the group consisting of deuterium, cyano, $C_{2-6}$ alkynyl, -S-$C_{1-6}$ alkyl, $C_{1-6}$ alkoxy, $C_{1-6}$ alkyl (e.g., methyl), $C_{1-6}$ alkylene-3- to 6-membered cycloalkyl, 3- to 6-membered cycloalkyl, 5- to 6-membered aryl, 5- to 6-membered heteroaryl, 5- to 12-membered heterocycloalkyl, -NH(C=O)-O$C_{1-6}$ alkyl, -(C=O)NH-$C_{1-6}$ alkyl, and (C=O)$NH_2$, wherein

the $C_{2-6}$ alkynyl, -S-$C_{1-6}$ alkyl, $C_{1-6}$ alkoxy, $C_{1-6}$ alkyl, 5- to 6-membered aryl, 5- to 6-membered heteroaryl, or 5- to 12-membered heterocycloalkyl is optionally substituted with one or more substituents independently selected from the group consisting of $R^C$, and $R^C$ is selected from the group consisting of deuterium, halogen, $C_{1-6}$ alkoxy, $C_{1-6}$ alkyl (e.g., methyl), hydroxy, amino, oxo, and $C_{2-6}$ alkynyl;

n is selected from the group consisting of 0, 1, 2, and 3.

**18.** The compound or the pharmaceutically acceptable salt thereof according to claim 17, wherein $L_2$ is -NH-.

**19.** The compound or the pharmaceutically acceptable salt thereof according to any one of claim 17 or 18, wherein ring B is selected from the group consisting of pyridyl and phenyl, preferably from phenyl, and each $R^{27}$ is independently selected from the group consisting of deuterium, halogen, hydroxy, carboxyl, $C_{1-6}$ alkyl, $C_{2-6}$ alkenyl, $C_{2-6}$ alkynyl, $C_{1-6}$ alkylene-3- to 6-membered cycloalkyl, and 3- to 6-membered cycloalkyl.

**20.** The compound or the pharmaceutically acceptable salt thereof according to any one of claims 17 to 19, being a compound represented by formula (VI-1-A) or formula (VI-1-B) or a pharmaceutically acceptable salt thereof, preferably a compound represented by formula (VI-1-A) or a pharmaceutically acceptable salt thereof,

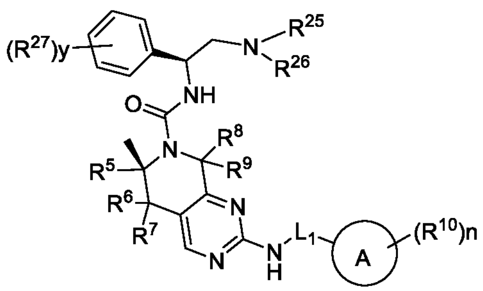

(VI-1-A)

or

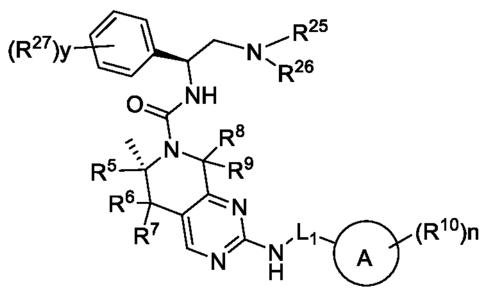

(VI-1-B)

,

wherein $R^5$, $R^6$, $R^7$, $R^8$, $R^9$, $L_1$, ring A, $R^{10}$, n, $R^{25}$, $R^{26}$, $R^{27}$, and y are each as defined in claim 17.

**21.** The compound or the pharmaceutically acceptable salt thereof according to any one of claims 17 to 20, wherein $L_1$ is a linking bond.

**22.** The compound or the pharmaceutically acceptable salt thereof according to any one of claims 17 to 20, wherein $L_1$ is selected from the group consisting of $C_{1-6}$ alkylene; or $L_1$ is selected from the group consisting of $C_{1-6}$ alkylene, wherein the $C_{1-6}$ alkylene is substituted with one or more substituents independently selected from the group consisting of $R^B$, and $R^B$ is selected from the group consisting of deuterium, $C_{1-6}$ alkyl (e.g., methyl), and oxo.

**23.** The compound or the pharmaceutically acceptable salt thereof according to claim 22, wherein $L_1$ is selected from the group consisting of methylene; or $L_1$ is selected from the group consisting of methylene, wherein the methylene is substituted with one or more substituents independently selected from the group consisting of $R^B$, and $R^B$ is selected from the group consisting of deuterium and $C_{1-6}$ alkyl (e.g., methyl).

**24.** The compound or the pharmaceutically acceptable salt thereof according to claim 22, wherein $L_1$ is selected from the group consisting of methylene, wherein the methylene is substituted with one or more substituents independently selected from the group consisting of $R^B$, and $R^B$ is selected from the group consisting of oxo.

**25.** The compound or the pharmaceutically acceptable salt thereof according to any one of claims 17 to 24, wherein $R^5$, $R^6$, $R^7$, $R^8$, and $R^9$ are each independently selected from the group consisting of hydrogen and deuterium.

**26.** The compound or the pharmaceutically acceptable salt thereof according to any one of claims 17 to 25, wherein ring A is selected from the group consisting of pyrazolyl, imidazolyl, pyridyl, phenyl, tetrahydropyranyl, pyrimidinyl, and cyclohexyl.

**27.** The compound or the pharmaceutically acceptable salt thereof according to any one of claims 17 to 26, wherein $R^{10}$ is selected from the group consisting of deuterium, $C_{1-6}$ alkyl, $C_{1-6}$ alkylene-3- to 6-membered cycloalkyl, 3- to 6-membered cycloalkyl, 5- to 6-membered aryl, 5- to 6-membered heteroaryl, 5- to 12-membered heterocycloalkyl, -NH(C=O)-O$C_{1-6}$ alkyl or -(C=O)NH-$C_{1-6}$ alkyl, and (C=O)$NH_2$, wherein

the $C_{1-6}$ alkyl, 5- to 6-membered aryl, or 5- to 6-membered heteroaryl is optionally substituted with one or more substituents independently selected from the group consisting of $R^C$, and $R^C$ is selected from the group consisting of deuterium, halogen, $C_{1-6}$ alkoxy, $C_{1-6}$ alkyl, hydroxy, amino, oxo, and $C_{2-6}$ alkynyl;
n is selected from the group consisting of 1, 2, and 3;
preferably, $R^{10}$ is selected from the group consisting of methyl, methylene-cyclopropyl, and cyclopropyl, wherein the methyl, methylene-cyclopropyl, or cyclopropyl is optionally substituted with one or more substituents independently selected from the group consisting of $R^C$, and $R^C$ is selected from the group consisting of deuterium.

**28.** The compound or the pharmaceutically acceptable salt thereof according to any one of claims 17 to 27, wherein

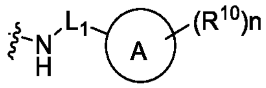

is selected from the group consisting of

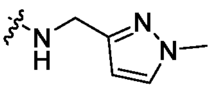 , 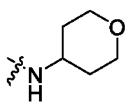 , 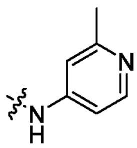 , 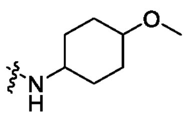 ,

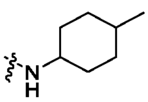 , 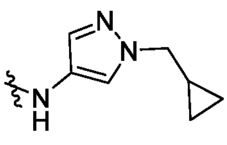 , 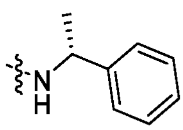 , 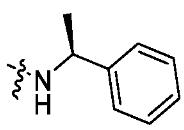 , 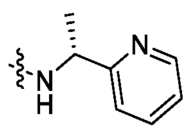 ,

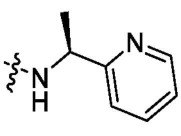 , 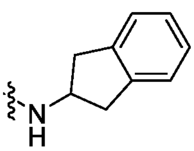 , 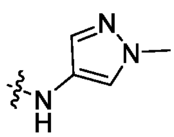 , 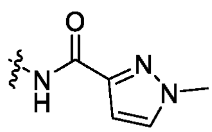 , 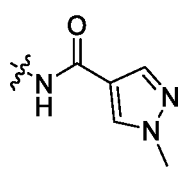 ,

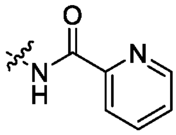, 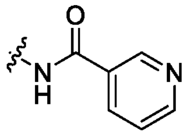, 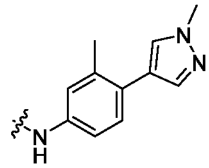, 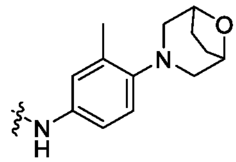,

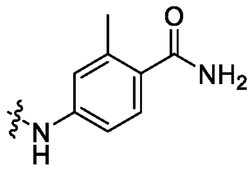, 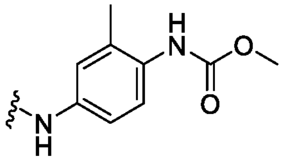, 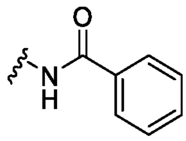, 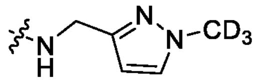,

and

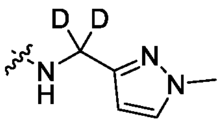.

**29.** The compound or the pharmaceutically acceptable salt thereof according to any one of claims 17 to 28, wherein each $R^{27}$ is independently selected from the group consisting of deuterium, halogen, $C_{1-6}$ alkyl, $C_{1-6}$ haloalkyl, $C_{2-6}$ alkynyl, $C_{1-6}$ alkylene-3- to 6-membered cycloalkyl, $C_{1-6}$ haloalkoxy, -NH-$C_{1-6}$ alkyl, and -NH($C_{1-6}$ alkyl)$_2$; preferably, each $R^{27}$ is independently selected from the group consisting of halogen and $C_{1-6}$ alkyl, most preferably fluorine, chlorine, and methyl.

**30.** The compound or the pharmaceutically acceptable salt thereof according to any one of claims 1 to 29, being selected from the group consisting of:

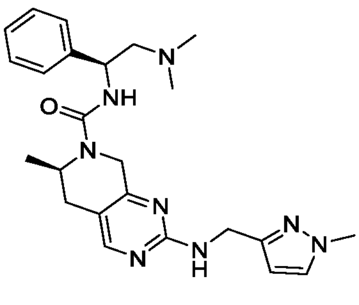, 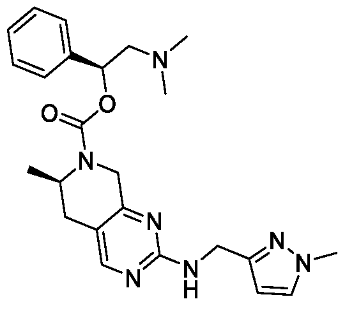, 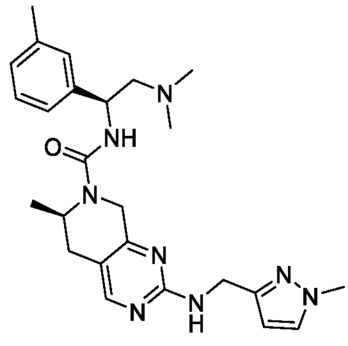,

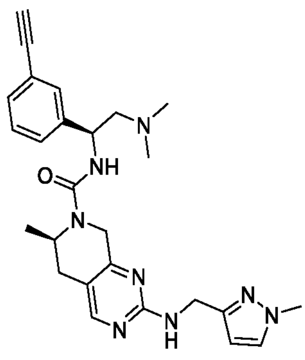, 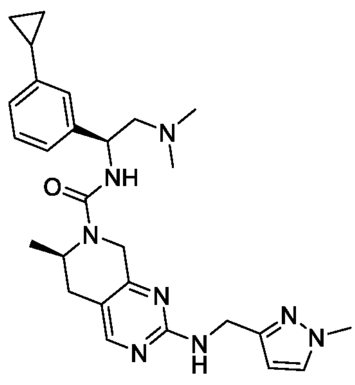, 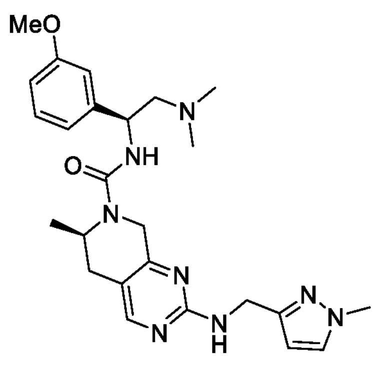,

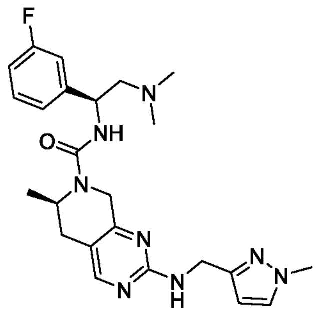,
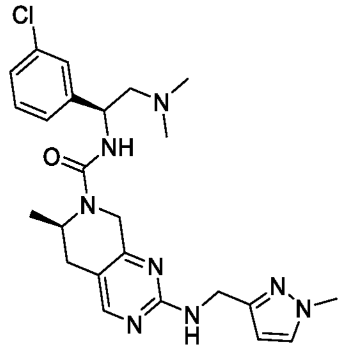,
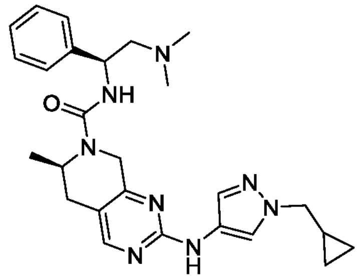,

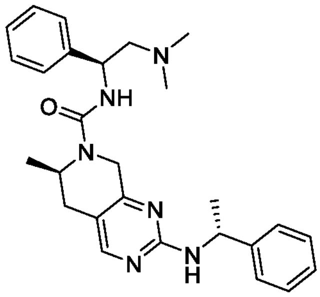,
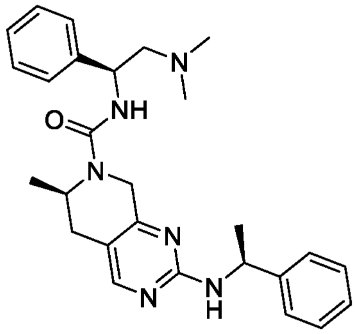,
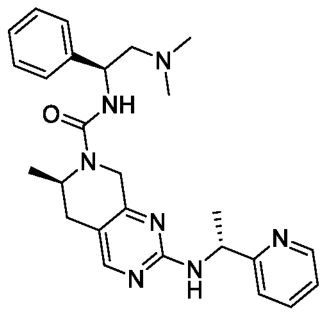,

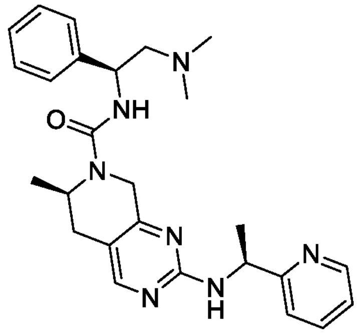,
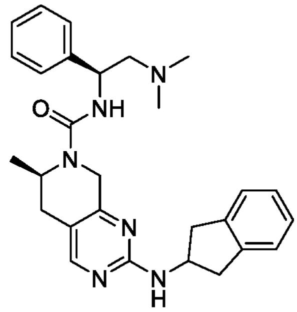,
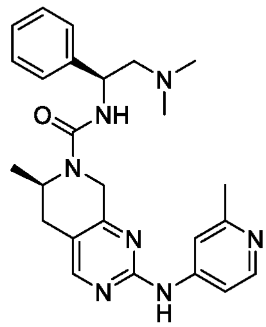,

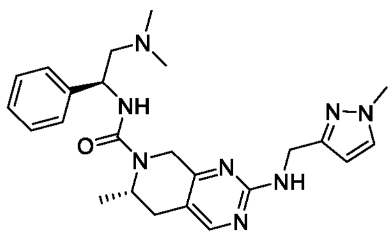,
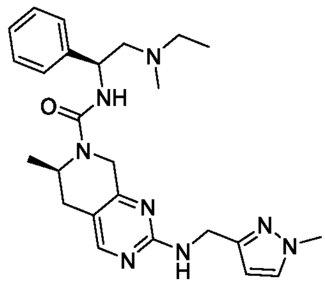,
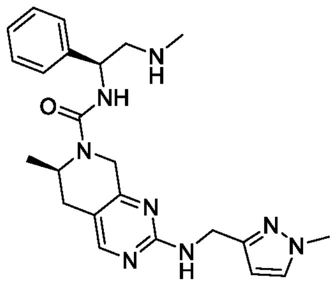,

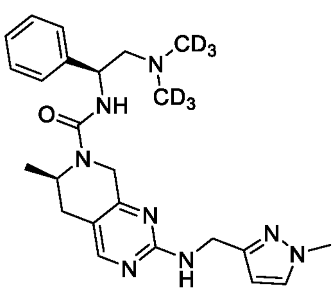,
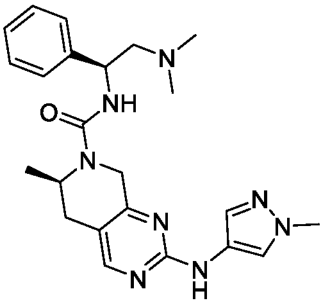,
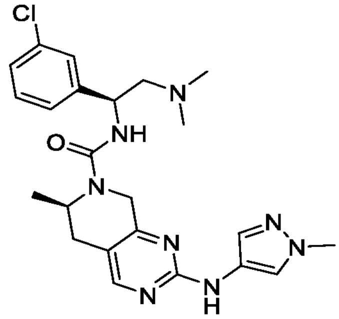,

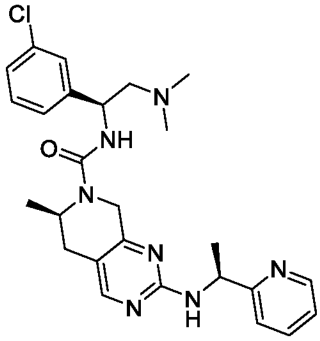,
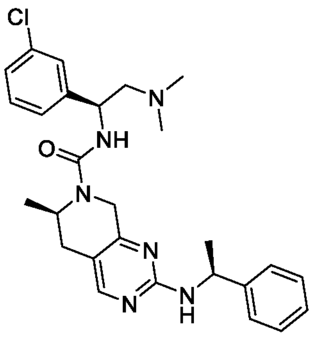,
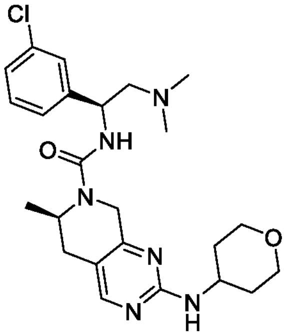,

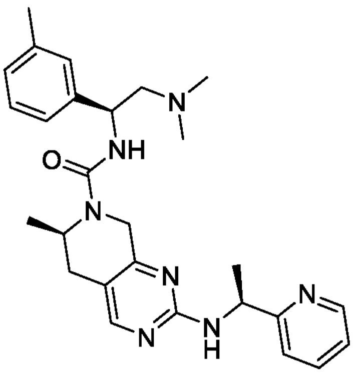,
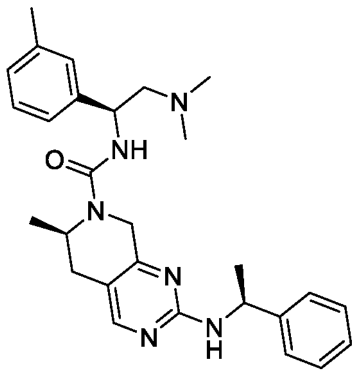,
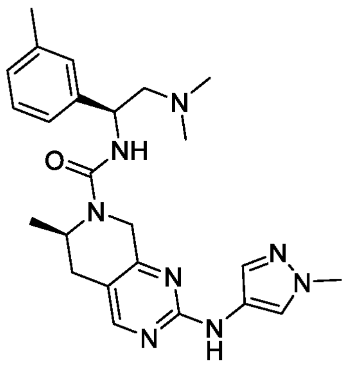,

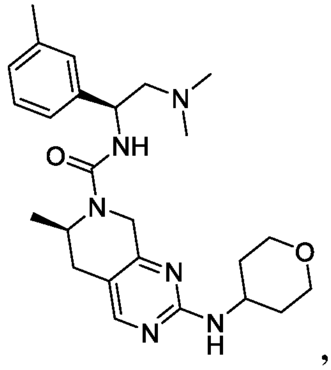,
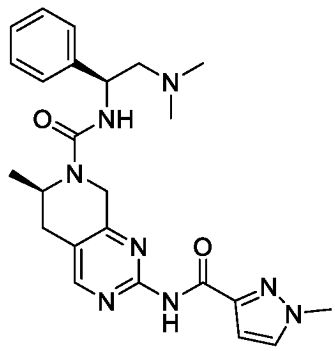,
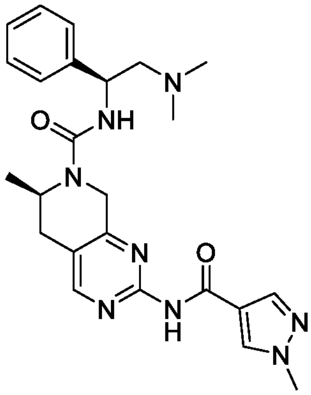,

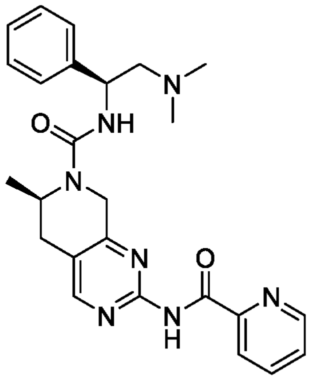,
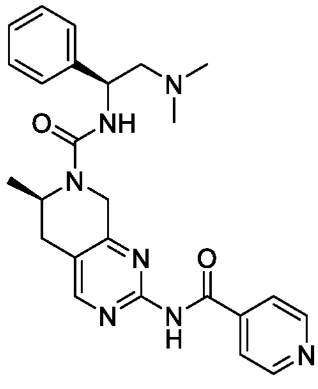,
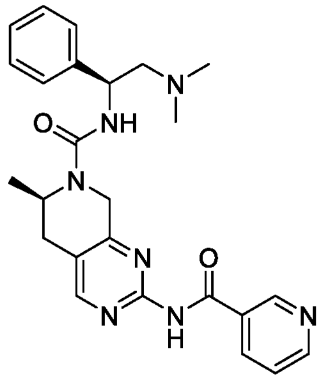,

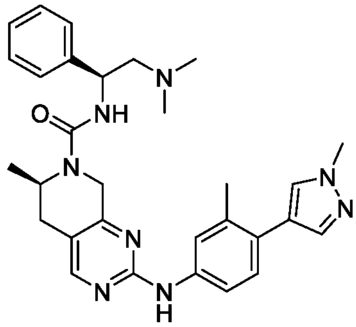,
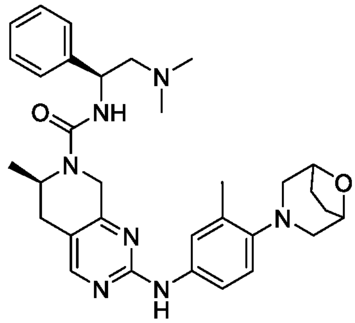,
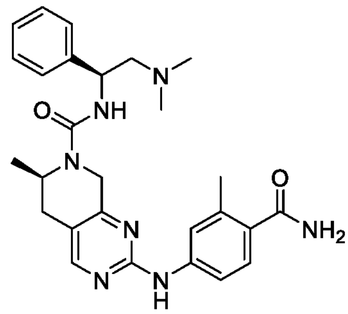,

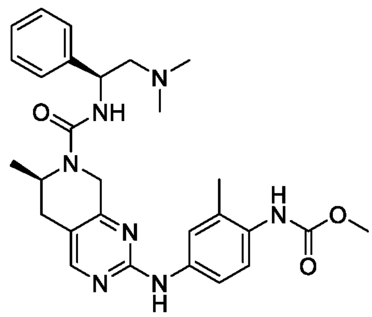,
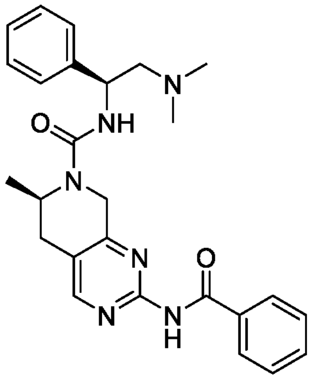,
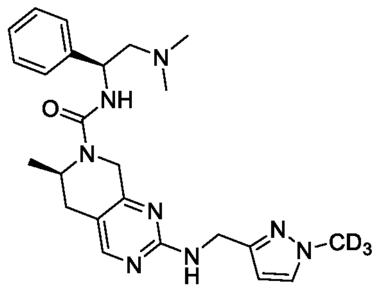,

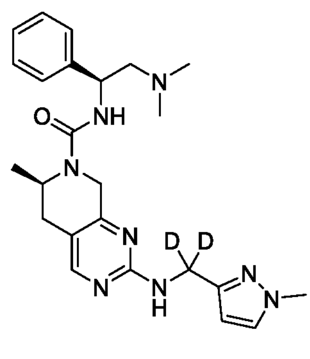,
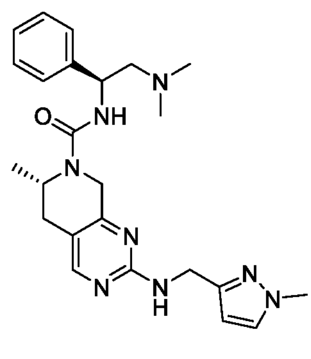,
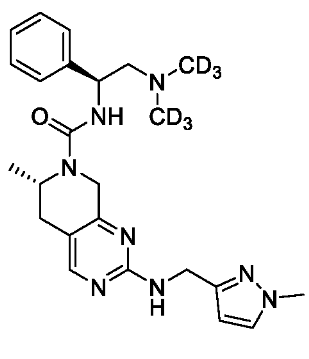,

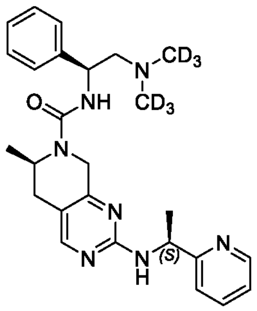,
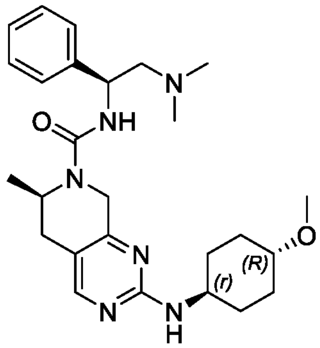,
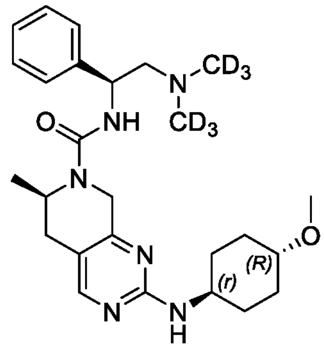,

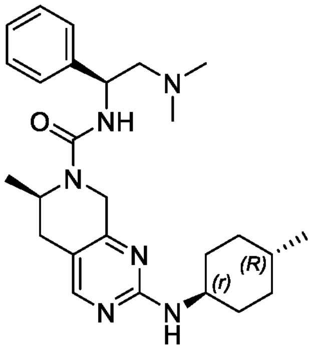,
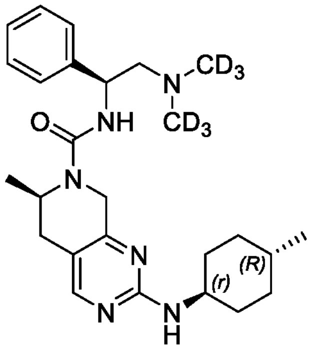,
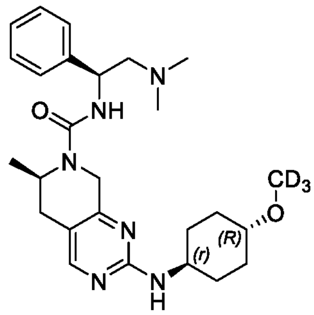,

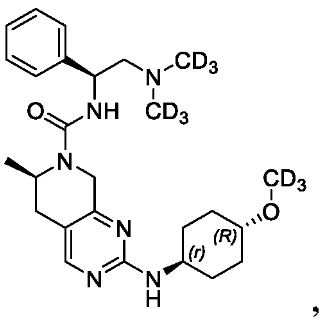,
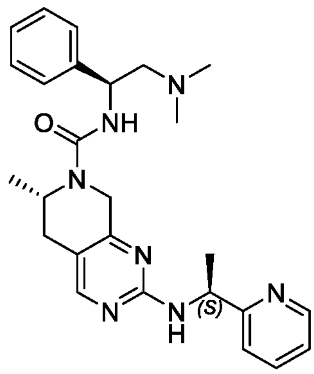,
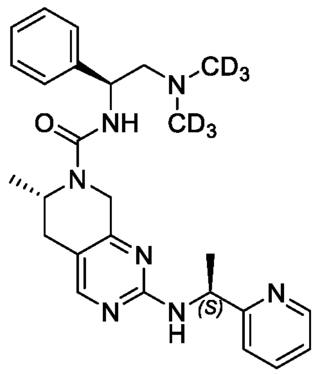,

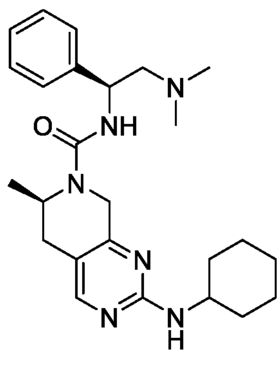, and
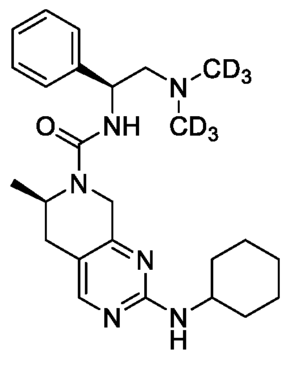.

**31.** An isotopically substituted form of the compound according to any one of claims 1 to 29, wherein preferably, the isotopically substituted form is obtained by substitution with a deuterium atom.

**32.** A method for preparing the compound represented by formula (VI-1-A) or formula (VI-1-B) or the pharmaceutically acceptable salt thereof, or the isotopically substituted form thereof according to any one of claims 20 to 31, comprising a step of subjecting formula (VI-1-A-01) or a pharmaceutically acceptable salt thereof, or (VI-1-B-01) or a pharmaceutically acceptable salt thereof, to a condensation reaction with formula (VI-1-A-02) or a pharmaceutically acceptable salt thereof under the action of carbonyldiimidazole, phosgene, or triphosgene,

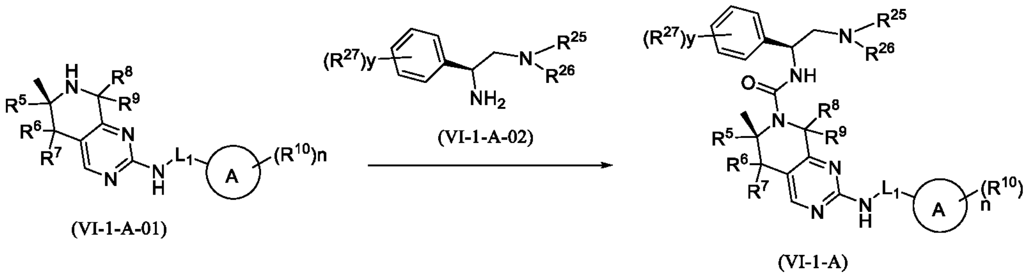

(VI-1-A-01) (VI-1-A-02) (VI-1-A)

or

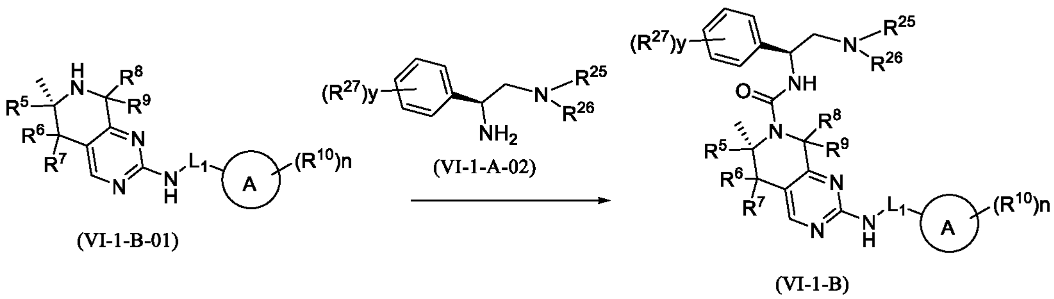

(VI-1-B-01) (VI-1-A-02) (VI-1-B) ,

wherein $R^5$, $R^6$, $R^7$, $R^8$, $R^9$, $L_1$, ring A, $R^{10}$, n, $R^{25}$, $R^{26}$, $R^{27}$, and y are each as defined in claim 17.

**33.** A pharmaceutical composition, comprising the compound or the pharmaceutically acceptable salt thereof according to any one of claims 1 to 30, or the isotopically substituted form according to claim 31, and a pharmaceutically acceptable excipient.

**34.** Use of the compound or the pharmaceutically acceptable salt thereof according to any one of claims 1 to 30, or the isotopically substituted form according to claim 31, or the pharmaceutical composition according to claim 33 in the preparation of a medicament for treating and/or preventing a disease or disorder associated with abnormal activity of serine/threonine kinases.

**35.** Use of the compound or the pharmaceutically acceptable salt thereof according to any one of claims 1 to 30, or the isotopically substituted form according to claim 31, or the pharmaceutical composition according to claim 33 in the preparation of a medicament for treating and/or preventing a disease or disorder associated with abnormal activity of CDK7, wherein preferably, the disease or disorder associated with abnormal activity of CDK7 is selected from the group consisting of a proliferative disease, an inflammatory disease, an autoinflammatory disease, an autoimmune disease, and an infectious disease.

**36.** Use of the compound or the pharmaceutically acceptable salt thereof according to any one of claims 1 to 30, or the isotopically substituted form according to claim 31, or the pharmaceutical composition according to claim 33 in the preparation of a medicament for treating and/or preventing a disease or disorder, wherein the disease or disorder is selected from the group consisting of a proliferative disease, an inflammatory disease, an autoinflammatory disease, an autoimmune disease, and an infectious disease.

**37.** The use according to any one of claim 35 or 36, wherein the proliferative disease is cancer; preferably, the cancer is selected from the group consisting of a hematological tumor and a solid tumor, wherein the hematological tumor is selected from the group consisting of chronic lymphocytic leukemia, acute lymphocytic leukemia, T-cell acute lymphocytic leukemia, chronic myelogenous leukemia, and acute myeloid leukemia, and the solid tumor is selected from the group consisting of breast cancer, intestinal cancer, lung cancer, pancreatic cancer, prostate cancer, Ewing's sarcoma, osteoma, neuroblastoma, cervical cancer, ovarian cancer, gastric cancer, and liver cancer.

**38.** The use according to claim 37, wherein the breast cancer is triple-negative breast cancer or ER/PR+ HER2- breast

cancer, and preferably, the ER/PR+ HER2- breast cancer is ER/PR+ HER2- breast cancer resistant to a CDK4/6 inhibitor; the lung cancer is selected from the group consisting of non-small cell lung cancer and small cell lung cancer; the intestinal cancer is selected from the group consisting of colon cancer and rectal cancer.

**INTERNATIONAL SEARCH REPORT**

International application No.
**PCT/CN2023/126415**

**A. CLASSIFICATION OF SUBJECT MATTER**

C07D407/04(2006.01)i; C07D471/04(2006.01)i; A61K31/4375(2006.01)i; A61K31/519(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS; CNTXT; DWPI; CNKI; ENTXT; ENTXTC; 超星读秀, DUXIU; ISI_Web of Science; STN-REG: 上海拓界生物医药科技有限公司, 朱国栋, 胡涛, 陈美君, 霍书华, 李娇, 李云飞, 哌啶并嘧啶, CDK7, cyclin-dependent kinase 7, 肿瘤, 癌症, tumor, cancer, pyrido 4w pyrimidine, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022064009 A1 (JANSSEN PHARMACEUTICA NV.) 31 March 2022 (2022-03-31)<br>claims 1 and 12-17, and description, pages 116-174 | 1, 9-16, 31, 33-38 |
| X | CN 103635472 A (ARRAY BIOPHARMA INC. et al.) 12 March 2014 (2014-03-12)<br>description, pages 39, 53, and 54, embodiments 1 and 2, and claims 1 and 22-27 | 2-38 |
| X | CAS. "RN: 2649602-21-9"<br>*REGISTRY,* 06 July 2021 (2021-07-06),<br>compound structure formula | 2, 3, 6-9, 12, 15 |
| X | CAS. "RN: 2649594-85-2"<br>*REGISTRY,* 06 July 2021 (2021-07-06),<br>compound structure formula | 2, 3, 6-9, 12, 15 |
| A | WO 2016105528 A2 (DANA-FARBER CANCER INSTITUTE INC.) 30 June 2016 (2016-06-30)<br>entire document | 1-38 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 November 2023** | **05 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/126415**

| Patent document cited in search report | Publication date (day/month/year) | Patent family member(s) | Publication date (day/month/year) |
|---|---|---|---|
| WO 2022064009 A1 | 31 March 2022 | AU 2021350161 A1 | 08 June 2023 |
| | | BR 112023005005 A2 | 18 April 2023 |
| | | KR 20230074762 A | 31 May 2023 |
| | | CA 3191993 A1 | 31 March 2022 |
| | | CN 116194103 A | 30 May 2023 |
| CN 103635472 A | 12 March 2014 | ES 2543050 T3 | 14 August 2015 |
| | | EP 2681215 A1 | 08 January 2014 |
| | | EP 2681215 B1 | 22 April 2015 |
| | | BR 112013021896 A2 | 08 November 2016 |
| | | RU 2013143839 A | 10 April 2015 |
| | | MX 2013009877 A | 11 February 2014 |
| | | MX 339873 B | 15 June 2016 |
| | | KR 20140014190 A | 05 February 2014 |
| | | KR 101961500 B1 | 22 March 2019 |
| | | CA 2828478 A1 | 07 September 2012 |
| | | CA 2828478 C | 31 December 2019 |
| | | JP 6085866 B2 | 01 March 2017 |
| | | US 2013338140 A1 | 19 December 2013 |
| | | US 9133187 B2 | 15 September 2015 |
| | | WO 2012118850 A1 | 07 September 2012 |
| | | JP 2014506930 W | 20 March 2014 |
| | | HK 1191010 A0 | 18 July 2014 |
| | | HK 1192543 A0 | 22 August 2014 |
| | | CN 103635472 B | 12 January 2018 |
| | | HK 1191010 A1 | 30 November 2018 |
| WO 2016105528 A2 | 30 June 2016 | US 2021115051 A1 | 22 April 2021 |
| | | US 2022024929 A9 | 27 January 2022 |
| | | EP 3236959 A2 | 01 November 2017 |
| | | EP 3236959 A4 | 25 April 2018 |
| | | CA 2972239 A1 | 30 June 2016 |
| | | AU 2015371251 A1 | 13 July 2017 |
| | | AU 2015371251 B2 | 11 June 2020 |
| | | WO 2016105528 A3 | 18 August 2016 |
| | | US 2019055248 A1 | 21 February 2019 |
| | | US 10870651 B2 | 22 December 2020 |
| | | JP 6854762 B2 | 07 April 2021 |
| | | HK 1245260 A0 | 24 August 2018 |
| | | JP 2018502098 W | 25 January 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016058544 A
- WO 2018013867 A
- WO 2019143719 A
- WO 2019143730 A
- WO 2019099298 A
- WO 2020093006 A
- WO 2020093011 A
- WO 2022064009 A
- WO 2022064009 A1